# EUROPEAN PATENT SPECIFICATION

(11) **EP 3 052 495 B1**
(45) Date of publication and mention of the grant of the patent: **26.06.2019**
(21) Application number: 14793620.7
(22) Date of filing: 22.09.2014
(51) Int. Cl.: C07D 471/04, C07D 498/04, A61K 31/498, A61P 25/00

(54) **NOVEL BICYCLIC PYRIDINONES AS GAMMA-SECRETASE MODULATORS**
NEUARTIGE BICYCLISCHE PYRIDINONE ALS GAMMA-SEKRETASE-MODULATOREN
NOUVELLES PYRIDINONES BICYCLIQUES UTILISÉES COMME MODULATEURS DE GAMMA-SÉCRÉTASE

(30) Priority: 04.10.2013 US 201361886705 P
(43) Date of publication of application: 10.08.2016
(73) Proprietor: Pfizer Inc., New York, NY 10017 (US)
(72) Inventor: PETTERSSON, Martin Youngjin, Littleton, Massachusetts 01460 (US); JOHNSON, Douglas Scott, Concord, Massachusetts 01742 (US); SUBRAMANYAM, Chakrapani, South Glastonbury, Connecticut 06733 (US); O'DONNELL, Christopher John, Mystic, Connecticut 06355 (US); AM ENDE, Christopher William, Mystic, Connecticut 06355 (US); GREEN, Michael Eric, Boston, Massachusetts 02127 (US); PATEL, Nandini Chaturbhai, Waltham, Massachusetts 02453 (US); STIFF, Cory Michael, Salem, Connecticut 06420 (US); TRAN, Tuan Phong, Ledyard, Connecticut 06339 (US); KAUFFMAN, Gregory Wayne, East Greenwich, Rhode Island 02818 (US); STEPAN, Antonia Friederike, Providence, Rhode Island 02903 (US); VERHOEST, Patrick Robert, Newton, Massachusetts 02460 (US)
(74) Representative: Pfizer
(86) International application number: PCT/IB2014/064738
(87) International publication number: WO 2015/049616

(56) References cited:
- WO-A1-2012/131539
- WO-A1-2013/171712
- WO-A1-2014/045156
- WO-A1-2014/111457
- MARTIN PETTERSSON ET AL: "Design, Synthesis, and Pharmacological Evaluation of a Novel Series of Pyridopyrazine-1,6-dione [gamma]-Secretase Modulators", JOURNAL OF MEDICINAL CHEMISTRY, vol. 57, no. 3, 13 February 2014 (2014-02-13), pages 1046-1062, XP055156862, ISSN: 0022-2623, DOI: 10.1021/jm401782h

## Description

### FIELD OF THE INVENTION

The present invention relates to novel bicyclic pyridinone compounds useful for the treatment of neurodegenerative and/or neurological disorders, such as Alzheimer's disease and Down's syndrome.

### BACKGROUND OF THE INVENTION

Dementia results from a wide variety of distinctive pathological processes. The most common pathological processes causing dementia are Alzheimer's disease (AD), cerebral amyloid angiopathy (CM) and prion-mediated diseases (see, e.g., Haan et al., Clin. Neurol. Neurosurg. 1990, 92(4):305-310; Glenner et al., J. Neurol. Sci. 1989, 94:1-28). AD affects nearly half of all people past the age of 85, the most rapidly growing portion of the United States population. As such, the number of AD patients in the United States is expected to increase from about 4 million to about 14 million by 2050.

Gamma-secretase modulators, useful for the treatment of neurodegenerative and/or neurological disorders are known in the art, e.g. in WO 2012/131539, WO 2013/171712, WO 2014/111457 and WO 2014/045156. However further γ-secretase modulators would be desirable. The present invention thus relates to a group of γ-secretase modulators, useful for the treatment of neurodegenerative and/or neurological disorders such as Alzheimer's disease and Down's syndrome. (see Ann. Rep. Med. Chem. 2007, Olsen et al., 42: 27-47).

### SUMMARY OF THE INVENTION

The present invention is directed to γ-secretase modulators or pharmaceutically acceptable salts thereof as shown in the appended claims.

Compounds of the invention include Examples 2, 4, 5, 53-56, 58, 60-63, 65, 67 and 69 or a pharmaceutically acceptable salt thereof as described herein.

Also provided herein are compositions comprising a pharmaceutically effective amount of one or more of the compounds described herein and a pharmaceutically acceptable vehicle, carrier or excipient.

The compounds of the present invention are γ-secretase modulators. γ-Secretase plays a role in the production of amyloid beta protein (Aβ) plaques associated with Alzheimer's disease. Accordingly, the compounds of the present invention are believed to be useful in treating a variety of neurodegenerative and/or neurological disorders related to Aβ production.

Other features and advantages of this invention will be apparent from this specification and the appending claims which describe the invention.

### DETAILED DESCRIPTION OF THE INVENTION

### Definitions and Exemplifications

As used throughout this application, including the claims, the following terms have the meanings defined below, unless specifically indicated otherwise. The plural and singular should be treated as interchangeable, other than the indication of number:
The term "(C₁-C₆)alkyl" refers to a linear or branched-chain saturated hydrocarbyl substituent (i.e., a substituent obtained from a hydrocarbon by removal of a hydrogen) containing from 1 to 6 carbon atoms. Examples of such substituents include methyl, ethyl, propyl (including n-propyl and isopropyl), butyl (including n-butyl, isobutyl, *sec-*butyl and *tert*-butyl), pentyl, and hexyl.

The term "(C₁-C₃)alkyl" refers to a linear or branched-chain saturated hydrocarbyl substituent (i.e., a substituent obtained from a hydrocarbon by removal of a hydrogen) containing from 1 to 3 carbon atoms. Examples of such substituents include methyl, ethyl, and propyl (including *n*-propyl and isopropyl).

The term "(C₂-C₆)alkenyl" refers to an aliphatic hydrocarbon having from 2 to 6 carbon atoms and having at least one carbon-carbon double bond, including straight chain or branched chain groups having at least one carbon-carbon double bond. Representative examples include, but are not limited to, ethenyl, 1-propenyl, 2-propenyl (allyl), isopropenyl, 2-methyl-1-propenyl, 1-butenyl, and 2-butenyl. When the compounds of the invention contain a (C₂-C₆)alkenyl group, the compound may exist as the pure E (entgegen) form, the pure Z (zusammen) form, or any mixture thereof.

The term "(C₂-C₆)alkynyl" refers to an aliphatic hydrocarbon having from 2 to 6 carbon atoms and having at least one carbon-carbon triple bond, including straight chain or branched chain groups having at least one carbon-carbon triple bond. Representative examples of an alkynyl include, but are not limited to, acetylenyl, 1-propynyl, 2-propynyl, 3-butynyl, 2-pentynyl, and 1-butynyl.

The term "halogen" refers to fluorine (which may be depicted as -F), chlorine (which may be depicted as -Cl), bromine (which may be depicted as -Br), or iodine (which may be depicted as -I).

The term "halo(C₁-C₆)alkyl" as used herein, refers to a (C₁-C₆)alkyl group, as defined above, wherein at least one hydrogen atom is replaced with a halogen, as defined above. Representative examples of a halo(C₁-C₆)alkyl include, but are not limited to, fluoromethyl,2-fluoroethyl, trifluoromethyl, pentafluoroethyl, and 2-chloro-3-fluoropentyl.

The term "(C₁-C₆)alkoxy" as used herein, means a (C₁-C₆)alkyl group, as defined above, attached to the parent molecular moiety through an oxygen atom. Examples include, but are not limited to, methoxy, ethoxy, propoxy, 2-propoxy, butoxy, *tert*-butoxy, pentyloxy, and hexyloxy.

The term "(C₁-C₆)alkoxy(C₁-C₆)alkyl" as used herein, means a (C₁-C₆)alkoxy group, as defined above, attached to the parent molecular moiety through a (C₁-C₆)alkyl group as defined above.

The term "halo(C₁-C₆)alkoxy" as used herein, refers to a (C₁-C₆)alkoxy group, as defined above, wherein at least one hydrogen atom is replaced with a halogen, as defined above. Representative examples of a halo(C₁-C₆)alkoxy include, but are not limited to, fluoromethoxy, 2-fluoroethoxy, trifluoromethoxy, and pentafluoroethoxy.

The term "(C₁-C₆)alkylthio" as used herein, means a (C₁-C₆)alkyl group, as defined above, appended to the parent molecular moiety through a sulfur atom. Representative examples of (C₁-C₆)alkylthio include, but are not limited to, methylthio, ethylthio, *tert*-butylthio, and hexylthio.

The term "(C₃-C₈)cycloalkyl" refers to a carbocyclic substituent obtained by removing a hydrogen from a saturated carbocyclic molecule having from 3 to 8 carbon atoms. A "(C₃-C₈)cycloalkyl" may be a monocyclic ring, examples of which include cyclopropyl, cyclobutyl, cyclopentyl, cyclohexyl, cycloheptyl, and cyclooctyl. Alternatively, a cycloalkyl may contain more than one ring, such as a (C₄-C₈)bicycloalkyl. The term "(C₄-C₈)bicycloalkyl" refers to a bicyclic system containing 4 to 8 carbon atoms. The bicycloalkyl may be fused, such as bicyclo[1.1.0]butane, bicyclo[2.1.0]pentane, bicyclo[2.2.0]hexane, bicyclo[3.1.0]hexane, bicylco[3.2.0]heptane and bicyclo[3.3.0]octane. The term "bicycloalkyl" also includes bridged bicycloalkyl systems such as, but not limited to, bicyclo[2.2.1]heptane and bicyclo[1.1.1]pentane.

The term "(C₆-C₁₀)aryl" refers to an aromatic substituent containing from 6 to 10 carbon atoms, including one ring or two fused rings. Examples of such aryl substituents include, but are not limited to, phenyl and naphthyl. The (C₆-C₁₀)aryl may also include phenyl and naphthyl substituents that are optionally fused to a (C₃-C₆)cycloalkyl ring (e.g., bicyclo[4.2.0]octa-1,3,5-trienyl) or a (5- to 6-membered)heterocycloalkyl ring (e.g., dihydrobenzofuranyl, benzodioxolyl, and oxoisoindolinyl) as defined herein, wherein a group having such a fused aryl group as a substituent is attached to a carbon atom of the aryl.

The term "(4- to 10-membered)heterocycloalkyl" refers to a substituent obtained by removing a hydrogen from a saturated or partially saturated ring structure containing a total of 4 to 10 ring atoms, wherein at least one of the ring atoms is a heteroatom selected from oxygen, nitrogen, or sulfur. Examples of a (4- to 10-membered)heterocycloalkyl include, but are not limited to, piperidinyl, piperazinyl, pyrrolidinyl, dihydrofuranyl, morpholinyl, tetrahydrofuranyl, tetrahydropyranyl, dihydrothiophenyl, and tetrahydrothiophenyl. Alternatively, a heterocycloalkyl as defined above may comprise 2 rings fused together (including spiro fused rings such as, but not limited to an azaspirooctane ring), wherein at least one such ring contains a heteroatom as a ring atom (i.e., nitrogen, oxygen, or sulfur). In a group that has a heterocycloalkyl substituent, the ring atom of the heterocycloalkyl substituent that is attached to the group may be the at least one heteroatom when the heteroatom is nitrogen, or it may be a ring carbon atom, where the ring carbon atom may be in the same ring as the at least one heteroatom or where the ring carbon atom may be in a different ring from the at least one heteroatom. Similarly, if the heterocycloalkyl substituent is in turn substituted with a group or substituent, the group or substituent may be bound to the at least one heteroatom when the heteroatom is nitrogen, or it may be bound to a ring carbon atom, where the ring carbon atom may be in the same ring as the at least one heteroatom or where the ring carbon atom may be in a different ring from the at least one heteroatom.

It is to be understood that the "(4- to 10-membered)heterocycloalkyl" may optionally be fused to a (C₆-C₁₀)aryl or to a (5- to 14-membered)heteroaryl as defined herein, to form a (7- to 22-membered) ring system. A group having such a fused heterocycloalkyl group as a substituent is attached to a heteroatom of the heterocycloalkyl group when the heteroatom is a nitrogen or to a carbon atom of the heterocycloalkyl group. Such a fused heterocycloalkyl group may be optionally substituted, where chemically permissible, with one or more substituents. For example, the fused (C₆-C₁₀)aryl or (5- to 14-membered)heteroaryl may be optionally substituted with halogen, (C₁-C₆)alkyl, (C₃-C₈)cycloalkyl, (C₁-C₆)alkoxy or oxo.

The term "(5- to 14-membered)heteroaryl" refers to an aromatic ring structure containing from 5 to 14 ring atoms in which at least one of the ring atoms is a heteroatom (i.e., oxygen, nitrogen, or sulfur), with the remaining ring atoms being independently selected from the group consisting of carbon, oxygen, nitrogen, and sulfur. A heteroaryl may be a single ring or 2 or 3 fused rings. Examples of heteroaryl substituents include but are not limited to: 6-membered ring substituents such as pyridinyl, pyrimidinyl, pyrazinyl, and pyridazinyl; 5-membered ring substituents such as triazolyl, imidazolyl, furanyl, thiophenyl (also known as "thiofuranyl"), pyrrolyl, pyrazolyl, oxazolyl, isoxazolyl, thiazolyl, thiadiazolyl, 1,2,3-, 1,2,4-, 1,2,5-, or 1,3,4-oxadiazolyl and isothiazolyl; 6/5-membered fused ring substituents such as indolyl, indazolyl, benzofuranyl, benzothiazolyl, isobenzothiofuranyl, benzothiofuranyl, benzothiophenyl, benzisoxazolyl, benzoxazolyl, benzodioxolyl, furanopyridinyl, purinyl, imidazopyridinyl, pyrrolopyridinyl, pyrazolopyridinyl, thienopyridinyl, and anthranilyl; and 6/6-membered fused ring substituents such as quinolinyl, isoquinolinyl, cinnolinyl, quinazolinyl, oxochromenyl, and 1,4-benzoxazinyl. In a group that has a heteroaryl substituent, the ring atom of the heteroaryl substituent that is attached to the group may be the at least one heteroatom when the heteroatom is nitrogen having an appropriate valence, or it may be a ring carbon atom, where the ring carbon atom may be in the same ring as the at least one heteroatom or where the ring carbon atom may be in a different ring from the at least one heteroatom. Similarly, if the heteroaryl substituent is in turn substituted with a group or substituent, the group or substituent may be bound to the at least one heteroatom when the heteroatom is nitrogen, or it may be attached to a ring carbon atom, where the ring carbon atom may be in the same ring as the at least one heteroatom or where the ring carbon atom may be in a different ring from the at least one heteroatom. The term "heteroaryl" also includes pyridyl *N*-oxides and groups containing a pyridine *N*-oxide ring.

It is to be understood that the "(5- to 14-membered)heteroaryl" may be optionally fused to a (C₃-C₈)cycloalkyl group, or to a (4- to 10-membered)heterocycloalkyl group, as defined herein to form an (6- to 22-membered) ring system (e.g., tetrahydrotriazolopyridinyl, carbazolyl, or dihydrooxazinoindolyl). A group having such a fused heteroaryl group as a substituent is attached to an aromatic carbon of the heteroaryl group or to a heteroatom of the heteroaryl group when the heteroatom is nitrogen. When such a fused heteroaryl group is substituted with one or more substituents, the one or more substituents, unless otherwise specified, are each bound to an aromatic carbon of the heteroaryl group or to a heteroatom of the heteroaryl group when the heteroatom is nitrogen. The fused (C₃-C₈)cycloalkyl group or (4- to 10-membered)heterocycloalkyl group may be optionally substituted with halogen, (C₁-C₆)alkyl, (C₃-C₈)cycloalkyl, or oxo.

The term "hydrogen" refers to a hydrogen substituent, and may be depicted as -H.

The term "hydroxy" or "hydroxyl" refers to -OH. When used in combination with another term(s), the prefix "hydroxy" indicates that the substituent to which the prefix is attached is substituted with one or more hydroxy substituents. Compounds bearing a carbon to which one or more hydroxy substituents are attached include, for example, alcohols, enols and phenol.

The term "cyano" (also referred to as "nitrile") means -CN, which also may be depicted:

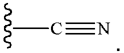

The tern "oxo" refers to a =O moiety.

If a substituent is described as being "substituted," a non-hydrogen substituent is in the place of a hydrogen substituent on a carbon or nitrogen of the substituent. Thus, for example, a substituted alkyl substituent is an alkyl substituent wherein at least one non-hydrogen substituent is in the place of a hydrogen substituent on the alkyl substituent. To illustrate, monofluoroalkyl is alkyl substituted with a fluoro substituent, and difluoroalkyl is alkyl substituted with two fluoro substituents. It should be recognized that if there is more than one substitution on a substituent, each non-hydrogen substituent may be identical or different (unless otherwise stated).

If a substituent is described as being "optionally substituted," the substituent may be either (1) not substituted, or (2) substituted. If a carbon of a substituent is described as being optionally substituted with one or more of a list of substituents, one or more of the hydrogens on the carbon (to the extent there are any) may separately and/or together be replaced with an independently selected optional substituent. If a nitrogen of a substituent is described as being optionally substituted with one or more of a list of substituents, one or more of the hydrogens on the nitrogen (to the extent there are any) may each be replaced with an independently selected optional substituent. As a further example, when there are optional substituents that can be present, e.g., R¹¹ or R¹³, those substituents are as specified in the present specification, and when not present, the group to which the optional substituent could be attached (i.e., C or N) would have the requisite number of hydrogens attached.

This specification uses the terms "substituent," "radical," and "group" interchangeably.

If a substituent is described as being optionally substituted with up to a particular number of non-hydrogen substituents, that substituent may be either (1) not substituted; or (2) substituted by up to that particular number of non-hydrogen substituents or by up to the maximum number of substitutable positions on the substituent, whichever is less. Thus, for example, if a substituent is described as a heteroaryl optionally substituted with up to 3 non-hydrogen substituents, then any heteroaryl with less than 3 substitutable positions would be optionally substituted by up to only as many non-hydrogen substituents as the heteroaryl has substitutable positions. To illustrate, tetrazolyl (which has only one substitutable position) would be optionally substituted with up to one non-hydrogen substituent. To illustrate further, if an amino nitrogen is described as being optionally substituted with up to 2 non-hydrogen substituents, then the nitrogen will be optionally substituted with up to 2 non-hydrogen substituents if the amino nitrogen is a primary nitrogen, whereas the amino nitrogen will be optionally substituted with up to only 1 non-hydrogen substituent if the amino nitrogen is a secondary nitrogen.

If substituents are described as being "independently selected" from a group, each substituent is selected independent of the other(s). Each substituent therefore may be identical to or different from the other substituent(s).

It is to be understood that the optional variables when shown in parenthesis (i.e., "(R¹³)₀₋₃" with a numerical range means that the optional variable is present as an integer selected from 0, 1, 2, or 3. The optional variable "(R¹³)₀₋₁" means that the optional variable is present as an integer selected from 0 or 1.

As used herein, unless specified, the point of attachment of a substituent can be from any suitable position of the substituent.

When a bond to a substituent is shown to cross a bond connecting two atoms in a ring, then such substituent may be bonded to any of the ring-forming atoms in that ring that are substitutable.

"Patient" refers to warm-blooded animals such as, for example, pigs, cows, chickens, horses, guinea pigs, mice, rats, gerbils, cats, rabbits, dogs, monkeys, chimpanzees, and humans.

"Treating" or "treat", as used herein, unless otherwise indicated, means reversing, alleviating, inhibiting the progress of, or preventing the disorder or condition to which such term applies, or one or more symptoms of such disorder or condition. The term "treatment", as used herein, unless otherwise indicated, refers to the act of treating as "treating" is defined immediately above. The term "treating" also includes adjuvant and neo-adjuvant treatment of a subject.

"Pharmaceutically acceptable" indicates that the substance or composition must be compatible, chemically and/or toxicologically, with the other ingredients comprising a formulation, and/or the mammal being treated therewith.

"Isomer" means "stereoisomer" and "geometric isomer" as defined below.

"Stereoisomer" refers to compounds that possess one or more chiral centers, which may each exist in the R or S configuration. Stereoisomers include all diastereomeric, enantiomeric and epimeric forms as well as racemates and mixtures thereof.

"Geometric isomer" refers to compounds that may exist in cis, trans, anti, *entgegen* (E), and *zusammen* (Z) forms as well as mixtures thereof.

As used herein the terms "Formula I", "Formula la", "Formula Ib", "Formula Ic", "Formula Id", "Formula Id'", and "Formula le" may be hereinafter referred to as "compound(s) of the invention." Such terms are also defined to include all forms of the compound of Formulas I through le including hydrates, solvates, isomers, crystalline and non-crystalline forms, isomorphs, polymorphs, and metabolites thereof. For example, the compounds of Formulas I through le, or pharmaceutically acceptable salts thereof, may exist in unsolvated and solvated forms with pharmaceutically acceptable solvents such as water, ethanol and the like. When the solvent or water is tightly bound, the complex will have a well-defined stoichiometry independent of humidity. When, however, the solvent or water is weakly bound, as in channel solvates and hygroscopic compounds, the water/solvent content will be dependent on humidity and drying conditions. In such cases, non-stoichiometry will be the norm. In general, the solvated forms are considered equivalent to the unsolvated forms for the purposes of the present invention.

The compounds of the invention may exist as clathrates or other complexes. Included within the scope of the invention are complexes such as clathrates, drug-host inclusion complexes wherein the drug and host are present in stoichiometric or non-stoichiometric amounts. Also included are complexes of the compounds of the present invention containing two or more organic and/or inorganic components, which may be in stoichiometric or non-stoichiometric amounts. The resulting complexes may be ionized, partially ionized, or non-ionized. For a review of such complexes, see J. Pharm. Sci., 64 (8), 1269-1288 by Haleblian (August 1975).

Compounds of the invention may exist as geometric isomers. The compounds of the invention may possess one or more asymmetric centers, thus existing as two, or more, stereoisomeric forms. The present invention includes all the individual stereoisomers and geometric isomers of the compounds of the invention and mixtures thereof. Individual enantiomers can be obtained by resolution, chiral chromatography, or other methods well-known to those skilled in the art, or by using the relevant enantiomeric reactant or reagent in the synthesis.

The carbon-carbon bonds of the compounds of the invention may be depicted herein using a solid line ( 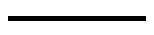 ), a solid wedge ( 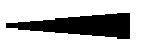 ), or a dotted wedge ( 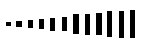 ). The use of a solid line to depict bonds to asymmetric carbon atoms is meant to indicate that all possible stereoisomers (e.g., specific enantiomers, racemic mixtures, etc.) at that carbon atom are included. The use of either a solid or dotted wedge to depict bonds to asymmetric carbon atoms is meant to indicate that the stereoisomer shown is present. When present in racemic compounds, solid and dotted wedges are used to define relative stereochemistry, rather than absolute stereochemistry. Racemic compounds possessing such indicated relative stereochemistry are marked with (+/-). For example, unless stated otherwise, it is intended that the compounds of the invention can exist as stereoisomers, which include cis and trans isomers, optical isomers such as R and S enantiomers, diastereomers, geometric isomers, rotational isomers, conformational isomers, atropisomers, and mixtures thereof. The compounds of the invention may exhibit more than one type of isomerism, and consist of mixtures thereof (such as racemates and diastereomeric pairs). Also included are acid addition or base addition salts wherein the counterion is optically active, for example, D-lactate or L-lysine, or racemic, for example, DL-tartrate or DL-arginine.

When any racemate crystallizes, crystals of two different types are possible. The first type is the racemic compound (true racemate) referred to above wherein one homogeneous form of crystal is produced containing both enantiomers in equimolar amounts. The second type is the racemic mixture or conglomerate wherein two forms of crystal are produced in equimolar amounts each comprising a single enantiomer.

The present invention also includes all pharmaceutically acceptable isotopically labeled compounds, which are identical to those recited in Formulas I through le except that one or more atoms are replaced by an atom having the same atomic number, but an atomic mass or mass number different from the atomic mass or mass number which predominates in nature. Examples of isotopes suitable for inclusion in the compounds of the present invention include, but are not limited to, isotopes of hydrogen, such as ²H, ³H; carbon, such as ¹¹C, ¹³C, and ¹⁴C; chlorine, such as ³⁶Cl; fluorine, such as ¹⁸F; iodine, such as ¹²³I and ¹²⁵I; nitrogen, such as ¹³N and ¹⁵N; oxygen, such as ¹⁵O, ¹⁷O, and ¹⁸O; phosphorus, such as ³²P; and sulfur, such as ³⁵S. Certain isotopically labeled compounds of the present invention, for example those incorporating a radioactive isotope, are useful in drug and/or substrate tissue distribution studies (e.g., assays). The radioactive isotopes tritium, i.e., ³H, and carbon-14, i.e., ¹⁴C, are particularly useful for this purpose in view of their ease of incorporation and ready means of detection. Substitution with heavier isotopes such as deuterium, i.e., ²H, may afford certain therapeutic advantages resulting from greater metabolic stability, for example, increased in vivo half-life or reduced dosage requirements and, hence, may be preferred in some circumstances. Substitution with positron emitting isotopes, such as ¹¹C, ¹⁸F, ¹⁵O and ¹³N, can be useful in positron emission tomography (PET) studies for examining substrate receptor occupancy. Isotopically labeled compounds of the present invention can generally be prepared by conventional techniques known to those skilled in the art or by processes analogous to those described in the accompanying Schemes and/or in the Examples and Preparations, by using an appropriate isotopically labeled reagent in place of the non-labeled reagent previously employed. Pharmaceutically acceptable solvates in accordance with the invention include those wherein the solvent of crystallization may be isotopically substituted, e.g., D₂O, acetone-*d₆*, or DMSO-*d₆*. Compounds of the present invention, as well as the compounds exemplified in Examples 1-174 described below, include isotopically labeled versions of these compounds, such as, but not limited to, the deuterated and tritiated isotopes and all other isotopes discussed above.

The compounds of this invention may be used in the form of salts derived from inorganic or organic acids. Depending on the particular compound, a salt of the compound may be advantageous due to one or more of the salt's physical properties, such as enhanced pharmaceutical stability in differing temperatures and humidities, or a desirable solubility in water or oil. In some instances, a salt of a compound also may be used as an aid in the isolation, purification, and/or resolution of the compound.

Where a salt is intended to be administered to a patient (as opposed to, for example, being used in an in vitro context), the salt preferably is pharmaceutically acceptable. The term "pharmaceutically acceptable salt" refers to a salt prepared by combining a compound of the invention with an acid whose anion, or a base whose cation, is generally considered suitable for human consumption. Pharmaceutically acceptable salts are particularly useful as products of the methods of the present invention because of their greater aqueous solubility relative to the parent compound.

Suitable pharmaceutically acceptable acid addition salts of the compounds of the present invention when possible include those derived from inorganic acids, such as hydrochloric, hydrobromic, hydrofluoric, boric, fluoroboric, phosphoric, metaphosphoric, nitric, carbonic, sulfonic, and sulfuric acids, and organic acids such as acetic, benzenesulfonic, benzoic, citric, ethanesulfonic, fumaric, gluconic, glycolic, isothionic, lactic, lactobionic, maleic, malic, methanesulfonic, trifluoromethanesulfonic, succinic, toluenesulfonic, tartaric, and trifluoroacetic acids. Suitable organic acids generally include but are not limited to aliphatic, cycloaliphatic, aromatic, araliphatic, heterocyclic, carboxylic, and sulfonic classes of organic acids.

Specific examples of suitable organic acids include but are not limited to acetate, trifluoroacetate, formate, propionate, succinate, glycolate, gluconate, digluconate, lactate, malate, tartrate, citrate, ascorbate, glucuronate, maleate, fumarate, pyruvate, aspartate, glutamate, benzoate, anthranilate, stearate, salicylate, p-hydroxybenzoate, phenylacetate, mandelate, embonate (pamoate), methanesulfonate, ethanesulfonate, benzenesulfonate, pantothenate, toluenesulfonate, 2-hydroxyethanesulfonate, sufanilate, cyclohexylaminosulfonate, β-hydroxybutyrate, galactarate, galacturonate, adipate, alginate, butyrate, camphorate, camphorsulfonate, cyclopentanepropionate, dodecylsulfate, glycoheptanoate, glycerophosphate, heptanoate, hexanoate, nicotinate, 2-naphthalenesulfonate, oxalate, palmoate, pectinate, 3-phenylpropionate, picrate, pivalate, thiocyanate, and undecanoate.

Furthermore, where the compounds of the invention carry an acidic moiety, suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts. In another embodiment, base salts are formed from bases which form non-toxic salts, including aluminum, arginine, benzathine, choline, diethylamine, diolamine, glycine, lysine, meglumine, olamine, tromethamine and zinc salts.

Organic salts may be made from secondary, tertiary or quaternary amine salts, such as tromethamine, diethylamine, *N,N'*-dibenzylethylenediamine, chloroprocaine, choline, diethanolamine, ethylenediamine, meglumine (*N*-methylglucamine), and procaine. Basic nitrogen-containing groups may be quaternized with agents such as lower alkyl (C₁-C₆) halides (e.g., methyl, ethyl, propyl, and butyl chlorides, bromides, and iodides), dialkyl sulfates (e.g., dimethyl, diethyl, dibutyl, and diamyl sulfates), long chain halides (e.g., decyl, lauryl, myristyl, and stearyl chlorides, bromides, and iodides), arylalkyl halides (e.g., benzyl and phenethyl bromides), and others.

In one embodiment, hemisalts of acids and bases may also be formed, for example, hemisulfate and hemicalcium salts.

There may also exist certain derivatives of the compound of the invention that may have little or no pharmacological activity themselves can, when administered into or onto the body, be converted into the compound of the invention having the desired activity, for example, by hydrolytic cleavage. Such derivatives are referred to as "prodrugs." Further information on the use of prodrugs may be found in "Pro-drugs as Novel Delivery Systems, Vol. 14, ACS Symposium Series (T. Higuchi and V. Stella) and "Bioreversible Carriers in Drug Design," Pergamon Press, 1987 (ed. E. B. Roche, American Pharmaceutical Association). Prodrugs can, for example, be produced by replacing appropriate functionalities present in the compounds of the present invention with certain moieties known to those skilled in the art as "pro-moieties" as described, for example, in "Design of Prodrugs" by H. Bundgaard (Elsevier, 1985).

One skilled in the art will appreciate that compounds of the invention can also be prepared with certain protecting groups that are useful for purification or storage and can be removed before administration to a patient. The protection and deprotection of functional groups is described in "Protective Groups in Organic Chemistry", edited by J. W. F. McOmie, Plenum Press (1973) and "Protective Groups in Organic Synthesis", 3rd edition, T. W. Greene and P. G. M. Wuts, Wiley-Interscience (1999).

Typically, a compound of the invention is administered in an amount effective to treat a condition as described herein. The compounds of the invention are administered by any suitable route in the form of a pharmaceutical composition adapted to such a route, and in a dose effective for the treatment intended. Therapeutically effective doses of the compounds required to treat the progress of the medical condition are readily ascertained by one of ordinary skill in the art using preclinical and clinical approaches familiar to the medicinal arts. The term "therapeutically effective amount" as used herein refers to that amount of the compound being administered which will relieve to some extent one or more of the symptoms of the disorder being treated.

### Compounds

The compounds of the invention are as depicted in Formula Ic immediately below: 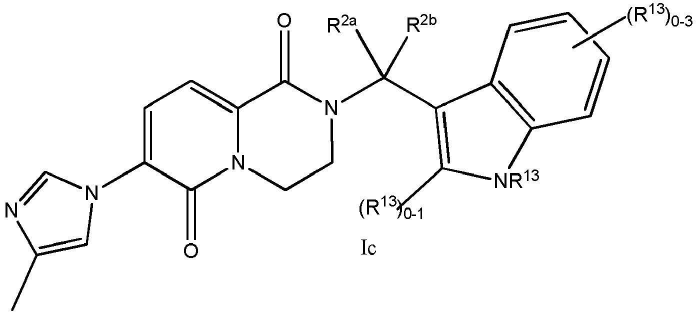 wherein R^{2a}, R^{2b} and each R¹³ are depicted so as to represent the compounds according to examples 2, 4, 5, 53-56, 58, 60-63, 65, 67 and 69.

### Pharmacology

Alzheimer's disease (AD) research indicates that the disease is associated with the buildup of plaques in variable shapes and sizes in the brain. The primary plaques associated with AD are composed of amyloid beta protein (Aβ). Aβ is produced when the amyloid protein precursor (APP) undergoes successive proteolysis by β- and γ-secretase (Haas et al., "Trafficking and proteolytic processing of APP." Cold Spring Harbor Perspect. Med., 2011). γ-Secretase is a large complex of four different integral proteins, one of which has been identified as a catalytic component that comprises an unusual membrane-embedded component (De Strooper, Bart et al., "Presenilins and γ-Secretase: Structure, Function, and Role in Alzheimer's Disease. "Cold Spring Harbor Perspect. Med. 2012;2:a006304). The catalytic components, known as presenilins, were first discovered as sites of missense mutations responsible for early-onset Alzheimer's disease. The encoded multipass membrane proteins were subsequently found to be the catalytic components of γ-secretases, membrane-embedded aspartyl protease complexes responsible for generating the carboxyl terminus of the amyloid beta protein from the amyloid protein precursor. (De Strooper, Bart et al.; 2012). Accordingly, targeting γ-secretase proteins for drug discovery has become a main focus of Alzheimer's disease research.

The compounds of the present invention are believed to be γ-secretase modulators and can be used for treating conditions or diseases of the central nervous system identified to have enhanced gamma secretase activity, such as Niemann-Pick disease type C; neurological disorders (such as migraine; epilepsy; Alzheimer's disease; Parkinson's disease; brain injury; stroke; cerebrovascular diseases (including cerebral arteriosclerosis, cerebral amyloid angiopathy, hereditary cerebral hemorrhage, and brain hypoxia-ischemia); cognitive disorders (including amnesia, senile dementia, HIV-associated dementia, Alzheimer's disease, Huntington's disease, Lewy body dementia, vascular dementia, drug-related dementia, myoclonus, dystonia, delirium, Pick's disease, Creutzfeldt-Jacob disease, HIV disease, Gilles de la Tourette's syndrome, epilepsy, and mild cognitive impairment); tardive dyskinesia; muscular spasms and disorders associated with muscular spasticity or weakness including tremors; mental deficiency (including spasticity, Down's syndrome and fragile X syndrome); sleep disorders (including hypersomnia, circadian rhythm sleep disorder, insomnia, parasomnia, and sleep deprivation) and psychiatric disorders such as anxiety (including acute stress disorder, generalized anxiety disorder, social anxiety disorder, panic disorder, post-traumatic stress disorder, agoraphobia, and obsessive-compulsive disorder); factitious disorders (including acute hallucinatory mania); impulse control disorders (including compulsive gambling and intermittent explosive disorder); mood disorders (including bipolar I disorder, bipolar II disorder, mania, mixed affective state, major depression, chronic depression, seasonal depression, psychotic depression, premenstrual syndrome (PMS), premenstrual dysphoric disorder (PDD), and postpartum depression); psychomotor disorders; psychotic disorders (including schizophrenia, schizoaffective disorder, schizophreniform, and delusional disorder); drug dependence (including narcotic dependence, alcoholism, amphetamine dependence, cocaine addiction, nicotine dependence, and drug withdrawal syndrome); eating disorders (including anorexia, bulimia, binge eating disorder, hyperphagia, obesity, compulsive eating disorders and pagophagia); sexual dysfunction disorders; urinary incontinence; neuronal damage disorders (including ocular damage, retinopathy or macular degeneration of the eye; tinnitus, hearing impairment and loss; and brain edema) and pediatric psychiatric disorders (including attention deficit disorder, attention deficit/hyperactive disorder, conduct disorder, and autism) in a mammal, preferably a human.

In certain embodiments, the compounds of the present invention can be utilized for treating a neurological disorder (such as migraine; epilepsy; Alzheimer's disease; Parkinson's disease; Niemann Pick type C; brain injury; stroke; cerebrovascular disease; cognitive disorder; sleep disorder) or a psychiatric disorder (such as anxiety; factitious disorder; impulse control disorder; mood disorder; psychomotor disorder; psychotic disorder; drug dependence; eating disorder; and pediatric psychiatric disorder) in a mammal, preferably a human.

Compounds of the present invention may also be useful for improving memory (both short term and long term) and learning ability.

The text revision of the fourth edition of the Diagnostic and Statistical Manual of Mental Disorders (DSM-IV-TR) (2000, American Psychiatric Association, Washington D.C.) provides a diagnostic tool for identifying many of the disorders described herein. The skilled artisan will recognize that there are alternative nomenclatures, nosologies, and classification systems for disorders described herein, including those as described in the DMS-IV and that terminology and classification systems evolve with medical scientific progress.

### Formulations

The compounds of the invention may be administered orally. Oral administration may involve swallowing, so that the compound enters the gastrointestinal tract, or buccal or sublingual administration may be employed, by which the compound enters the blood stream directly from the mouth.

In another embodiment, the compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

In another embodiment, the compounds of the invention may also be administered topically to the skin or mucosa, that is, dermally or transdermally. In another embodiment, the compounds of the invention can also be administered intranasally or by inhalation. In another embodiment, the compounds of the invention may be administered rectally or vaginally. In another embodiment, the compounds of the invention may also be administered directly to the eye or ear.

The dosage regimen for the compounds and/or compositions containing the compounds is based on a variety of factors, including the type, age, weight, sex and medical condition of the patient; the severity of the condition; the route of administration; and the activity of the particular compound employed. Thus the dosage regimen may vary widely. Dosage levels of the order from about 0.01 mg to about 100 mg per kilogram of body weight per day are useful in the treatment of the above-indicated conditions. In one embodiment, the total daily dose of a compound of the invention (administered in single or divided doses) is typically from about 0.01 to about 100 mg/kg. In another embodiment, the total daily dose of the compound of the invention is from about 0.1 to about 50 mg/kg, and in another embodiment, from about 0.5 to about 30 mg/kg (i.e., mg compound of the invention per kg body weight). In one embodiment, dosing is from 0.01 to 10 mg/kg/day. In another embodiment, dosing is from 0.1 to 1.0 mg/kg/day. Dosage unit compositions may contain such amounts or submultiples thereof to make up the daily dose. In many instances, the administration of the compound will be repeated a plurality of times in a day (typically no greater than 4 times). Multiple doses per day typically may be used to increase the total daily dose, if desired.

For oral administration, the compositions may be provided in the form of tablets containing 0.01, 0.05, 0.1, 0.5, 1.0, 2.5, 5.0, 10.0, 15.0, 25.0, 50.0, 75.0, 100, 125, 150, 175, 200, 250 and 500 milligrams of the active ingredient for the symptomatic adjustment of the dosage to the patient. A medicament typically contains from about 0.01 mg to about 500 mg of the active ingredient, or in another embodiment, from about 1 mg to about 100 mg of active ingredient. Intravenously, doses may range from about 0.1 to about 10 mg/kg/minute during a constant rate infusion.

Suitable subjects according to the present invention include mammalian subjects. Mammals according to the present invention include, but are not limited to, canine, feline, bovine, caprine, equine, ovine, porcine, rodents, lagomorphs, primates, and the like, and encompass mammals in utero. In one embodiment, humans are suitable subjects. Human subjects may be of either gender and at any stage of development.

In another embodiment, the invention comprises the use of one or more compounds of the invention for the preparation of a medicament for the treatment of the conditions recited herein.

For the treatment of the conditions referred to above, the compounds of the invention can be administered as compound per se. Alternatively, pharmaceutically acceptable salts are suitable for medical applications because of their greater aqueous solubility relative to the parent compound.

In another embodiment, the present invention comprises pharmaceutical compositions. Such pharmaceutical compositions comprise a compound of the invention presented with a pharmaceutically acceptable carrier. The carrier can be a solid, a liquid, or both, and may be formulated with the compound as a unit-dose composition, for example, a tablet, which can contain from 0.05% to 95% by weight of the active compounds. A compound of the invention may be coupled with suitable polymers as targetable drug carriers. Other pharmacologically active substances can also be present.

The compounds of the present invention may be administered by any suitable route, preferably in the form of a pharmaceutical composition adapted to such a route, and in a dose effective for the treatment intended. The active compounds and compositions, for example, may be administered orally, rectally, parenterally, or topically.

Oral administration of a solid dose form may be, for example, presented in discrete units, such as hard or soft capsules, pills, cachets, lozenges, or tablets, each containing a predetermined amount of at least one compound of the present invention. In another embodiment, the oral administration may be in a powder or granule form. In another embodiment, the oral dose form is sub-lingual, such as, for example, a lozenge. In such solid dosage forms, the compounds of the invention are ordinarily combined with one or more adjuvants. Such capsules or tablets may contain a controlled-release formulation. In the case of capsules, tablets, and pills, the dosage forms also may comprise buffering agents or may be prepared with enteric coatings.

In another embodiment, oral administration may be in a liquid dose form. Liquid dosage forms for oral administration include, for example, pharmaceutically acceptable emulsions, solutions, suspensions, syrups, and elixirs containing inert diluents commonly used in the art (i.e., water). Such compositions also may comprise adjuvants, such as wetting, emulsifying, suspending, flavoring (e.g., sweetening), and/or perfuming agents.

In another embodiment, the present invention comprises a parenteral dose form. "Parenteral administration" includes, for example, subcutaneous injections, intravenous injections, intraperitoneal injections, intramuscular injections, intrasternal injections, and infusion. Injectable preparations (i.e., sterile injectable aqueous or oleaginous suspensions) may be formulated according to the known art using suitable dispersing, wetting, and/or suspending agents.

In another embodiment, the present invention comprises a topical dose form. "Topical administration" includes, for example, transdermal administration, such as via transdermal patches or iontophoresis devices, intraocular administration, or intranasal or inhalation administration. Compositions for topical administration also include, for example, topical gels, sprays, ointments, and creams. A topical formulation may include a compound which enhances absorption or penetration of the active ingredient through the skin or other affected areas. When the compounds of this invention are administered by a transdermal device, administration will be accomplished using a patch either of the reservoir and porous membrane type or of a solid matrix variety. Typical formulations for this purpose include gels, hydrogels, lotions, solutions, creams, ointments, dusting powders, dressings, foams, films, skin patches, wafers, implants, sponges, fibers, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated - see, for example, Finnin and Morgan, J. Pharm. Sci., 88 (10), 955-958 (1999).

Formulations suitable for topical administration to the eye include, for example, eye drops wherein the compound of this invention is dissolved or suspended in a suitable carrier. A typical formulation suitable for ocular or aural administration may be in the form of drops of a micronized suspension or solution in isotonic, pH-adjusted, sterile saline. Other formulations suitable for ocular and aural administration include ointments, biodegradable (e.g., absorbable gel sponges, collagen) and non-biodegradable (e.g., silicone) implants, wafers, lenses and particulate or vesicular systems, such as niosomes or liposomes. A polymer such as crossed-linked polyacrylic acid, polyvinyl alcohol, hyaluronic acid, a cellulosic polymer, for example, hydroxypropylmethyl cellulose, hydroxyethyl cellulose, or methyl cellulose, or a heteropolysaccharide polymer, for example, gelan gum, may be incorporated together with a preservative, such as benzalkonium chloride. Such formulations may also be delivered by iontophoresis.

For intranasal administration or administration by inhalation, the active compounds of the invention are conveniently delivered in the form of a solution or suspension from a pump spray container that is squeezed or pumped by the patient or as an aerosol spray presentation from a pressurized container or a nebulizer, with the use of a suitable propellant. Formulations suitable for intranasal administration are typically administered in the form of a dry powder (either alone; as a mixture, for example, in a dry blend with lactose; or as a mixed component particle, for example, mixed with phospholipids, such as phosphatidylcholine) from a dry powder inhaler or as an aerosol spray from a pressurized container, pump, spray, atomizer (preferably an atomizer using electrohydrodynamics to produce a fine mist), or nebulizer, with or without the use of a suitable propellant, such as 1,1,1,2-tetrafluoroethane or 1,1,1,2,3,3,3-heptafluoropropane. For intranasal use, the powder may comprise a bioadhesive agent, for example, chitosan or cyclodextrin.

In another embodiment, the present invention comprises a rectal dose form. Such rectal dose form may be in the form of, for example, a suppository. Cocoa butter is a traditional suppository base, but various alternatives may be used as appropriate.

Other carrier materials and modes of administration known in the pharmaceutical art may also be used. Pharmaceutical compositions of the invention may be prepared by any of the well-known techniques of pharmacy, such as effective formulation and administration procedures. The above considerations in regard to effective formulations and administration procedures are well known in the art and are described in standard textbooks. Formulation of drugs is discussed in, for example, Hoover, John E., Remington's Pharmaceutical Sciences, Mack Publishing Co., Easton, Pennsylvania, 1975; Liberman et al., Eds., Pharmaceutical Dosage Forms, Marcel Decker, New York, N.Y., 1980; and Kibbe et al., Eds., Handbook of Pharmaceutical Excipients (3rd Ed.), American Pharmaceutical Association, Washington, 1999.

The compounds of the present invention can be used, alone or in combination with other therapeutic agents, in the treatment of various conditions or disease states. The compound(s) of the present invention and other therapeutic agent(s) may be administered simultaneously (either in the same dosage form or in separate dosage forms) or sequentially. An exemplary therapeutic agent may be, for example, a metabotropic glutamate receptor agonist.

The administration of two or more compounds "in combination" means that the two compounds are administered closely enough in time that the presence of one alters the biological effects of the other. The two or more compounds may be administered simultaneously, concurrently or sequentially. Additionally, simultaneous administration may be carried out by mixing the compounds prior to administration or by administering the compounds at the same point in time but at different anatomic sites or using different routes of administration.

The phrases "concurrent administration," "co-administration," "simultaneous administration," and "administered simultaneously" mean that the compounds are administered in combination.

The present invention includes the use of a combination of a γ-secretase modulator compound as provided by the compounds of the invention and one or more additional pharmaceutically active agent(s). If a combination of active agents is administered, then they may be administered sequentially or simultaneously, in separate dosage forms or combined in a single dosage form. Accordingly, the present invention also includes pharmaceutical compositions comprising an amount of: (a) a first agent comprising a compound of the present invention or a pharmaceutically acceptable salt of the compound; (b) a second pharmaceutically active agent; and (c) a pharmaceutically acceptable carrier, vehicle or diluent.

Various pharmaceutically active agents may be selected for use in conjunction with the compounds of the present invention, depending on the disease, disorder, or condition to be treated. Pharmaceutically active agents that may be used in combination with the compositions of the present invention include, without limitation:
(i) acetylcholinesterase inhibitors, such as donepezil hydrochloride (ARICEPT, MEMAC), physostigmine salicylate (ANTILIRIUM), physostigmine sulfate (ESERINE), metrifonate, neostigmine, ganstigmine, pyridostigmine (MESTINON), ambenonium (MYTELASE), demarcarium, Debio 9902 (also known as ZT-1; Debiopharm), rivastigmine (EXELON), ladostigil, NP-0361, galantamine hydrobromide (RAZADYNE, RIMINYL, NIVALIN), tacrine (COGNEX), tolserine, velnacrine maleate, memoquin, huperzine A (HUP-A; NeuroHitech), phenserine, edrophonium (ENLON, TENSILON), and INM-176;
(ii) amyloid-β (or fragments thereof), such as Aβ₁₋₁₅ conjugated to pan HLA DR-binding epitope (PADRE), ACC-001 (Elan/Wyeth), ACI-01, ACI-24, AN-1792, Affitope AD-01, CAD106, and V-950;
(iii) antibodies to amyloid-β (or fragments thereof), such as ponezumab, solanezumab, bapineuzumab (also known as AAB-001), AAB-002 (Wyeth/Elan), ACI-01-Ab7, BAN-2401, intravenous Ig (GAMMAGARD), LY2062430 (humanized m266; Lilly), R1450 (Roche), ACU-5A5, huC091, and those disclosed in International Patent Publication Nos WO04/032868, WO05/025616, WO06/036291, WO06/069081, WO06/118959, in US Patent Publication Nos US2003/0073655, US2004/0192898, US2005/0048049, US2005/0019328, in European Patent Publication Nos EP0994728 and 1257584, and in US Patent No 5,750,349;
(iv) amyloid-lowering or -inhibiting agents (including those that reduce amyloid production, accumulation and fibrillization) such as dimebon, davunetide, eprodisate, leuprolide, SK-PC-B70M, celecoxib, lovastatin, anapsos, oxiracetam, pramiracetam, varenicline, nicergoline, colostrinin, bisnorcymserine (also known as BNC), NIC5-15 (Humanetics), E-2012 (Eisai), pioglitazone, clioquinol (also known as PBT1), PBT2 (Prana Biotechnology), flurbiprofen (ANSAID, FROBEN) and its R-enantiomer tarenflurbil (FLURIZAN), nitroflurbiprofen, fenoprofen (FENOPRON, NALFON), ibuprofen (ADVIL, MOTRIN, NUROFEN), ibuprofen lysinate, meclofenamic acid, meclofenamate sodium (MECLOMEN), indomethacin (INDOCIN), diclofenac sodium (VOLTAREN), diclofenac potassium, sulindac (CLINORIL), sulindac sulfide, diflunisal (DOLOBID), naproxen (NAPROSYN), naproxen sodium (ANAPROX, ALEVE), ARC031 (Archer Pharmaceuticals), CAD-106 (Cytos), LY450139 (Lilly), insulin-degrading enzyme (also known as insulysin), the gingko biloba extract EGb-761 (ROKAN, TEBONIN), tramiprosate (CEREBRIL, ALZHEMED), eprodisate (FIBRILLEX, KIACTA), compound W (3,5-bis(4-nitrophenoxy)benzoic acid), NGX-96992, neprilysin (also known as neutral endopeptidase (NEP)), scyllo-inositol (also known as scyllitol), atorvastatin (LIPITOR), simvastatin (ZOCOR), KLVFF-(EEX)3, SKF-74652, ibutamoren mesylate, BACE inhibitors such as ASP-1702, SCH-745966, JNJ-715754, AMG-0683, AZ-12304146, BMS-782450, GSK-188909, NB-533, E2609 and TTP-854; gamma secretase modulators such as ELND-007; and RAGE (receptor for advanced glycation end-products) inhibitors, such as TTP488 (Transtech) and TTP4000 (Transtech), and those disclosed in US Patent No 7,285,293, including PTI-777;
(v) alpha-adrenergic receptor agonists, such as guanfacine (INTUNIV, TENEX), clonidine (CATAPRES), metaraminol (ARAMINE), methyldopa (ALDOMET, DOPAMET, NOVOMEDOPA), tizanidine (ZANAFLEX), phenylephrine (also known as neosynephrine), methoxamine, cirazoline, guanfacine (INTUNIV), lofexidine, xylazine, modafinil (PROVIGIL), adrafinil, and armodafinil (NUVIGIL);
(vi) beta-adrenergic receptor blocking agents (beta blockers), such as carteolol, esmolol (BREVIBLOC), labetalol (NORMODYNE, TRANDATE), oxprenolol (LARACOR, TRASACOR), pindolol (VISKEN), propanolol (INDERAL), sotalol (BETAPACE, SOTALEX, SOTACOR), timolol (BLOCADREN, TIMOPTIC), acebutolol (SECTRAL, PRENT), nadolol (CORGARD), metoprolol tartrate (LOPRESSOR), metoprolol succinate (TOPROL-XL), atenolol (TENORMIN), butoxamine, and SR 59230A (Sanofi);
(vii) anticholinergics, such as amitriptyline (ELAVIL, ENDEP), butriptyline, benztropine mesylate (COGENTIN), trihexyphenidyl (ARTANE), diphenhydramine (BENADRYL), orphenadrine (NORFLEX), hyoscyamine, atropine (ATROPEN), scopolamine (TRANSDERM-SCOP), scopolamine methylbromide (PARMINE), dicycloverine (BENTYL, BYCLOMINE, DIBENT, DILOMINE), tolterodine (DETROL), oxybutynin (DITROPAN, LYRINEL XL, OXYTROL), penthienate bromide, propantheline (PRO-BANTHINE), cyclizine, imipramine hydrochloride (TOFRANIL), imipramine maleate (SURMONTIL), lofepramine, desipramine (NORPRAMIN), doxepin (SINEQUAN, ZONALON), trimipramine (SURMONTIL), and glycopyrrolate (ROBINUL);
(viii) anticonvulsants, such as carbamazepine (TEGRETOL, CARBATROL), oxcarbazepine (TRILEPTAL), phenytoin sodium (PHENYTEK), fosphenytoin (CEREBYX, PRODILANTIN), divalproex sodium (DEPAKOTE), gabapentin (NEURONTIN), pregabalin (LYRICA), topirimate (TOPAMAX), valproic acid (DEPAKENE), valproate sodium (DEPACON), 1-benzyl-5-bromouracil, progabide, beclamide, zonisamide (TRERIEF, EXCEGRAN), CP-465022, retigabine, talampanel, and primidone (MYSOLINE);
(ix) antipsychotics, such as lurasidone (LATUDA, also known as SM-13496; Dainippon Sumitomo), aripiprazole (ABILIFY), chlorpromazine (THORAZINE), haloperidol (HALDOL), iloperidone (FANAPTA), flupentixol decanoate (DEPIXOL, FLUANXOL), reserpine (SERPLAN), pimozide (ORAP), fluphenazine decanoate, fluphenazine hydrochloride, prochlorperazine (COMPRO), asenapine (SAPHRIS), loxapine (LOXITANE), molindone (MOBAN), perphenazine, thioridazine, thiothixine, trifluoperazine (STELAZINE), ramelteon, clozapine (CLOZARIL), norclozapine (ACP-104), risperidone (RISPERDAL), paliperidone (INVEGA), melperone, olanzapine (ZYPREXA), quetiapine (SEROQUEL), talnetant, amisulpride, ziprasidone (GEODON), blonanserin (LONASEN), and ACP-103 (Acadia Pharmaceuticals);
(x) calcium channel blockers such as lomerizine, ziconotide, nilvadipine (ESCOR, NIVADIL), diperdipine, amlodipine (NORVASC, ISTIN, AMLODIN), felodipine (PLENDIL), nicardipine (CARDENE), nifedipine (ADALAT, PROCARDIA), MEM 1003 and its parent compound nimodipine (NIMOTOP), nisoldipine (SULAR), nitrendipine, lacidipine (LACIPIL, MOTENS), lercanidipine (ZANIDIP), lifarizine, diltiazem (CARDIZEM), verapamil (CALAN, VERELAN), AR-R 18565 (AstraZeneca), and enecadin;
(xi) catechol O-methyltransferase (COMT) inhibitors, such as nitecapone, tolcapone (TASMAR), entacapone (COMTAN), and tropolone;
(xii) central nervous system stimulants, such as atomoxetine, reboxetine, yohimbine, caffeine, phenmetrazine, phendimetrazine, pemoline, fencamfamine (GLUCOENERGAN, REACTIVAN), fenethylline (CAPTAGON), pipradol (MERETRAN), deanol (also known as dimethylaminoethanol), methylphenidate (DAYTRANA), methylphenidate hydrochloride (RITALIN), dexmethylphenidate (FOCALIN), amphetamine (alone or in combination with other CNS stimulants, e.g. ADDERALL (amphetamine aspartate, amphetamine sulfate, dextroamphetamine saccharate, and dextroamphetamine sulfate)), dextroamphetamine sulfate (DEXEDRINE, DEXTROSTAT), methamphetamine (DESOXYN), lisdexamfetamine (VYVANSE), and benzphetamine (DIDREX);
(xiii) corticosteroids, such as prednisone (STERAPRED, DELTASONE), prednisolone (PRELONE), prednisolone acetate (OMNIPRED, PRED MILD, PRED FORTE), prednisolone sodum phosphate (ORAPRED ODT), methylprednisolone (MEDROL); methylprednisolone acetate (DEPO-MEDROL), and methylprednisolone sodium succinate (A-METHAPRED, SOLU-MEDROL);
(xiv) dopamine receptor agonists, such as apomorphine (APOKYN), bromocriptine (PARLODEL), cabergoline (DOSTINEX), dihydrexidine, dihydroergocryptine, fenoldopam (CORLOPAM), lisuride (DOPERGIN), terguride spergolide (PERMAX), piribedil (TRIVASTAL, TRASTAL), pramipexole (MIRAPEX), quinpirole, ropinirole (REQUIP), rotigotine (NEUPRO), SKF-82958 (GlaxoSmithKline), cariprazine, pardoprunox and sarizotan;
(xv) dopamine receptor antagonists, such as chlorpromazine, fluphenazine, haloperidol, loxapine, risperidone, thioridazine, thiothixene, trifluoperazine, tetrabenazine (NITOMAN, XENAZINE), 7-hydroxyamoxapine, droperidol (INAPSINE, DRIDOL, DROPLETAN), domperidone (MOTILIUM), L-741742, L-745870, raclopride, SB-277011A, SCH-23390, ecopipam, SKF-83566, and metoclopramide (REGLAN);
(xvi) dopamine reuptake inhibitors such as bupropion, safinamide, nomifensine maleate (MERITAL), vanoxerine (also known as GBR-12909) and its decanoate ester DBL-583, and amineptine;
(xvii) gamma-aminobutyric acid (GABA) receptor agonists, such as baclofen (LIORESAL, KEMSTRO), siclofen, pentobarbital (NEMBUTAL), progabide (GABRENE), and clomethiazole;
(xviii) histamine 3 (H3) antagonists such as ciproxifan, tiprolisant, S-38093, irdabisant, pitolisant, GSK-239512, GSK-207040, JNJ-5207852, JNJ-17216498, HPP-404, SAR-110894, trans-*N*-ethyl-3-fluoro-3-[3-fluoro-4-(pyrrolidin-1-ylmethyl)phenyl]cyclobutanecarboxamide (PF-3654746 and those disclosed in US Patent Publication Nos US2005-0043354, US2005-0267095, US2005-0256135, US2008-0096955, US2007-1079175, and US2008-0176925; International Patent Publication Nos WO2006/136924, WO2007/063385, WO2007/069053, WO2007/088450, WO2007/099423, WO2007/105053, WO2007/138431, and WO2007/088462; and US Patent No 7,115,600);
(xix) immunomodulators such as glatiramer acetate (also known as copolymer-1; COPAXONE), MBP-8298 (synthetic myelin basic protein peptide), dimethyl fumarate, fingolimod (also known as FTY720), roquinimex (LINOMIDE), laquinimod (also known as ABR-215062 and SAIK-MS), ABT-874 (human anti-IL-12 antibody; Abbott), rituximab (RITUXAN), alemtuzumab (CAMPATH), daclizumab (ZENAPAX), and natalizumab (TYSABRI);
(xx) immunosuppressants such as methotrexate (TREXALL, RHEUMATREX), mitoxantrone (NOVANTRONE), mycophenolate mofetil (CELLCEPT), mycophenolate sodium (MYFORTIC), azathioprine (AZASAN, IMURAN), mercaptopurine (PURI-NETHOL), cyclophosphamide (NEOSAR, CYTOXAN), chlorambucil (LEUKERAN), cladribine (LEUSTATIN, MYLINAX), alpha-fetoprotein, etanercept (ENBREL), and 4-(benzyloxy)-5-[(5-undecyl-2*H*-pyrrol-2-ylidene)methyl]-1*H*,1'*H*-2,2'-bipyrrole (also known as PNU-156804);
(xxi) interferons, including interferon beta-1a (AVONEX, REBIF) and interferon beta-1b (BETASERON, BETAFERON);
(xxii) levodopa (or its methyl or ethyl ester), alone or in combination with a DOPA decarboxylase inhibitor (e.g., carbidopa (SINEMET, CARBILEV, PARCOPA), benserazide (MADOPAR), α-methyldopa, monofluoromethyldopa, difluoromethyldopa, brocresine, or m-hydroxybenzylhydrazine);
(xxiii) *N*-methyl-D-aspartate (NMDA) receptor antagonists, such as memantine (NAMENDA, AXURA, EBIXA), amantadine (SYMMETREL), acamprosate (CAMPRAL), besonprodil, ketamine (KETALAR), delucemine, dexanabinol, dexefaroxan, dextromethorphan, dextrorphan, traxoprodil, CP-283097, himantane, idantadol, ipenoxazone, L-701252 (Merck), lancicemine, levorphanol (DROMORAN), LY-233536 and LY-235959 (both Lilly), methadone, (DOLOPHINE), neramexane, perzinfotel, phencyclidine, tianeptine (STABLON), dizocilpine (also known as MK-801), EAB-318 (Wyeth), ibogaine, voacangine, tiletamine, riluzole (RILUTEK), aptiganel (CERES0TAT), gavestinel, and remacimide;
(xxiv) monoamine oxidase (MAO) inhibitors, such as selegiline (EMSAM), selegiline hydrochloride (L-deprenyl, ELDEPRYL, ZELAPAR), desmethylselegiline, brofaromine, phenelzine (NARDIL), tranylcypromine (PARNATE), moclobemide (AURORIX, MANERIX), befloxatone, safinamide, isocarboxazid (MARPLAN), nialamide (NIAMID), rasagiline (AZILECT), iproniazid (MARSILID, IPROZID, IPRONID), CHF-3381 (Chiesi Farmaceutici), iproclozide, toloxatone (HUMORYL, PERENUM), bifemelane, desoxypeganine, harmine (also known as telepathine or banasterine), harmaline, linezolid (ZYVOX, ZYVOXID), and pargyline (EUDATIN, SUPIRDYL);
(xxv) muscarinic receptor (particularly M1 subtype) agonists, such as cevimeline, levetiracetam, bethanechol chloride (DUVOID, URECHOLINE), itameline, pilocarpine (SALAGEN), NGX267, arecoline, L-687306 (Merck), L-689660 (Merck), furtrethonium iodide (FURAMON, FURANOL), furtrethonium benzensulfonate, furtrethonium p-toluenesulfonate, McN-A-343, oxotremorine, sabcomeline, AC-90222 (Acadia Pharmaceuticals), and carbachol (CARBASTAT, MIOSTAT, CARBOPTIC);
(xxvi) neuroprotective drugs such as bosutinib, condoliase, airmoclomol, lamotrigine, perampanel, aniracetam, minaprime, 2,3,4,9-tetrahydro-1*H*-carbazol-3-one oxime, desmoteplase, anatibant, astaxanthin, neuropeptide NAP (e.g., AL-108 and AL-208; both Allon Therapeutics), neurostrol, perampenel, ispronicline, bis(4-β-D-glucopyranosyloxybenzyl)-2-β-D-glucopyranosyl-2-isobutyltartrate (also known as dactylorhin B or DHB), formobactin, xaliproden (XAPRILA), lactacystin, dimeboline hydrochloride (DIMEBON), disufenton (CEROVIVE), arundic acid (ONO-2506, PROGLIA, CEREACT), citicoline (also known as cytidine 5'-diphosphocholine), edaravone (RADICUT), AEOL-10113 and AEOL-10150 (both Aeolus Pharmaceuticals), AGY-94806 (also known as SA-450 and Msc-1), granulocyte-colony stimulating factor (also known as AX-200), BAY-38-7271 (also known as KN-387271; Bayer AG), ancrod (VIPRINEX, ARWIN), DP-b99 (D-Pharm Ltd), HF-0220 (17-β-hydroxyepiandrosterone; Newron Pharmaceuticals), HF-0420 (also known as oligotropin), pyridoxal 5'-phosphate (also known as MC-1), microplasmin, S-18986, piclozotan, NP031112, tacrolimus, L-seryl-L-methionyl-L-alanyl-L-lysyl-L-glutamyl-glycyl-L-valine, AC-184897 (Acadia Pharmaceuticals), ADNF-14 (National Institutes of Health), stilbazulenyl nitrone, SUN-N8075 (Daiichi Suntory Biomedical Research), and zonampanel;
(xxvii) nicotinic receptor agonists, such as epibatidine, bupropion, CP-601927, varenicline, ABT-089 (Abbott), ABT-594, AZD-0328 (AstraZeneca), EVP-6124, R3487 (also known as MEM3454; Roche/Memory Pharmaceuticals), R4996 (also known as MEM63908; Roche/Memory Pharmaceuticals), TC-4959 and TC-5619 (both Targacept), and RJR-2403;
(xxviii) norepinephrine (noradrenaline) reuptake inhibitors, such as atomoxetine (STRATTERA), doxepin (APONAL, ADAPIN, SINEQUAN), nortriptyline (AVENTYL, PAMELOR, NORTRILEN), amoxapine (ASENDIN, DEMOLOX, MOXIDIL), reboxetine (EDRONAX, VESTRA), viloxazine (VIVALAN), maprotiline (DEPRILEPT, LUDIOMIL, PSYMION), bupropion (WELLBUTRIN), and radaxafine;
(xxix) phosphodiesterase (PDE) inhibitors, including but not limited to, (a) PDE1 inhibitors (e.g., vinpocetine (CAVINTON, CERACTIN, INTELECTOL) and those disclosed in US Patent No 6,235,742), (b) PDE2 inhibitors (e.g., erythro-9-(2-hydroxy-3-nonyl)adenine (EHNA), BAY 60-7550, and those described in US Patent No. 6,174,884), (c) PDE3 inhibitors (e.g., anagrelide, cilostazol, milrinone, olprinone, parogrelil, and pimobendan), (d) PDE4 inhibitors (e.g., apremilast, ibudilast, roflumilast, rolipram, Ro 20-1724, ibudilast (KETAS), piclamilast (also known as RP73401), CDP840, cilomilast (ARIFLO), roflumilast, tofimilast, oglemilast (also known as GRC 3886), tetomilast (also known as OPC-6535), lirimifast, theophylline (UNIPHYL, THEOLAIR), arofylline (also known as LAS-31025), doxofylline, RPR-122818, or mesembrine), and (e) PDE5 inhibitors (e.g., sildenafil (VIAGRA, REVATIO), tadalafil (CIALIS), vardenafil (LEVITRA, VIVANZA), udenafil, avanafil, dipyridamole (PERSANTINE), E-4010, E-4021, E-8010, zaprinast, iodenafil, mirodenafil, DA-8159, and those disclosed in International Patent Applications WO2002/020521, WO2005/049616, WO2006/120552, WO2006/126081, WO2006/126082, WO2006/126083, and WO2007/122466), (f) PDE7 inhibitors; (g) PDE8 inhibitors; (h) PDE9 inhibitors (e.g., BAY 73-6691 (Bayer AG) and those disclosed in US Patent Publication Nos US2003/0195205, US2004/0220186, US2006/0111372, US2006/0106035, and USSN 12/118,062 (filed May 9, 2008)), (i) PDE10 inhibitors such as 2-({4-[1-methyl-4-(pyridin-4-yl)-1*H*-pyrazol-3-yl]phenoxy}methyl)quinoline (PF-2545920), and SCH-1518291, and (j) PDE11 inhibitors;
(xxx) quinolines, such as quinine (including its hydrochloride, dihydrochloride, sulfate, bisulfate and gluconate salts), chloroquine, sontoquine, hydroxychloroquine (PLAQUENIL), mefloquine (LARIAM), and amodiaquine (CAMOQUIN, FLAVOQUINE);
(xxxi) β-secretase inhibitors, such as ASP-1702, SCH-745966, JNJ-715754, AMG-0683, AZ-12304146, BMS-782450, GSK-188909, NB-533, LY-2886721, E-2609, HPP-854, (+)-phenserine tartrate (POSIPHEN), LSN-2434074 (also known as LY-2434074), KMI-574, SCH-745966, Ac-rER (*N*²-acetyl-D-arginyl-L-arginine), loxistatin (also known as E64d), and CA074Me;
(xxxii) γ-secretase inhibitors and modulators, such as BMS-708163 (Avagacest), WO20060430064 (Merck), DSP8658 (Dainippon), ITI-009, L-685458 (Merck), ELAN-G, ELAN-Z, 4-chloro-*N*-[2-ethyl-1(*S*)-(hydroxymethyl)butyl]benzenesulfonamide;
(xxxiii) serotonin (5-hydroxytryptamine) 1A (5-HT_{1A}) receptor antagonists, such as spiperone, *levo*-pindolol, BMY 7378, NAD-299, S(-)-UH-301, NAN 190, lecozotan;
(xxxiv) serotonin (5-hydroxytryptamine) 2C (5-HT_{2c}) receptor agonists, such as vabicaserin, and zicronapine;
(xxxv) serotonin (5-hydroxytryptamine) 4 (5-HT₄) receptor agonists, such as PRX-03140 (Epix);
(xxxvi) serotonin (5-hydroxytryptamine) 6 (5-HT₆) receptor antagonists, such as A-964324, AVI-101, AVN-211, mianserin (TORVOL, BOLVIDON, NORVAL), methiothepin (also known as metitepine), ritanserin, ALX-1161, ALX-1175, MS-245, LY-483518 (also known as SGS518; Lilly), MS-245, Ro 04-6790, Ro 43-68544, Ro 63-0563, Ro 65-7199, Ro 65-7674, SB-399885, SB-214111, SB-258510, SB-271046, SB-357134, SB-699929, SB-271046, SB-742457 (GlaxoSmithKline), Lu AE58054 (Lundbeck A/S), and PRX-07034 (Epix);
(xxxvii) serotonin (5-HT) reuptake inhibitors such as alaproclate, citalopram (CELEXA, CIPRAMIL), escitalopram (LEXAPRO, CIPRALEX), clomipramine (ANAFRANIL), duloxetine (CYMBALTA), femoxetine (MALEXIL), fenfluramine (PONDIMIN), norfenfluramine, fluoxetine (PROZAC), fluvoxamine (LUVOX), indalpine, milnacipran (IXEL), paroxetine (PAXIL, SEROXAT), sertraline (ZOLOFT, LUSTRAL), trazodone (DESYREL, MOLIPAXIN), venlafaxine (EFFEXOR), zimelidine (NORMUD, ZELMID), bicifadine, desvenlafaxine (PRISTIQ), brasofensine, vilazodone, cariprazine, neuralstem and tesofensine;
(xxxviii) trophic factors, such as nerve growth factor (NGF), basic fibroblast growth factor (bFGF; ERSOFERMIN), neurotrophin-3 (NT-3), cardiotrophin-1, brain-derived neurotrophic factor (BDNF), neublastin, meteorin, and glial-derived neurotrophic factor (GDNF), and agents that stimulate production of trophic factors, such as propentofylline, idebenone, PYM50028 (COGANE; Phytopharm), and AIT-082 (NEOTROFIN);
(xxxix) Glycine transporter-1 inhibitors such as paliflutine, ORG-25935, JNJ-17305600, and ORG-26041;
(xl) AMPA-type glutamate receptor modulators such as perampanel, mibampator, selurampanel, GSK-729327, and *N*-{(3*S*,4*S*)-4-[4-(5-cyanothiophen-2-yl)phenoxy] tetrahydrofuran-3-yl}propane-2-sulfonamide;
and the like.

The present invention further comprises kits that are suitable for use in performing the methods of treatment described above. In one embodiment, the kit contains a first dosage form comprising one or more of the compounds of the present invention and a container for the dosage, in quantities sufficient to carry out the methods of the present invention.

In another embodiment, the kit of the present invention comprises one or more compounds of the invention.

The compounds of the present invention, or their pharmaceutically acceptable salts, may be prepared by the methods described below, together with synthetic methods known in the art of organic chemistry, or modifications and derivatizations that are familiar to those of ordinary skill in the art. The starting materials used herein are commercially available or may be prepared by routine methods known in the art [such as those methods disclosed in standard reference books such as the COMPENDIUM OF ORGANIC SYNTHETIC METHODS, Vol. I-XII (published by Wiley-Interscience)]. Preferred methods include, but are not limited to, those described below.

During any of the following synthetic sequences, it may be necessary and/or desirable to protect sensitive or reactive groups on any of the molecules concerned. This can be achieved by means of conventional protecting groups, such as those described in T. W. Greene, Protective Groups in Organic Chemistry, John Wiley & Sons, 1981; T. W. Greene and P. G. M. Wuts, Protective Groups in Organic Chemistry, John Wiley & Sons, 1991; and T. W. Greene and P. G. M. Wuts, Protective Groups in Organic Chemistry, John Wiley & Sons, 1999, which are hereby incorporated by reference.

Compounds of the present invention, or their pharmaceutically acceptable salts, can be prepared according to the reaction Schemes discussed herein below. Unless otherwise indicated, the substituents in the Schemes are defined as above. Isolation and purification of the products is accomplished by standard procedures, which are known to a chemist of ordinary skill.

### Experimental Procedures and Working Examples

The following illustrate the synthesis of various compounds of the present invention.

It will be understood that the intermediate compounds of the invention depicted above are not limited to the particular enantiomer shown, but also include all stereoisomers and mixtures thereof.

### Experimental Procedures

Experiments were generally carried out under inert atmosphere (nitrogen or argon), particularly in cases where oxygen- or moisture-sensitive reagents or intermediates were employed. Commercial solvents and reagents were generally used without further purification, including anhydrous solvents where appropriate (generally Sure-Seal™ products from the Aldrich Chemical Company, Milwaukee, Wisconsin). Products were generally dried under vacuum before being carried on to further reactions or submitted for biological testing. Mass spectrometry data is reported from either liquid chromatography-mass spectrometry (LCMS), atmospheric pressure chemical ionization (APCI) or gas chromatography-mass spectrometry (GCMS) instrumentation. Chemical shifts for nuclear magnetic resonance (NMR) data are expressed in parts per million (ppm, δ) referenced to residual peaks from the deuterated solvents employed.

For syntheses referencing procedures in other Examples or Methods, reaction conditions (length of reaction and temperature) may vary. In general, reactions were followed by thin layer chromatography or mass spectrometry, and subjected to work-up when appropriate. Purifications may vary between experiments: in general, solvents and the solvent ratios used for eluents / gradients were chosen to provide appropriate R_{f}s or retention times.

### Preparations

### Preparation 1: 5-(4-Methyl-1H-imidazol-1-yl)-6-oxo-1,6-dihydropyridine-2-carboxylic acid, hydrobromide salt (P1)

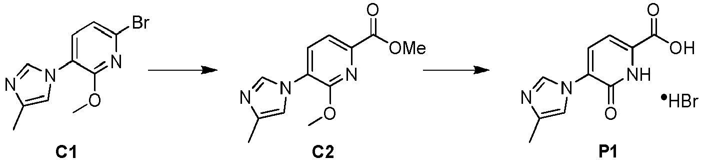

Step 1. Synthesis of methyl 6-methoxy-5-(4-methyl-1*H*-imidazol-1-yl)pyridine-2-carboxylate (**C2**). To a solution of the known 6-bromo-2-methoxy-3-(4-methyl-1*H-*imidazol-1-yl)pyridine (**C1**, T. Kimura et al., *U.S. Pat. Appl. Publ.* **2009,** US 20090062529 A1) (44.2 g, 165 mmol) in methanol (165 mL) was added triethylamine (46 mL, 330 mmol) and [1,1'-bis(diphenylphosphino)ferrocene]dichloropalladium(II), dichloromethane complex (6.7 g, 8.2 mmol). The mixture was degassed several times with nitrogen. The reaction was heated to 70° C under CO atmosphere (3 bar) in a Parr apparatus. After 30 min, the pressure dropped to 0.5 bar; additional CO was added until the pressure stayed constant for a period of 30 min. The mixture was allowed to cool to room temperature and filtered through a pad of Celite. The Celite pad was washed twice with methanol and the combined filtrates were concentrated under reduced pressure. The residue (88 g) was dissolved in ethyl acetate (1 L) and water (700 mL), and the layers were separated. The organic layer was washed with water (200 mL), and the aqueous layer was extracted with ethyl acetate (500 mL). The combined organic layers were dried over magnesium sulfate, filtered and concentrated to provide the title compound. Yield: 42.6 g, quantitative.

Step 2. Synthesis of 5-(4-methyl-1*H*-imidazol-1-yl)-6-oxo-1,6-dihydropyridine-2-carboxylic acid, hydrobromide salt (**P1**). A solution of methyl 6-methoxy-5-(4-methyl-1*H*-imidazol-1-yl)pyridine-2-carboxylate (**C2**) (3.82 g, 15.9 mmol) in acetic acid (30 mL) and aqueous hydrobromic acid (48%, 30 mL) was heated at reflux for 4 h. The reaction was allowed to cool to room temperature, and then chilled in an ice bath; the resulting precipitate was collected via filtration and washed with ice water (30 mL). Recrystallization from ethanol (20 mL) provided the title compound as a light yellow solid. Yield: 3.79 g, 12.6 mmol, 79%. LCMS *m*/*z* 220.1 (M+1); ¹H NMR (400 MHz, DMSO-*d₆*) δ 2.34 (br s, 3H), 7.09 (d, *J*=7.4 Hz, 1H), 7.88-7.91 (m, 1H), 8.07 (d, *J*=7.6 Hz, 1H), 9.58-9.60 (m, 1H), 12.6 (br s, 1H).

### Preparation 2: 5-(4-Methyl-1H-imidazol-1-yl)-6-oxo-1,6-dihydropyridine-2-carboxylic acid, hydrochloride salt (P2)

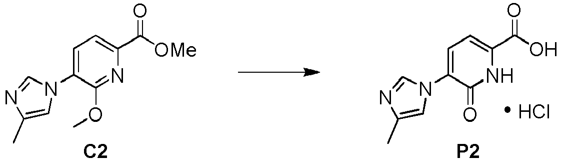

A mixture of **C2** (12.8 g, 51.8 mmol) and 37% hydrochloric acid (25 mL) was heated at reflux for 18 h. After the reaction mixture had cooled to room temperature, the solid was collected via filtration; it was stirred with 1,4-dioxane (2 x 20 mL) and filtered again, to afford the product as a yellow solid. Yield: 13 g, 51 mmol, 98%. ¹H NMR (400 MHz, CD₃OD) δ 9.52 (br s, 1H), 8.07 (d, *J*=7.5 Hz, 1H), 7.78 (br s, 1H), 7.21 (d, *J*=7.5 Hz, 1H), 2.44 (s, 3H).

### Preparation 3: 7-(4-Methyl-1H-imidazol-1-yl)-3,4-dihydropyrido[2,1-c][1,]4oxazine-1,6-dione (P3)

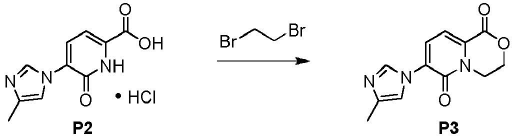

Compound **P2** (65 g, 250 mmol), 1,2-dibromoethane (52.5 g, 280 mmol) and cesium carbonate (124 g, 381 mmol) were combined in *N,N*-dimethylformamide (850 mL) and heated at 90 °C for 6 h. The reaction mixture was then cooled and filtered through Celite. After concentration of the filtrate under reduced pressure, the residue was dissolved in dichloromethane (500 mL), washed with brine (100 mL), washed with water (50 mL), dried over magnesium sulfate, filtered, and concentrated under reduced pressure. The resulting solid was washed with acetonitrile to provide the title compound. Yield: 46.5 g, 190 mmol, 76%. ¹H NMR (400 MHz, CDCl₃) δ 8.33 (d, *J*=1.4 Hz, 1H), 7.43 (AB quartet, *J*_{AB}=7.7 Hz, Δv_{AB}=33.4 Hz, 2H), 7.15-7.17 (m, 1H), 4.66-4.70 (m, 2H), 4.38-4.42 (m, 2H), 2.30 (d, *J*=0.8 Hz, 3H).

### Preparation 4: 2-(3-Bromobenzyl)-7-(4-methyl-1H-imidazol-1-yl)-3,4-dihydro-2H-pyrido[1,2-a]pyrazine-1,6-dione (P4)

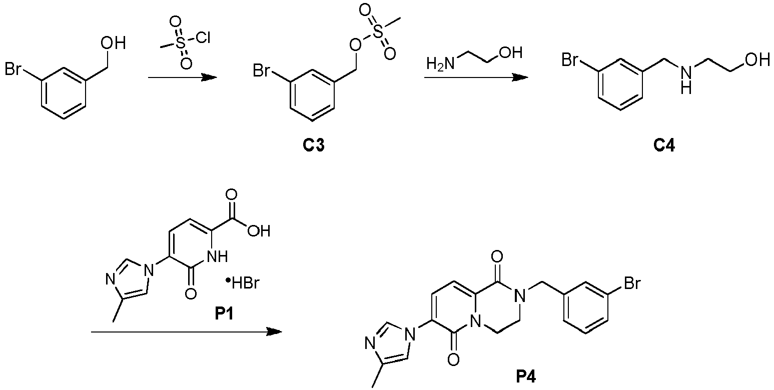

Step 1. Synthesis of 3-bromobenzyl methanesulfonate (**C3**). To a solution of 3-bromobenzyl alcohol (3.00 g, 16.0 mmol) and triethylamine (2.91 mL, 20.9 mmol) in dichloromethane (50 mL) at 0 °C was added methanesulfonyl chloride drop-wise. After 2 h, the reaction mixture was washed with saturated aqueous sodium bicarbonate solution and water. The organic layer was dried over magnesium sulfate, filtered, and concentrated under reduced pressure to afford the title compound as a light yellow oil. Yield: 4.3 g, 16 mmol, 100%. ¹H NMR (400 MHz, CDCl₃) δ 2.99 (s, 3H), 5.21 (s, 2H), 7.30 (dd, *J*=7.8, 7.7 Hz, 1H), 7.34-7.38 (m, 1H), 7.52-7.56 (m, 1H), 7.57-7.59 (m, 1H).

Step 2. Synthesis of 2-[(3-bromobenzyl)amino]ethanol (**C4**). A solution of 3-bromobenzyl methanesulfonate (**C3**) (4.25 g, 16.0 mmol) and 2-aminoethanol (9.79 g, 160 mmol) in 2-propanol (30 mL) was heated to 70 °C for 4 h. The reaction mixture was cooled to room temperature and concentrated under reduced pressure. The resulting residue was taken up in ethyl acetate and washed with saturated aqueous sodium bicarbonate solution and with water. The organic layer was dried over magnesium sulfate, filtered, and concentrated under reduced pressure to afford the title compound as a gum. Yield: 3.7 g, 16 mmol, 100%. ¹H NMR (400 MHz, CDCl₃) δ 2.81 (dd, *J*=5.5, 4.9 Hz, 2H), 3.68 (dd, *J*=5.3, 5.1 Hz, 2H), 3.80 (s, 2H), 7.20 (dd, *J*=7.8, 7.6 Hz, 1H), 7.24-7.27 (m, 1H, assumed; partially obscured by solvent peak), 7.38-7.42 (m, 1H), 7.49 (br s, 1H).

Step 3. Synthesis of 2-(3-bromobenzyl)-7-(4-methyl-1*H*-imidazol-1-yl)-3,4-dihydro-2*H-*pyrido[1,2-*a*]pyrazine-1,6-dione (**P4**). A mixture of 5-(4-methyl-1*H*-imidazol-1-yl)-6-oxo-1,6-dihydropyridine-2-carboxylic acid, hydrobromide salt (**P1**) (1.4 g, 4.7 mmol), 2-[(3-bromobenzyl)amino]ethanol (**C4**) (1.61 g, 7.00 mmol), *O*-(7-azabenzotriazol-1-yl)-*N,N,N',N'*-tetramethyluronium hexafluorophosphate (HATU, 6.08 g, 16 mmol) and *N,N-*diisopropylethylamine (3.30 g, 25.5 mmol) in dichloromethane (70 mL) was stirred for 20 h. The reaction was poured into water, and the aqueous phase was extracted with dichloromethane (2 x 50 mL). The combined organic layers were washed with saturated aqueous sodium bicarbonate solution, dried over sodium sulfate, filtered, and concentrated under reduced pressure. The residue was treated with a mixture of ethyl acetate and petroleum ether (1:3 ratio, 10 mL) and filtered. The collected solid was washed with additional 1:3 ethyl acetate / petroleum ether to afford the title compound as a yellow solid. Yield: 1.7 g, 4.1 mmol, 87%. LCMS *m*/*z* 414.9 (M+1); ¹H NMR (400 MHz, DMSO-*d₆*) δ 2.15 (s, 3H), 3.65-3.72 (m, 2H), 4.22-4.28 (m, 2H), 4.71 (s, 2H), 7.14 (d, *J*=7.8 Hz, 1H), 7.30-7.38 (m, 2H), 7.41 (br s, 1H), 7.50 (br d, *J*=7.3 Hz, 1H), 7.57 (br s, 1H), 7.80 (d, *J*=7.8 Hz, 1H), 8.25 (br s, 1H).

### Preparation 5: 2-(2-Bromobenzyl)-7-(4-methyl-1H-imidazol-1-yl)-3,4-dihydro-2H-pyrido[1,2-a]pyrazine-1,6-dione (P5)

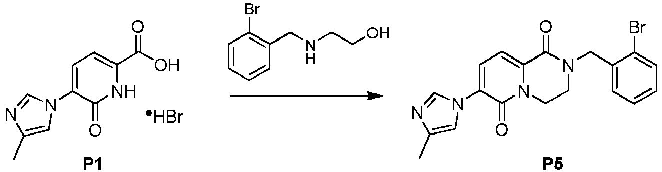

5-(4-Methyl-1*H*-imidazol-1-yl)-6-oxo-1,6-dihydropyridine-2-carboxylic acid, hydrobromide salt (**P1**) (1.5 g, 5.0 mmol) was reacted with 2-[(2-bromobenzyl)amino]ethanol (see R. Gosain et al., Tetrahedron 2001, 57, 1399-1410) (1.72 g, 7.53 mmol) according to the method described for the synthesis of 2-(3-bromobenzyl)-7-(4-methyl-1*H*-imidazol-1-yl)-3,4-dihydro-2*H*-pyrido[1,2-*a*]pyrazine-1,6-dione (**P4**) in Preparation 4. The title compound was obtained as a yellow solid. Yield: 1.63 g, 3.94 mmol, 79%. LCMS *m*/*z* 414.9 (M+1); ¹H NMR (400 MHz, DMSO-*d₆*) δ 2.16 (s, 3H), 3.68-3.76 (m, 2H), 4.27-4.35 (m, 2H), 4.75 (s, 2H), 7.13 (d, *J*=7.5 Hz, 1H), 7.23-7.31 (m, 1H), 7.35-7.44 (m, 3H), 7.67 (br d, *J*=8.0 Hz, 1H), 7.81 (d, *J*=8.0 Hz, 1H), 8.27 (br s, 1H).

### Preparation 6: 2-[(1S)-1-(6-Bromopyridin-2-yl)ethyl]-7-(4-methyl-1H-imidazol-1-yl)-3,4-dihydro-2H-pyrido[1,2-a]pyrazine-1,6-dione (P6)

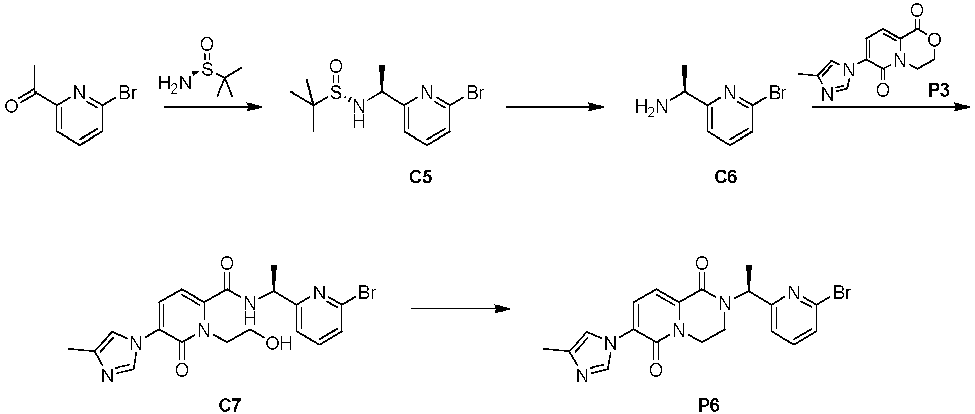

Step 1: Synthesis of *N*-[(1*S*)-1-(6-bromopyridin-2-yl)ethyl]-2-methylpropane-2-(*S*)-sulfinamide (**C5**). To a solution of 1-(6-bromopyridin-2-yl)ethanone (25 g, 125 mmol) in THF (250 mL) was added Ti(OEt)₄ (57 g, 250 mmol) and (*S*)-(-)-2-methyl-2-propanesulfinamide (30.3 g, 250 mmol). The reaction mixture was heated to reflux overnight, whereupon it was allowed to cool to room temperature and stirred for 30 min. The reaction was cooled to -30 °C (internal temperature) and sodium borohydride (9.5 g, 250 mmol) was added. The reaction mixture was stirred at 4 °C for 2 h and then quenched by drop-wise addition of water. The mixture was filtered through Celite, and the solids were washed with dichloromethane. The organic layer was washed with brine, dried over sodium sulfate, and concentrated under reduced pressure. The resulting solid was washed with petroleum ether / ethyl acetate (2:1) to afford the title compound as a light yellow solid. Yield: 11 g, 29%. ¹H NMR (400 MHz, CDCl₃) δ 7.52 (app t, *J*=7.8 Hz, 1H), 7.36 (d, *J*=8.0 Hz, 1H), 7.27 (d, *J*=7.5 Hz, 1H), 4.56-4.48 (m, 2H), 1.51 (d, *J*=6.5 Hz, 3H), 1.25 (s, 9H).

Step 2: Synthesis of (1*S*)-1-(6-bromopyridin-2-yl)ethanamine (**C6**). A solution of *N*-[(1*S)-*1-(6-bromopyridin-2-yl)ethyl]-2-methylpropane-2-sulfinamide (**C5**) (15 g, 49 mmol) in methanolic hydrogen chloride (150 mL, 4.0 M) was stirred at room temperature for 2 h. The mixture was concentrated under reduced pressure, and the resulting residue was dissolved in water and a saturated aqueous solution of NaHCO₃ (pH = 8) and extracted with dichloromethane (3 x 300 mL). The organic layer was washed with brine, dried over sodium sulfate and concentrated under reduced pressure. Purification via silica gel chromatography (dichloromethane / methanol = 95:5) afforded the title compound as a golden oil. Yield: 5 g, 51%. ¹H NMR (400 MHz, CDCl₃) δ 7.44 (app t, *J*=7.5 Hz, 1H), 7.27 (d, *J*=8.0 Hz, 1H), 7.21 (d, *J*=7.5 Hz, 1H), 4.08-4.03 (m, 1H), 1.40 (d, *J*=6.5 Hz, 3H).

Step 3: Synthesis of *N*-[(1*S*)-1-(6-bromopyridin-2-yl)ethyl]-1-(2-hydroxyethyl)-5-(4-methyl-1*H*-imidazol-1-yl)-6-oxo-1,6-dihydropyridine-2-carboxamide (**C7**). To a solution of (1*S*)-1-(6-bromopyridin-2-yl)ethanamine (**C6**) (5.4 g, 27 mmol) in toluene (100 mL) was added drop-wise a solution of trimethylaluminum in toluene (16.8 mL, 33.6 mmol, 2.0 M) at 0 °C. The reaction was stirred for 45 min at room temperature, whereupon 7-(4-methyl-1*H*-imidazol-1-yl)-3,4-dihydropyrido[2,1-*c*][1,4]oxazine-1,6-dione (**P3**) (5.5 g, 22.4 mmol) was added in one portion. The mixture was stirred at 70 °C overnight, and then cooled to 0 °C and quenched by the addition of water. The mixture was extracted with dichloromethane (3 x 100 mL), and the organic layer was washed with brine, dried over sodium sulfate, and concentrated under reduced pressure. The solid was washed with a mixture of petroleum ether / ethyl acetate (10:1) to afford the title compound as a white solid. Yield: 6 g, 60%. ¹H NMR (400 MHz, CDCl₃) δ 8.56 (d, *J*=7.0 Hz, 1H), 8.00 (s, 1H), 7.60 (app t, *J*=7.5 Hz, 1H), 7.44 (d, *J*=8.0, 1H), 7.30 (d, *J*=7.5 Hz, 1H), 7.26 (d, *J*=8.0, Hz, 1H), 7.05 (s, 1H), 6.45 (d, *J*=7.5 Hz, 1H), 5.77-5.69 (m, 1H), 5.22-5.19 (m, 1H), 4.50-4.42 (m, 1H), 4.39-4.28 (m, 1H), 4.26-4.13 (m, 2H), 2.20 (s, 3H), 1.58 (d, *J*=7.5 Hz, 3H).

Step 4: Synthesis of 2-[(1*S*)-1-(6-bromopyridin-2-yl)ethyl]-7-(4-methyl-1*H*-imidazol-1-yl)-3,4-dihydro-2*H*-pyrido[1,2-*a*]pyrazine-1,6-dione (**P6**). To a suspension of *N*-[(1*S*)-1-(6-bromopyridin-2-yl)ethyl]-1-(2-hydroxyethyl)-5-(4-methyl-1*H*-imidazol-1-yl)-6-oxo-1,6-dihydropyridine-2-carboxamide (**C7**) (6.0 g, 13.4 mmol) in THF (100 mL) was added PPh₃ (8.8 g, 34 mmol) followed by drop-wise addition of diisopropyl azodicarboxylate (DIAD, 6.79 g, 33.6 mmol) at 0 °C. The mixture was stirred at room temperature overnight, whereupon it was concentrated under reduced pressure. Purification via silica gel chromatography (dichloromethane / methanol = 95:5) afforded the title compound as a yellow solid. Yield: 3 g, 52%. ¹H NMR (400 MHz, CDCl₃) δ 8.21 (s, 1H), 7.56 (t, *J*=7.5 Hz, 1H), 7.42 (t, *J*=8.0 Hz, 2H), 7.30 (d, *J*=7.5 Hz, 1H), 7.27 (d, *J*=8.0 Hz, 1H), 7.13 (s, 1H), 5.96 (q, *J*=7.0 Hz, 1H), 4.55-4.49 (m, 1H), 4.21-4.14 (m, 1H), 3.85-3.73 (m, 2H), 2.28 (s, 3H), 1.64 (d, *J*=7.0 Hz, 3H).

### Examples

### Reference Example 1

### 2-{[6-Fluoro-1-methyl-5-(trifluoromethyl)-1H-indol-3-yl]methyl}-7-(4-methyl-1H-imidazol-1-yl)-3,4-dihydro-2H-pyrido[1,2-a]pyrazine-1,6-dione (1)

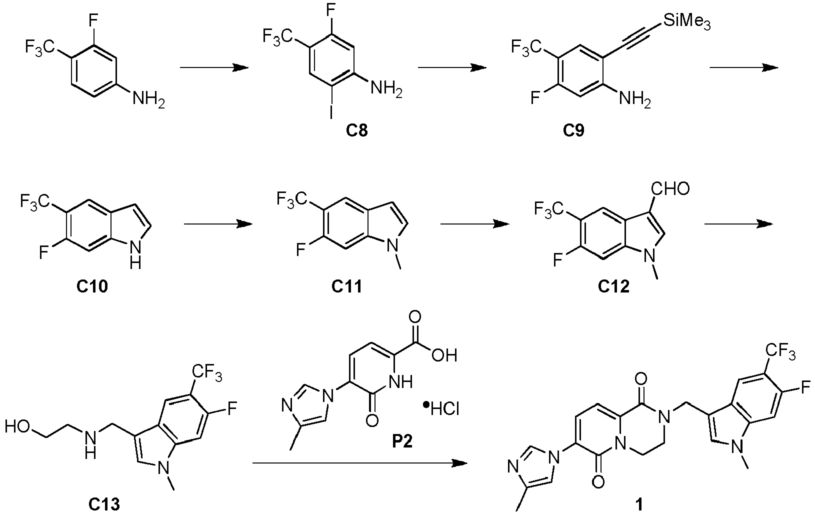

Step 1. Synthesis of 5-fluoro-2-iodo-4-(trifluoromethyl)aniline (C8). To a solution of 3-fluoro-4-(trifluoromethyl)aniline (5.0 g, 28 mmol) in methanol (50 mL) was added a solution of iodine monochloride (9.0 g, 56 mmol) in dichloromethane (100 mL) slowly at 0 °C. After addition, the solution was stirred for 2 h at room temperature, whereupon it was concentrated under reduced pressure. The residue was re-dissolved in dichloromethane (200 mL), washed with a saturated aqueous solution of sodium bicarbonate and with brine, dried over sodium sulfate and concentrated under reduced pressure. Purification via silica gel chromatography eluting with petroleum ether (100%) afforded the title compound. Yield: 4.9 g, 58%. ¹H NMR (400 MHz, CDCl₃) δ 7.76 (d, *J*=8.0 Hz, 1H), 6.59 (d, *J*=13.2 Hz, 1H), 4.92 (br s, 2H).

Step 2. Synthesis of 5-fluoro-4-(trifluoromethyl)-2-[(trimethylsilyl)ethynyl]aniline(**C9**). To a mixture of 5-fluoro-2-iodo-4-(trifluoromethyl)aniline (**C8**) (4.3 g, 14.1 mmol), Pd(dppf)Cl₂ (220 mg, 0.3 mmol), and copper(I) iodide (260 mg, 1.4 mmol) in triethylamine (21 mL) and tetrahydrofuran (21 mL) was added ethynyltrimethylsilane (2 mL) in portions at room temperature. After addition, the mixture was stirred for 1 h at room temperature. The mixture was filtered through Celite and the solids were washed with ethyl acetate (100 mL). The combined filtrate and washings were concentrated under reduced pressure. Purification via silica gel chromatography eluting with petroleum ether (100%) afforded the title compound. Yield: 3.3 g, 85%. GCMS *m*/*z* 243 (M⁺). ¹H NMR (400 MHz, CDCl₃) δ 7.51 (d, *J*=8.0 Hz, 1H), 6.43 (d, *J*=12.0 Hz, 1H), 4.66 (br s, 2H), 0.27 (s, 9H).

Step 3. Synthesis of 6-fluoro-5-(trifluoromethyl)-1*H*-indole (**C10**). A mixture of 5-fluoro-4-(trifluoromethyl)-2-[(trimethylsilyl)ethynyl]aniline (**C9**) (3.2 g, 11.6 mmol) and copper(I) iodide (230 mg, 1.2 mmol) in *N,N*-dimethylformamide (24 mL) was stirred for 20 h at 100 °C. The mixture was filtered through Celite and the solids were washed with ethyl acetate (100 mL). The combined filtrate and washings were diluted with water and extracted with ethyl acetate (3 x 100 mL). The combined organic layers were washed with water and with brine, dried over sodium sulfate, and concentrated under reduced pressure. Purification via silica gel chromatography (Gradient: 0% to 10% ethyl acetate in petroleum ether) afforded the title compound. Yield: 2 g, 84%. ¹H NMR (400 MHz, CDCl₃) δ 7.87 (d, *J*=6.8 Hz, 1H), 7.30-7.28 (m, 1H), 7.18 (d, *J*=10.8 Hz, 1H), 6.60-6.64 (m, 1H).

Step 4. Synthesis of 6-fluoro-1-methyl-5-(trifluoromethyl)-1*H*-indole (C**11**). To a solution of 6-fluoro-5-(trifluoromethyl)-1*H*-indole (**C10**) (500 mg, 2.5 mmol) and potassium carbonate (700 mg, 4.9 mmol) in dry *N,N*-dimethylformamide (5 mL) was added iodomethane (700 mg, 4.9 mmol) in one portion. The mixture was stirred at 50 °C for 20 h, whereupon it was quenched with water and extracted with ethyl acetate (3 x 50 mL). The combined organic layers were washed with water and with brine, dried over sodium sulfate, and concentrated under reduced pressure to give the title compound as a yellow oil. Yield: 443 mg, 83%. ¹H NMR (400 MHz, CDCl₃) δ 7.81 (d, *J*=6.4 Hz, 1H), 7.11-7.06 (m, 2H), 6.52 (d, *J*=2.8 Hz, 1H), 3.76 (s, 3H).

Step 5. Synthesis of 6-fluoro-1-methyl-5-(trifluoromethyl)-1*H*-indole-3-carbaldehyde (**C12**). To a solution of 6-fluoro-1-methyl-5-(trifluoromethyl)-1*H*-indole (**C11**) (534 mg, 2.5 mmol) in dry *N,N*-dimethylformamide (20 mL) was added phosphorus oxychloride (1.1 g, 7.2 mmol) slowly at room temperature. The reaction mixture was stirred for 2 h at room temperature, whereupon it was concentrated under reduced pressure. The residue was diluted with water, and extracted with ethyl acetate (3 x 50 mL). The combined organic layers were washed with water and with brine, dried over sodium sulfate, and concentrated under reduced pressure to give the title compound as a yellow solid, which was used directly in the next step. Yield: 630 mg. ¹H NMR (400 MHz, CDCl₃) δ 9.99 (s, 1H), 8.58 (d, *J*=6.8 Hz, 1H), 7.76 (s, 1H), 7.17 (d, *J*=10.4 Hz, 1H), 3.89 (s, 3H).

Step 6. Synthesis of 2-({[6-fluoro-1-methyl-5-(trifluoromethyl)-1*H*-indol-3-yl]methyl}amino)ethanol (**C13**). A solution of 6-fluoro-1-methyl-5-(trifluoromethyl)-1*H-*indole-3-carbaldehyde (**C12**) (627 mg, 2.5 mmol) and ethanolamine (900 mg, 14.8 mmol) in ethanol (15 mL) was stirred at room temperature for 20 h, whereupon sodium borohydride (300 mg, 7.9 mmol) was added in portions. Upon completion of the reaction as determined by thin layer chromatography (dichloromethane / methanol = 10:1), the solution was diluted with water and extracted with dichloromethane (2 x 30 mL). The combined organic layers were washed with brine, dried over sodium sulfate, and concentrated under reduced pressure to give the title compound. Yield: 410 mg, 56% over two steps. ¹H NMR (400 MHz, CDCl₃) δ 7.87 (d, *J*=6.8 Hz, 1H), 7.07 (d, *J*=11.6 Hz, 1H), 7.07 (s, 1H), 3.98 (s, 2H), 3.75 (s, 3H), 3.69 (t, *J*=5.2 Hz, 2H), 2.87 (t, *J*=5.2 Hz, 2H).

Step 7. Synthesis of 2-{[6-fluoro-1-methyl-5-(trifluoromethyl)-1*H*-indol-3-yl]methyl)-7-(4-methyl-1*H*-imidazol-1-yl)-3,4-dihydro-2*H*-pyrido[1,2-*a*]pyrazine-1,6-dione (**1**). To a mixture of 2-({[6-fluoro-1-methyl-5-(trifluoromethyl)-1*H*-indol-3-yl]methyl}amino)ethanol (**C13**) (400 mg, 1.4 mmol) and 5-(4-methyl-1*H*-imidazol-1-yl)-6-oxo-1,6-dihydropyridine-2-carboxylic acid, hydrochloride salt (**P2**) (352 mg, 1.6 mmol) in dichloromethane (30 mL) was added *N,N*-diisopropylethylamine (542 mg, 4.2 mmol) and *O*-(7-azabenzotriazol-1-yl)-*N,N,N',N'*-tetramethyluronium hexafluorophosphate (HATU, 1.33 g, 3.5 mmol). The mixture was stirred for 20 h at room temperature and then diluted with dichloromethane. The solution was filtered and the filtrate was washed with water and with brine, dried over sodium sulfate, and concentrated under reduced pressure. Purification via silica gel chromatography (Gradient: 0% to 8% methanol in dichloromethane) afforded the title compound as a yellow solid, which was washed with methanol to give pure title compound as a white solid. Yield: 150 mg, 23%. LCMS *m*/*z* 474.0 (M+1); ¹H NMR (400 MHz, CDCl₃) δ 8.24 (s, 1H), 8.07 (d, *J*=7.2 Hz, 1H), 7.80 (d, *J*=8.0 Hz, 1H), 7.65 (d, *J*=12.0 Hz, 1H), 7.63 (s, 1H), 7.40 (s, 1H), 7.16 (d, *J*=7.6 Hz, 1H), 4.85 (s, 2H), 4.18 (t, *J*=5.2 Hz, 2H), 3.80 (s, 3H), 3.62 (t, *J*=5.6 Hz, 2H), 2.15 (s, 3H).

### Example 2

### 2-{[7-Fluoro-1-methyl-5-(trifluoromethyl)-1H-indol-3-yl]methyl}-7-(4-methyl-1H-imidazol-1-yl)-3,4-dihydro-1H-pyrido[1,2-a]pyrazine-1,6(2H)-dione (2)

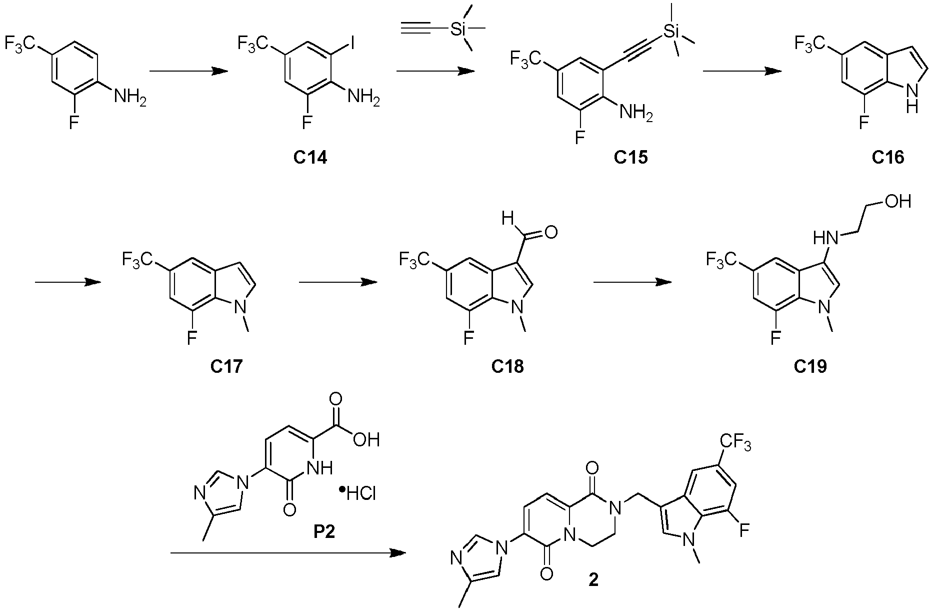

Step 1. Synthesis of 2-fluoro-6-iodo-4-(trifluoromethyl)aniline (**C14**). To a solution of 2-fluoro-4-(trifluoromethyl)aniline (5.1 g, 28.5 mmol) in methanol (25 mL) at 0 °C was slowly added a solution of iodine monochloride (6.95 g, 42.7 mmol) in dichloromethane (25 mL). The reaction was stirred at room temperature for 2 h, and then concentrated under reduced pressure. The residue was dissolved in dichloromethane (200 mL), and washed sequentially with a saturated aqueous solution of sodium thiosulfate, a saturated aqueous solution of sodium bicarbonate and with brine. The organic layer was dried over sodium sulfate, filtered, and concentrated under reduced pressure. Purification via silica gel chromatography (Gradient: 0% to 5% ethyl acetate in *n-*hexanes) afforded the title compound. Yield: 6.5 g, 75%. ¹H NMR (400 MHz, CDCl₃) δ 7.68 (s, 1H), 7.24 (d, *J*= 10.8 Hz, 1H), 4.49 (br s, 2H).

Step 2. Synthesis of 2-fluoro-4-(trifluoromethyl)-6-[(trimethylsilyl)ethynyl]aniline (**C15**). To a mixture of 2-fluoro-6-iodo-4-(trifluoromethyl)aniline (**C14**) (2.3 g, 7.54 mmol), Pd(dppf)Cl₂ (550 mg, 0.75 mmol), and copper(I) iodide (143 mg, 0.75 mmol) in triethylamine (5 mL) and tetrahydrofuran (10 mL) was added portion-wise ethynyltrimethylsilane (887 mg, 9.05 mmol). The reaction mixture was stirred for 1 h at room temperature, whereupon it was filtered through Celite. The solids were washed with ethyl acetate (100 mL) and the combined filtrate and washings were concentrated under reduced pressure. Purification via silica gel chromatography (Gradient: 0% to 3% ethyl acetate in *n*-hexanes) afforded the title compound as a brown oil. Yield: 1.8 g, 81%. ¹H NMR (400 MHz, CDCl₃) δ 7.38 (s, 1H), 7.20 (d, *J*=10.4 Hz, 1H), 4.58 (br s, 2H), 0.29 (s, 9H).

Step 3. Synthesis of 7-fluoro-5-(trifluoromethyl)-1*H*-indole (**C16**). A mixture of 2-fluoro-4-(trifluoromethyl)-6-[(trimethylsilyl)ethynyl]aniline (**C15**) (1.5 g, 5.45 mmol) and copper(I) iodide (104 mg, 0.55 mmol) in *N,N*-dimethylformamide (10 mL) was stirred for 20 h at 100 °C. The mixture was filtered through Celite and the solids were washed with ethyl acetate (100 mL). The combined filtrate and washings were diluted with water, the layers were separated, and the aqueous phase was extracted with ethyl acetate (3 x 100 mL). The combined organic layers were washed with water and with brine, dried over sodium sulfate, and concentrated under reduced pressure. Purification via silica gel chromatography (Gradient: 0% to 10% ethyl acetate in n-hexanes) provided the title compound as a brown oil. Yield: 830 mg, 75%. ¹H NMR (400 MHz, CDCl₃) δ 8.60 (br s, 1H), 7.75 (s, 1H), 7.32-7.38 (m, 1H), 7.17 (d, *J*=10.8 Hz, 1H), 6.66-6.73 (m, 1H).

Step 4. Synthesis of 7-fluoro-1-methyl-5-(trifluoromethyl)-1*H*-indole (**C17**). To a solution of 7-fluoro-5-(trifluoromethyl)-1*H*-indole (**C16**) (830 mg, 4.1 mmol) in *N,N-*dimethylformamide (10 mL) was added sodium hydride (328 mg, 8.2 mmol, 60% in mineral oil) at 0 °C. The reaction was stirred at 0 °C for 30 min, whereupon methyl iodide (873 mg, 6.15 mmol) was added. The reaction mixture was stirred for 2 h at room temperature, and water was then added. The aqueous phase was extracted with ethyl acetate (3 x 50 mL), and the combined organic layers were washed with water and with brine, dried over sodium sulfate, and concentrated under reduced pressure. Purification via silica gel chromatography (Gradient: 0% to 5% ethyl acetate in n-hexanes) gave the title compound. Yield: 800 mg, 90%. ¹H NMR (400 MHz, CDCl₃) δ 7.69 (s, 1H), 7.09-7.12 (m, 2H), 6.57-6.58 (m, 1H), 4.04 (s, 3H).

Step 5. Synthesis of 7-fluoro-1-methyl-5-(trifluoromethyl)-1*H*-indole-3-carbaldehyde (**C18**). To a solution of 7-fluoro-1-methyl-5-(trifluoromethyl)-1*H*-indole (**C17**) (800 mg, 3.7 mmol) in *N,N*-dimethylformamide (10 mL) was added phosphorus oxychloride (1.7 g, 11 mmol) slowly at room temperature. The solution was stirred for 2 h at room temperature and then concentrated under reduced pressure. Water was added, and the aqueous phase was extracted with ethyl acetate (3 x 50 mL). The combined organic layers were washed with water and with brine, dried over sodium sulfate, and concentrated under reduced presure. Purification via silica gel chromatography (20% ethyl acetate in *n*-hexanes) gave the title compound as a white solid. Yield: 700 mg, 78%.
¹H NMR (400 MHz, CDCl₃) δ 10.02 (s, 1H), 8.40 (s, 1H), 7.72 (s, 1H), 7.25 (d, *J*=11.6 Hz, 1H), 4.12 (s, 3H).

Step 6. Synthesis of 2-{[7-fluoro-1-methyl-5-(trifluoromethyl)-1*H*-indol-3-yl]amino}ethanol (**C19**). A solution of 7-fluoro-1-methyl-5-(trifluoromethyl)-1*H*-indole-3-carbaldehyde (**C18**) (500 mg, 2.04 mmol) and ethanolamine (374 mg, 6.12 mmol) in methanol (5 mL) was heated to reflux for 2 h. The solution was then cooled to -20 °C, and sodium borohydride (233 mg, 6.12 mmol) was added. The reaction was stirred at - 20 °C for 30 min, whereupon water was added. The layers were separated and the aqueous phase was extracted with ethyl acetate (3 x 50 mL). The combined organic layers were dried over sodium sulfate, and concentrated under reduced pressure. Purification via silica gel chromatography (Gradient: 0% to 10% methanol in dichloromethane) afforded the title compound as a white solid. Yield: 450 mg, 76%. ¹H NMR (400 MHz, CD₃OD) δ 7.83 (s, 1H), 7.33 (s, 1H), 7.15 (d, *J*=12.4 Hz, 1H), 4.03 (s, 3H), 3.99 (s, 2H), 3.71 (t, *J*=5.6 Hz, 2H), 2.81 (t, *J*=5.2 Hz, 2H).

Step 7. Synthesis of 2-{[7-fluoro-1-methyl-5-(trifluoromethyl)-1*H*-indol-3-yl]methyl}-7-(4-methyl-1*H*-imidazol-1-yl)-3,4-dihydro-1*H*-pyrido[1,2-*a*]pyrazine-1,6(2*H*)-dione (**2**). To a mixture of 2-{[7-fluoro-1-methyl-5-(trifluoromethyl)-1*H*-indol-3-yl]amino}ethanol (**C19**) (450 mg, 1.55 mmol) and hydrochloride salt **P2** (397 mg, 1.55 mmol) in dichloromethane (10 mL) was added *N,N*-diisopropylethylamine (600 mg, 4.65 mmol) and *O*-(7-azabenzotriazol-1-yl)-*N,N,N',N'*-tetramethyluronium hexafluorophosphate (HATU) (1.8 g, 4.65 mmol) and the resulting mixture was stirred for 20 h at room temperature. The reaction mixture was filtered, and the filtrate was washed with water and with brine, dried over sodium sulfate, and concentrated under reduced pressure. The residue was purified by preparative thin layer chromatography (Gradient: 0% to 10% methanol in dichloromethane) to provide the title compound as a white solid. Yield: 30 mg, 4%. LCMS *m*/*z* 473.9 (M+1); ¹H NMR (400 MHz, CD₃OD) δ 8.36 (s, 1H), 7.86 (s, 1H), 7.79 (d, *J*=8.0 Hz, 1H), 7.48 (s, 1H), 7.33-7.34 (m, 2H), 7.14 (d, *J*=13.2 Hz, 1H), 4.94 (s, 2H), 4.23 (t, *J*=5.8 Hz, 2H), 4.03 (s, 3H), 3.66 (t, *J*=5.8 Hz, 2H), 2.24 (s, 3H).

### Reference Example 3

### 7-(4-Methyl-1H-imidazol-1-yl)-2-1[5-(pentafluoro-λ⁶-sulfanyl)-1H-indol-3-yl]methyl}-3,4-dihydro-2H-pyrido[1,2-a]pyrazine-1,6-dione (3)

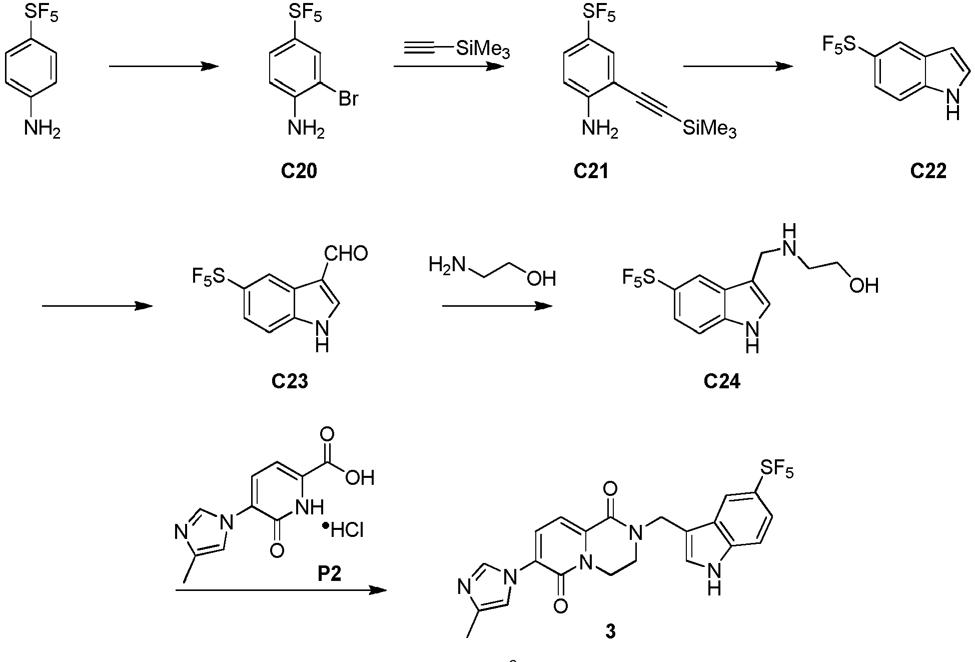

Step 1: Synthesis of 2-bromo-4-(pentafluoro-λ⁶-sulfanyl)aniline (**C20**). To a solution of 4-(pentafluoro-λ⁶-sulfanyl)aniline (4.56 g, 20.8 mmol) in *N,N*-dimethylformamide (20 mL) was added tetrabutylammonium tribromide (12.4 g, 24.9 mmol) at 0 °C. The reaction was stirred at 0 °C for 2 h, whereupon it was allowed to warm to room temperature and stirred for 3 days. A saturated aqueous solution of ammonium chloride (60 mL) was added, and the mixture was extracted with diethyl ether (3 x 40 mL). The combined organic layers were dried over sodium sulfate, filtered, and concentrated under reduced pressure. Purification via silica gel chromatography (Gradient: 0% to 10% ethyl acetate in heptane) gave the title compound as a yellow solid. Yield: 5.96 g, 90%. GCMS *m*/*z* 299 (M⁺). ¹H NMR (400 MHz, CDCl₃) δ 4.42 (br s, 2H), 6.72 (d, *J*=8.6 Hz, 1H), 7.50 (dd, *J*=9.0*,* 2.7 Hz, 1H), 7.82 (d, *J*=2.7 Hz, 1H).

Step 2: Synthesis of 4-(pentafluoro-λ⁶-sulfanyl)-2-[(trimethylsilyl)ethynyl]aniline (**C21**). To a solution of 2-bromo-4-(pentafluoro-λ⁶-sulfanyl)aniline (**C20**) (5.60 g, 18.8 mmol) and ethynyltrimethylsilane (8.02 mL, 56.3 mmol) in triethylamine (50 mL, degassed by sparging with nitrogen for 20 min) was added copper(I) iodide (722 mg, 3.75 mmol) and Pd(PPh₃)₄ (1.11 g, 0.94 mmol). The mixture was degassed for an additional 5 min by sparging with nitrogen, and then heated to 60 °C for 18 h. The mixture was allowed to cool to room temperature, diluted with a saturated aqueous solution of ammonium chloride (80 mL), and extracted with ethyl acetate (3 x 40 mL). The combined organic layers were dried over sodium sulfate, filtered, and concentrated under reduced pressure. Purification via silica gel chromatography (Gradient: 0% to 30% ethyl acetate in heptane) afforded the title compound as a yellow oil. Yield: 5.31 g, 90%. GCMS *m*/*z* 315 (M⁺). ¹H NMR (400 MHz, CDCl₃) δ 0.29 (s, 9H), 6.64 (d, *J*=9.0 Hz, 1H), 7.47 (dd, *J*=9.0, 2.3 Hz, 1H), 7.69 (d, *J*=2.7 Hz, 1H).

Step 3: Synthesis of 5-(pentafluoro-λ⁶-sulfanyl)-1*H*-indole (**C22**). To a solution of 4-(pentafluoro-λ⁶-sulfanyl)-2-[(trimethylsilyl)ethynyl]aniline (**C21**) (4.51 g, 14.3 mmol) in methanol (40 mL) was added potassium carbonate (5.93 g, 42.9 mmol). The mixture was stirred at room temperature for 3 h, whereupon it was diluted with water (70 mL), and extracted with diethyl ether (3 x 30 mL). The combined organic layers were dried over sodium sulfate, filtered, and concentrated under reduced pressure. The resulting solid (3.48 g, 14.3 mmol) was dissolved in *N*-methyl-2-pyrrolidone (NMP, 20 mL) and KO*t*-Bu (4.81 g, 42.9 mmol) was added at 0 °C. The reaction was allowed to warm to room temperature and was stirred for 18 h, whereupon it was diluted with water (70 mL), 1 M hydrochloric acid (5 mL), and a saturated aqueous solution of ammonium chloride (10 mL). The mixture was extracted with diethyl ether (3 x 30 mL), and the combined organic layers were dried over sodium sulfate, fitered, and concentrated under reduced pressure. Purification via silica gel chromatography (Gradient: 0% to 30% ethyl acetate in heptane) afforded the title compound as a yellow solid. Yield: 2.73 g, 79%. GCMS *m*/*z* 243 (M⁺). ¹H NMR (400 MHz, CDCl₃) δ 6.68 (br s, 1H), 7.35 (br s, 1H), 7.41 (d, *J*=9.0 Hz, 1H), 7.62 (d, *J*=9.0 Hz, 1H), 8.11 (s, 1H), 8.39 (br s, 1H).

Step 4: Synthesis of 5-(pentafluoro-λ⁶-sulfanyl)-1*H*-indole-3-carbaldehyde (**C23**). Phosphorous oxychloride (95 µL, 1.01 mmol) was added to *N,N*-dimethylformamide (2 mL) at 0 °C. To this solution was added a solution of 5-(pentafluoro-λ⁶-sulfanyl)-1*H-*indole (**C22**) (123 mg, 0.506 mmol) in *N,N*-dimethylformamide (2 mL). The reaction was allowed to warm to room temperature and was stirred for 2 h. Crushed ice and 1 M sodium hydroxide (40 mL) were added, and the mixture was extracted with diethyl ether (3 x 20 mL). The combined organic layers were dried over sodium sulfate, filtered, and concentrated under reduced pressure. Purification via silica gel chromatography (Gradient: 0% to 50% ethyl acetate in heptane) afforded the title compound as a white solid. Yield: 61 mg, 44%. LCMS *m*/*z* 272.2 (M+1); ¹H NMR (400 MHz, CD₃OD) δ 7.61 (d, *J*=9.0 Hz, 1H), 7.74 (dd, *J*=9.0, 2.3 Hz, 1H), 8.29 (s, 1H), 8.67 (d, *J*=2.3 Hz, 1H), 9.98 (s, 1H).

Step 5: Synthesis of 2-({[5-(pentafluoro-λ⁶-sulfanyl)-1*H*-indol-3-yl]methyl}amino)ethanol (**C24**). To a solution of 5-(pentafluoro-λ⁶-sulfanyl)-1*H*-indole-3-carbaldehyde (C23) (60 mg, 0.22 mmol) in methanol (10 mL) was added ethanolamine (27 µL, 0.44 mmol). The mixture was stirred at room temperature for 40 min followed by the addition of sodium borohydride (25.6 mg, 0.66 mmol) at 0 °C. The reaction was stirred at 0 °C for 10 min, whereupon the cooling bath was removed and the reaction was stirred for an additional 30 min. The reaction was concentrated under reduced pressure, and water (20 mL) was added. The mixture was extracted with dichloromethane (3 x 10 mL). The combined organic layers were dried over sodium sulfate, filtered, and concentrated to afford the title compound as a yellow solid. Yield: 67 mg, 96%. LCMS *m*/*z* 317.1 (M+1); ¹H NMR (400 MHz, CD₃OD) δ 2.83 (t, *J*=5.5 Hz, 2H), 3.71 (t, *J*=5.7 Hz, 2H), 4.03 (s, 2H), 7.41-7.50 (m, 2H), 7.58 (dd, *J*=9.0, 2.3 Hz, 1H), 8.17 (d, *J*=2.0 Hz, 1H).

Step 6: Synthesis of 7-(4-methyl-1*H*-imidazol-1-yl)-2-{[5-(pentafluoro-λ⁶-sulfanyl)-1*H-*indol-3-yl]methyl}-3,4-dihydro-2*H*-pyrido[1,2-*a*]pyrazine-1,6-dione (**3**). To a solution of 5-(4-methyl-1*H*-imidazol-1-yl)-6-oxo-1,6-dihydropyridine-2-carboxylic acid, hydrochloride salt (P2) (53.7 mg, 0.21 mmol) and HATU (165 mg, 0.42 mmol) in dichloromethane (3 mL) was added *N,N*-diisopropylethylamine (0.185 mL, 1.05 mmol). The reaction was stirred for 5 min, and a solution of 2-({[5-(pentafluoro-λ⁶-sulfanyl)-1*H*-indol-3-yl]methyl}amino)ethanol (**C24**) (67 mg, 0.21 mmol) in dichloromethane (3 mL) was added. The reaction was stirred for 18 h, whereupon a saturated aqueous solution of NaHCO₃ (30 mL) was added. The mixture was extracted with dichloromethane (3 x 20 mL), and the organic layers were dried over sodium sulfate, filtered, and concentrated under reduced pressure. Purification via silica gel chromatography (Gradient: 0% to 6% [2 N ammonia in methanol] in dichloromethane) afforded the title compound as a white solid. Yield: 47 mg, 43%. LCMS *m*/*z* 500.2 (M+1); ¹H NMR (400 MHz, DMSO-*d₆*) δ 2.10-2.18 (m, 3H), 3.53-3.64 (m, 2H), 4.13-4.24 (m, 2H), 4.88 (s, 2H), 7.15 (d, *J*=7.4 Hz, 1H), 7.37-7.39 (m, 1H), 7.50-7.61 (m, 2H), 7.70 (d, *J*=2.3 Hz, 1H), 7.79 (d, *J*=7.8 Hz, 1H), 8.22 (d, *J*=1.2 Hz, 1H) 8.26 (d, *J*=2.3 Hz, 1H), 11.62 (s, 1H).

### Example 4

### 2-{[1-Ethyl-5-(pentafluoro-λ⁶-sulfanyl)-1H-indol-3-yl]methyl}-7-(4-methyl-1H-imidazol-1-yl)-3,4-dihydro-2H-pyrido[1,2-a]pyrazine-1,6-dione (4)

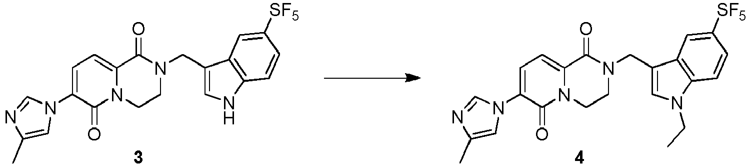

To a solution of 7-(4-methyl-1*H*-imidazol-1-yl)-2-{[5-(pentafluoro-λ⁶-sulfanyl)-1*H*-indol-3-yl]methyl}-3,4-dihydro-2*H*-pyrido[1,2-*a*]pyrazine-1,6-dione (**3**) (47 mg, 94 µmol) in *N,N-*dimethylformamide (2 mL) was added cesium carbonate (62.3 mg, 0.188 mmol) and ethyl iodide (12 µL, 0.14 mmol). The reaction mixture was stirred at room temperature for 18 h. Water (20 mL) was added, and the mixture was extracted with ethyl acetate (3 x 10 mL). The combined organic layers were dried over sodium sulfate, filtered, and concentrated under reduced pressure. Purification via silica gel chromatography (Gradient: 0% to 6% [2 M ammonia in methanol] in dichloromethane) afforded the title compound as a white solid. Yield: 27 mg, 55%. LCMS *m*/*z* 528.2 (M+1); ¹H NMR (400 MHz, CDCl₃) δ 1.47 (t, *J*=7.4 Hz, 3H), 2.25 (s, 3H), 3.42-3.70 (m, 2H), 4.05-4.30 (m, 4H), 4.92 (s, 2H), 7.09 (s, 1H), 7.28-7.37 (m, 3H), 7.43 (d, *J*=7.8 Hz, 1H), 7.60 (dd, *J*=9.4, 2.0 Hz, 1H), 7.99 (s, 1H), 8.14 (d, *J*=2.0 Hz, 1H).

### Example 5

### 2-{[1-Ethyl-5-(trifluoromethyl)-1H-pyrrolo[2,3-b]pyridin-3-yl]methyl}-7-(4-methyl-1H-imidazol-1-yl)-3,4-dihydro-2H-pyrido[1,2-a]pyrazine-1,6-dione (5).

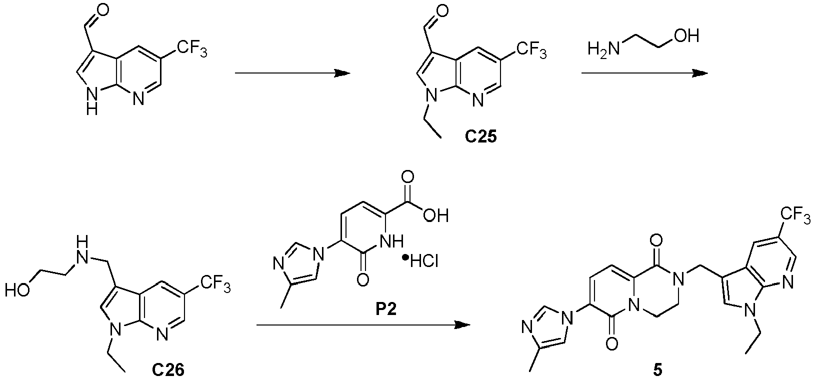

Step 1: Synthesis of 1-ethyl-5-(trifluoromethyl)-1*H*-pyrrolo[2,3-*b*]pyridine-3-carbaldehyde (**C25**). To a solution of 5-(trifluoromethyl)-1*H*-pyrrolo[2,3-*b*]pyridine-3-carbaldehyde (400 mg, 1.87 mmol) and ethyl iodide (450 mg, 2.89 mmol) in *N,N*-dimethylformamide (4 mL) was added potassium carbonate (500 mg, 3.62 mmol). The reaction mixture was stirred at 50 °C for 18 h, whereupon it was poured into water (50 mL) and extracted with dichloromethane (2 x 50 mL). The organic layer was dried over sodium sulfate and concentrated under reduced pressure to give the crude title compound (2.6 g) as a yellow solution in *N,N-*dimethylformamide. This solution was used directly in the next step. ¹H NMR (400 MHz, CDCl₃) δ 10.01 (s, 1H), 8.83 (d, *J*=2.0 Hz, 1H), 8.68 (d, *J*=1.5 Hz, 1H), 8.01 (s, 1H), 4.45 (q, *J*=7.5 Hz, 2H), 1.57 (t, *J*=7.3 Hz, 3H).

Step 2: Synthesis of 2-({[1-ethyl-5-(trifluoromethyl)-1*H*-pyrrolo[2,3-*b*]pyridin-3-yl]methyl}amino) ethanol (**C26**). A mixture of 1-ethyl-5-(trifluoromethyl)-1*H*-pyrrolo[2,3-*b*]pyridine-3-carbaldehyde (**C25**) (1.3 g, crude product from the previous step) and ethanolamine (1.0 g, 16.4 mmol) in ethanol (20 mL) was stirred at room temperature for 5 h. Sodium borohydride (200 mg, 5.29 mmol) was added in one portion, and the reaction was stirred at room temperature for 1 h. The reaction was concentrated under reduced pressure, and the residue was diluted with water (100 mL). The mixture was extracted with dichloromethane (3 x 50 mL), and the combined organic layers were dried over sodium sulfate and concentrated under reduced pressure to afford the title compound as a yellow solid. Yield: 260 mg, 97% over 2 steps. ¹H NMR (400 MHz, CDCl₃) δ 8.56 (d, *J*=1.5 Hz, 1H), 8.20 (d, *J*=1.5 Hz, 1H), 7.30 (s, 1H), 4.34 (q, *J*=7.5 Hz, 2H), 3.99 (s, 2H), 3.75-3.66 (m, 2H), 2.89-2.84 (m, 2H), 1.68 (br s, 2H), 1.47 (t, *J*=7.3 Hz, 3H).

Step 3: Synthesis of 2-{[1-ethyl-5-(trifluoromethyl)-1*H*-pyrrolo[2,3-*b*]pyridin-3-yl]methyl}-7-(4-methyl-1*H*-imidazol-1-yl)-3,4-dihydro-2*H*-pyrido[1,2-*a*]pyrazine-1,6-dione (**5**). A mixture of 2-({[1-ethyl-5-(trifluoromethyl)-1*H*-pyrrolo[2,3-*b*]pyridin-3-yl]methyl}amino)ethanol (**C26**) (260 mg, 0.91 mmol), 5-(4-methyl-1*H*-imidazol-1-yl)-6-oxo-1,6-dihydropyridine-2-carboxylic acid, hydrochloride salt (**P2**) (250 mg, 0.98 mmol), HATU (800 mg, 2.11 mmol) and triethylamine (1.0 g, 9.88 mmol) in dichloromethane (10 mL) was stirred at room temperature for 70 h. The mixture was washed with water (2 x 5 mL), dried over sodium sulfate, and concentrated under reduced pressure. The crude material was purified by silica gel chromatography (10:1 dichloromethane / methanol) and then re-purified by preparative thin layer chromatography (10:1 dichloromethane / methanol) to afford the title compound as an off-white solid. Yield: 62 mg, 14%. LCMS *m*/*z* 471.1 (M+H); ¹H NMR (400 MHz, CD₃OD) δ 8.64 (s, 1H), 8.55 (d, *J*=1.5 Hz, 1H), 8.45 (d, *J*=1.5 Hz, 1H), 7.86 (d, *J*=8.0 Hz, 1H), 7.79 (s, 1H), 7.43 (s, 1H), 7.36 (d, *J*=8.0 Hz, 1H), 4.96 (s, 2H), 4.40 (q, *J*=7.2 Hz, 2H), 4.30-4.22 (m, 2H), 3.75-3.66 (m, 2H), 2.29 (d, *J*=1.0 Hz, 3H), 1.48 (t, *J*=7.3 Hz, 3H).

### Reference Examples 6a and 6b

### 7-(4-Methyl-1H-imidazol-1-yl)-2-1(1S)-1-[1-methyl-5-(trifluoromethyl)-1H-indol-3-yl]ethyl}-3,4-dihydro-2H-pyrido[1,2-a]pyrazine-1,6-dione (6a) and 7-(4-Methyl-1H-imidazol-1-yl)-2-{(1R)-1-[1-methyl-5-(trifluoromethyl)-1H-indol-3-yl]ethyl}-3,4-dihydro-2H-pyrido[1,2-a]pyrazine-1,6-dione (6b).

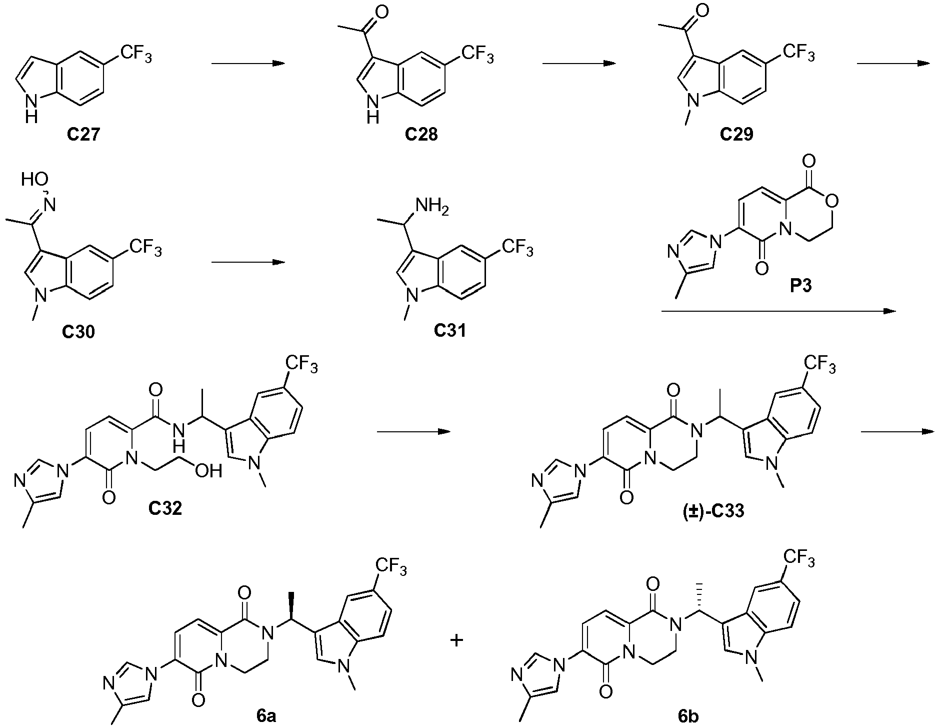

Step 1: Synthesis of 1-[5-(trifluoromethyl)-1*H*-indol-3-yl]ethanone (**C28**). To a solution of 5-(trifluoromethyl)-1*H*-indole (**C27**) (1.00 g, 5.40 mmol) in acetic anhydride (15 mL) was added indium(III) chloride (597 mg, 2.70 mmol) slowly at 0 °C under nitrogen atmosphere. The reaction mixture was stirred at room temperature for 3 h, whereupon cold water (10 mL) was added. The pH was adjusted to 7 via addition of a saturated aqueous solution of sodium bicarbonate, and the resulting mixture was extracted with ethyl acetate (2 × 50 mL). The combined organic layers were washed with brine (10 mL), dried over sodium sulfate, and concentrated under reduced pressure to give the crude title compound (1.2 g) as a brown solid. The crude material was purified by silica gel chromatography (10:1 ethyl acetate / hexane) to afford the title compound as a brown solid. Yield: 800 mg, 65%. LCMS *m*/*z* 225.6 (M-H); ¹H NMR (400 MHz, DMSO-*d₆*) δ 12.3 (s, 1H), 8.51-8.50 (m, 2H), 7.67 (d, *J*=8.5 Hz, 1H), 7.52 (d, *J*=7.5 Hz, 1H), 2.50 (s, 3H).

Step 2: Synthesis of 1-[1-methyl-5-(trifluoromethyl)-1*H*-indol-3-yl]ethanone (**C29**). To a solution of 1-[5-(trifluoromethyl)-1*H*-indol-3-yl]ethanone (**C28**) (800 mg, 3.52 mmol) in tetrahydrofuran (20 mL) was added sodium hydride (60% dispersion in mineral oil, 211 mg, 5.28 mmol) slowly at 0 °C, and the resulting mixture was stirred at room temperature for 10 min. Methyl iodide (0.44 mL, 7.04 mmol) was then added and the reaction mixture was stirred at room temperature for 2 h, whereupon cold water (10 mL) was added. The mixture was extracted with ethyl acetate (2 × 25 mL), and the combined organic layers were washed with brine (20 mL), dried over sodium sulfate, filtered, and concentrated under reduced pressure. The resulting residue was purified by silica gel chromatography (1:1 ethyl acetate / hexane) to afford the title compound as a brown solid. Yield: 600 mg, 71%. LCMS *m*/*z* 242.0 (M+H); ¹H NMR (400 MHz, CDCl₃) δ 8.70 (s, 1H), 7.78 (s, 1H), 7.55 (d, *J*=7.6 Hz, 1H), 7.41 (d, *J*=8.6 Hz, 1H), 3.89 (s, 3H), 2.53 (s, 3H).

Step 3: Synthesis of *N*-hydroxy-1-[1-methyl-5-(trifluoromethyl)-1*H*-indol-3-yl]ethanimine (**C30**). To a solution of 1-[1-methyl-5-(trifluoromethyl)-1*H*-indol-3-yl]ethanone (**C29**) (200 mg, 0.83 mmol) in ethanol (0.7 mL) were added hydroxylamine hydrochloride (86.4 mg, 1.2 mmol) and triethylamine (60 µL, 0.61 mmol), and the reaction mixture was irradiated at 120 °C in a microwave reactor for 20 min. The solvent was removed under reduced pressure, and the resulting residue was diluted with cold water (10 mL) and extracted with ethyl acetate (2 × 25 mL). The combined organic layers were washed with brine (10 mL), dried over sodium sulfate, and concentrated under reduced pressure to give the crude title compound (200 mg, 94%), which was used in the next step without further purification.

Step 4: Synthesis of 1-[1-methyl-5-(trifluoromethyl)-1*H*-indol-3-yl]ethanamine (**C31**). Crude *N*-hydroxy-1-[1-methyl-5-(trifluoromethyl)-1*H*-indol-3-yl]ethanimine (**C30**) from Step 3 (200 mg, 0.78 mmol), ethanol (25 mL), ammonium hydroxide (10 µL, 0.42 mmol) and Raney nickel (200 mg) were combined in a Parr shaker and the mixture was hydrogenated for 6 h under an atmosphere of 50 psi hydrogen. The reaction mixture was then filtered through a pad of Celite, and the solids were washed with methanol. The filtrate was concentrated under reduced pressure to give the crude title compound (200 mg, assumed quantitative) as a yellow solid, which was used directly in the next step without further purification.

Step 5: Synthesis of 1-(2-hydroxyethyl)-5-(4-methyl-1*H*-imidazol-1-yl)-*N*-{1-[1-methyl-5-(trifluoromethyl)-1*H*-indol-3-yl]ethyl}-6-oxo-1,6-dihydropyridine-2-carboxamide (**C32**). To a solution of crude 1-[1-methyl-5-(trifluoromethyl)-1*H*-indol-3-yl]ethanamine (**C31**) (400 mg, 1.65 mmol) in tetrahydrofuran (20 mL) was added bis(trimethylaluminum)-1,4-diazabicyclo[2.2.2]octane adduct (DABAL-Me₃, 847 mg, 3.30 mmol) portion-wise at room temperature, and the resulting mixture was stirred for 3 h at 70 °C. 7-(4-Methyl-1*H*-imidazol-1-yl)-3,4-dihydropyrido[2,1-*c*][1,4]oxazine-1,6-dione (**P3**) (607 mg, 2.48 mmol) was added and the reaction mixture was stirred for 20 h at 75 °C. The solvent was removed under reduced pressure, and dichloromethane (10 mL) followed by water (50 mL) were added slowly at room temperature. The resulting mixture was diluted with additional dichloromethane (100 mL), stirred for 30 min at room temperature, and then filtered. The layers were separated and the aqueous layer was extracted with dichloromethane (3 × 10 mL). The combined organic layers were dried over sodium sulfate and concentrated under reduced pressure. The crude material was purified by silica gel chromatography (10:1 dichloromethane / methanol) to afford the title compound as a light yellow solid. Yield: 130 mg, 16%. LCMS *m*/*z* 488.2 (M+H); ¹H NMR (400 MHz, CDCl₃) δ 8.26 (br s, 1H), 8.05 (s, 1H), 7.96 (s, 1H), 7.52 (d, J=8.4 Hz, 1H), 7.42 (d, *J*=8.8 Hz, 1H), 7.23 (s, 1H), 7.17 (d, *J*=7.5 Hz, 1H), 6.97 (s, 1H), 6.42 (d, *J*=7.5 Hz, 1H), 5.67-5.64 (m, 1H), 4.29 (br s, 2H), 4.09-3.97 (m, 2H), 3.84 (s, 3H), 1.98 (s, 3H), 1.80 (d, *J*=6.8 Hz, 3H).

Step 6: Synthesis of 7-(4-methyl-1*H*-imidazol-1-yl)-2-{1-[1-methyl-5-(trifluoromethyl)-1*H-*indol-3-yl]ethyl}-3,4-dihydro-2*H*-pyrido[1,2-*a*]pyrazine-1,6-dione [(±)-**C33**]. To a solution of 1-(2-hydroxyethyl)-5-(4-methyl-1*H*-imidazol-1-yl)-*N*-{1-[1-methyl-5-(trifluoromethyl)-1*H*-indol-3-yl]ethyl}-6-oxo-1,6-dihydropyridine-2-carboxamide (**C32**) (130 mg, 0.27 mmol) in dichloromethane (10 mL) was added triethylamine (31 µL, 0.53 mmol) followed by methanesulfonyl chloride (31 µL, 0.40 mmol) at -10 °C. The reaction mixture was stirred at room temperature for 2 h, whereupon the solvent was removed under reduced pressure. The resulting residue was dissolved in tetrahydrofuran (10 mL); 1,3,4,6,7,8-hexahydro-2*H*-pyrimido[1,2-*a*]pyrimidine (TBD, 223 mg, 1.60 mmol) was added under a nitrogen atmosphere, and the reaction mixture was stirred at room temperature for 2 h. A 1 M aqueous solution of sodium hydroxide was then added and the resulting mixture was extracted with dichloromethane (3 × 10 mL), washed with brine (10 mL), and dried over sodium sulfate. The solvent was removed under reduced pressure, and the crude material was purified by preparative HPLC (Column: Waters XBridge C18 OBD, 5 µm; Mobile phase A: 5 mM ammonium acetate in water; Mobile phase B: acetonitrile; Gradient: 90% A / 10% B to 0% A / 100% B) to afford the title compound as an off-white solid. Yield: 15.0 mg, 12%. LCMS *m*/*z* 469.8 (M); ¹H NMR (400 MHz, DMSO-*d₆*) δ 8.22 (s, 1H), 7.82-7.80 (m, 2H), 7.70 (s, 1H), 7.66 (d, *J*=8.6 Hz, 1H), 7.46 (d, *J*=7.6 Hz, 1H), 7.39 (s, 1H), 7.20 (d, *J*=7.7 Hz, 1H), 6.16 (q, *J*=7.0 Hz, 1H), 4.18-4.12 (m, 1H), 3.94-3.88 (m, 1H), 3.86 (s, 3H), 3.62-3.57 (m, 1H), 3.12-3.05 (m, 1H), 2.14 (s, 3H), 1.62 (d, *J*=7.0 Hz, 3H).

Step 6: Synthesis of 7-(4-methyl-1*H*-imidazol-1-yl)-2-1(1*S*)-1-[1-methyl-5-(trifluoromethyl)-1*H*-indol-3-yl]ethyl}-3,4-dihydro-2*H*-pyrido[1,2-*a*]pyrazine-1,6-dione **(6a)** and 7-(4-methyl-1*H*-imidazol-1-yl)-2-{(1*R*)-1-[1-methyl-5-(trifluoromethyl)-1*H*-indol-3-yl]ethyl}-3,4-dihydro-2*H*-pyrido[1,2-*a*]pyrazine-1,6-dione **(6b).** The enantiomers of racemic 7-(4-methyl-1*H*-imidazol-1-yl)-2-{1-[1-methyl-5-(trifluoromethyl)-1*H*-indol-3-yl]ethyl}-3,4-dihydro-2*H*-pyrido[1,2-*a*]pyrazine-1,6-dione [(±)-**C33**] (400 mg) were separated by chiral HPLC (Column: Chiralpak AD-H, 21 × 250 mm, 5 µm; Mobile phase: 100: 0.1 methanol / diethylamine) to give **6a** and **6b** (retention times: 6.76 min and 12.01 min, respectively). Yield **6a:** 75 mg, 38%. Yield **6b:** 75 mg, 38%. The ¹H NMR and mass spectral data were identical to that reported for racemate (±)-**C33**.

### Reference Example 7

### 7-(4-Methyl-1H-imidazol-1-yl)-2-{[8-(trifluoromethyl)-3,4-dihydro-1H-[1,4]oxazino[4,3-a]indol-10-yl]methyl}-3,4-dihydro-2H-pyrido[1,2-a]pyrazine-1,6-dione (7).

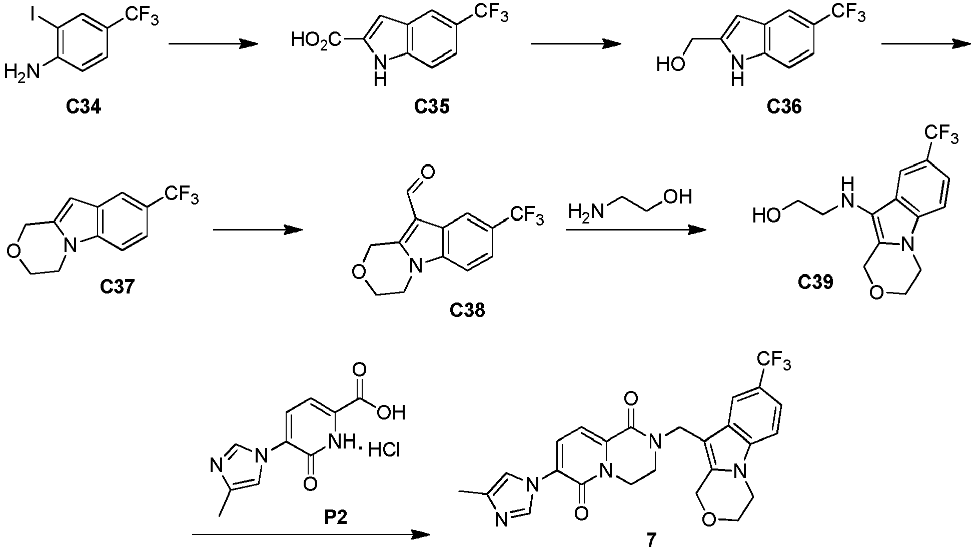

Step 1: Synthesis of 5-(trifluoromethyl)-1*H*-indole-2-carboxylic acid **(C35).** A mixture of 2-iodo-4-(trifluoromethyl)aniline **(C34)** (250 mg, 0.871 mmol), pyruvic acid (0.123 mL, 1.74 mmol), 1,4-diazabicyclo[2.2.2]octane (195 mg, 1.74 mmol), and palladium(II) acetate (10 mg, 44 µmol) in dry *N,N*-dimethylformamide (10 mL) was degassed via vacuum / nitrogen purges and heated at 105 °C for 10 h. The reaction mixture was allowed to cool to room temperature and was partitioned between ethyl acetate and water. The organic layer was washed with brine, dried over sodium sulfate, and concentrated under vacuum. The residue was purified by silica gel chromatography, eluting with 10% ethyl acetate in hexane, to give the title compound as a brown solid. Yield: 130 mg, 65%. LCMS m/z 228.0 (M-H); ¹H NMR (400 MHz, DMSO-*d₆*) δ 13.27 (s, 1H), 12.20 (s, 1H), 8.09 (s, 1H), 7.61 (d, *J*=8.7 Hz, 1H), 7.51 (d, *J*=8.6 Hz, 1H), 7.25 (s, 1H).

Step 2: Synthesis of [5-(trifluoromethyl)-1*H*-indol-2-yl]methanol **(C36)*.*** A mixture of 5-(trifluoromethyl)-1*H*-indole-2-carboxylic acid **(C35)** (250 mg, 1.09 mmol) and lithium aluminum hydride (1 M solution in tetrahydrofuran, 5.5 mL, 5.5 mmol) in dry tetrahydrofuran (10 mL) was heated at 70 °C for 4 h. After completion (by LCMS), the reaction mixture was cooled to 0 °C, quenched with a saturated aqueous solution of sodium sulfate, and filtered through a Celite pad; the solids were washed with dichloromethane. The filtrate layers were separated, and the organic layer was dried over sodium sulfate and concentrated under reduced pressure. The residue was purified by silica gel chromatography, eluting with 30% ethyl acetate in hexane, to give the title compound as a brown solid. Yield: 120 mg, 51%. LCMS m/z 214.2 (M-H); ¹H NMR (400 MHz, DMSO-*d₆*) δ 11.48 (s, 1H), 7.85 (s, 1H), 7.49 (d, *J*=8.4 Hz, 1H), 7.31 (d, *J*=8.4 Hz, 1H), 6.87 (s, 1H), 6.44 (s, 1H), 4.63 (d, *J*=5.6 Hz, 2H).

Step 3: Synthesis of 8-(trifluoromethyl)-3,4-dihydro-1*H*-[1,4]oxazino[4,3-*a*]indole **(C37)**. To a solution of [5-(trifluoromethyl)-1*H*-indol-2-yl]methanol **(C36)** (50 mg, 0.23 mmol) in dichloromethane (7 mL) was added potassium hydroxide (33 mg, 0.58 mmol) and diphenyl(vinyl)sulfonium trifluoromethanesulfonate (101 mg, 0.28 mmol) at 0 °C, and the reaction mixture was stirred for 2 h at room temperature. The reaction mixture was diluted with dichloromethane and the organic layer was washed with water and with brine, dried over sodium sulfate, and concentrated under reduced pressure. The residue was purified by silica gel chromatography, eluting with 10% ethyl acetate in hexane, to afford the title compound as a white solid. Yield: 15 mg, 27%. ¹H NMR (400 MHz, CDCl₃) δ 7.85 (s, 1H), 7.40 (d, *J*=8.0 Hz, 1H), 7.34 (d, *J*=8.5 Hz, 1H), 6.30 (s, 1H), 5.00 (s, 2H), 4.19 (t, *J*=4.4 Hz, 2H), 4.12 (t, *J*=4.4 Hz, 2H).

Step 4: Synthesis of 8-(trifluoromethyl)-3.4-dihydro-1*H*-[1,4]oxazino[4,3-*a*]indole-10-carbaldehyde **(C38)*.*** To a solution of 8-(trifluoromethyl)-3,4-dihydro-1*H*-[1,4]oxazino[4,3-a]indole **(C37)** (400 mg, 1.66 mmol) in acetonitrile (20 mL) was added N-(chloromethylene)-*N*-methylmethanaminium chloride (722 mg, 5.64 mmol) at 0 °C. The reaction mixture was stirred for 20 minutes at room temperature, whereupon it was quenched with water (1 mL) and warmed to 50 °C for 1 h. The resulting pink solution was allowed to cool to room temperature and poured into water. The mixture was extracted with ethyl acetate; the combined organic layers were washed with brine, dried over sodium sulfate, and concentrated under reduced pressure. The residue was purified by silica gel chromatography, eluting with 20-25% ethyl acetate in hexane, to afford the title compound as a white solid. Yield: 350 mg, 78%. LCMS m/z 270.0 (M+H); ¹H NMR (400 MHz, DMSO-*d₆*) δ 10.07 (s, 1H), 8.42 (s, 1H), 7.78 (d, *J*=8.6 Hz, 1H), 7.61 (d, *J*=8.5 Hz, 1H), 5.35 (s, 2H), 4.28 (t, *J*=5.0 Hz, 2H), 4.19 (t, *J*=4.9 Hz, 2H).

Step 5: Synthesis of 2-{[8-(trifluoromethyl)-3,4-dihydro-1*H*-[1,4]oxazino[4,3-*a*]indol-10-yl]amino}ethanol **(C39).** To a solution of 8-(trifluoromethyl)-3,4-dihydro-1*H-*[1,4]oxazino[4,3-*a*]indole-10-carbaldehyde **(C38)** (300 mg, 1.25 mmol) in toluene (15 mL) was added ethanolamine (84 mg, 1.4 mmol) and ytterbium(III) trifluoromethanesulfonate (7.7 mg, 12 µmol) at room temperature and the reaction mixture was heated to reflux for 6 h. The reaction mixture was concentrated under reduced pressure; methanol (10 mL) was then added, followed by sodium borohydride (70.6 mg, 1.87 mmol) at 0 °C. The reaction mixture was stirred at room temperature for 2 h and then quenched with ice-water. The methanol in the reaction mixture was removed under reduced pressure and the resulting residue was extracted with 10% methanol in dichloromethane. The organic layer was washed with brine, dried over sodium sulfate, and concentrated to give the title compound, which was directly used in the next step without further purification (230 mg, 59%).

Step 6: Synthesis of 7-(4-methyl-1*H*-imidazol-1-yl)-2-{[8-(trifluoromethyl)-3,4-dihydro-1*H*-[1,4]oxazino[4,3-*a*]indol-10-yl]methyl}-3,4-dihydro-2*H*-pyrido[1,2-*a*]pyrazine-1,6-dione (**7**). To a solution of 5-(4-methyl-1*H*-imidazol-1-yl)-6-oxo-1,6-dihydropyridine-2-carboxylic acid hydrochloride salt **(P2)** (69.4 mg, 0.271 mmol) in dichloromethane (10 mL) was added triethylamine (0.132 mL, 0.955 mmol) followed by *O*-(7-azabenzotriazol-1-yl)-*N,N,N',N'*-tetramethyluronium hexafluorophosphate (HATU, 243 mg, 0.637 mmol) at room temperature. The reaction mixture was stirred at room temperature for 30 min and crude 2-{[8-(trifluoromethyl)-3,4-dihydro-1*H*-[1,4]oxazino[4,3-*a*]indol-10-yl]amino}ethanol **(C39)** (100 mg, 0.318 mmol) was added. The reaction mixture was stirred for 16 h and was then diluted with dichloromethane. The organic layer was washed with aqueous sodium bicarbonate solution and with brine, dried over sodium sulfate, and concentrated. The residue was purified by preparative HPLC (Column: Waters XTerra RP18 OBD, 250 x 19 mm, 10 µM; Mobile phase A: 5 mM ammonium acetate in water; Mobile phase B: acetonitrile; Gradient: 10% to 35% B over 3.0 min, 35% to 40% B over 11.0 min, 40% to 50% B over 16 min), affording the title compound as an off-white solid. Yield: 11 mg, 7%. LCMS *m*/*z* 498.2 (M+H); ¹H NMR (400 MHz, DMSO-*d₆*) δ 8.22 (s, 1H), 8.06 (s, 1H), 7.78 (d, *J*=7.6 Hz, 1H), 7.62 (d, *J*=8.3 Hz, 1H), 7.44 (d, *J*=8.8 Hz, 1H), 7.38 (s, 1H), 7.15 (d, *J*=7.8 Hz, 1H), 5.10 (bs, 2H), 4.84 (bs, 2H), 4.15 (bs, 6H), 3.57 (bs, 2H), 2.14 (s, 3H).

### Methods

### Method A

### Preparation of 2-substituted 7-(4-methyl-1H-imidazol-1-yl)-3,4-dihydro-2H-pyrido[1,2-a]pyrazine-1,6-dione (M1) via initial reductive amination

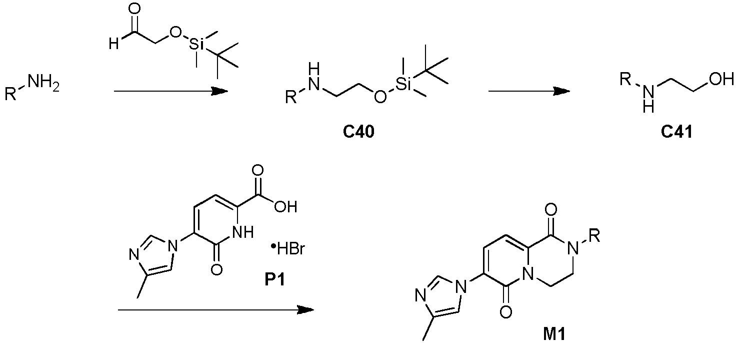

Step 1: Synthesis of *N*-substituted 2-{[*tert*-butyl(dimethyl)silyl]oxy}ethanamine **C40.** A solution of the primary amine (300 µmol) in methanol (1 mL) was treated with {[*tert-*butyl(dimethyl)silyl]oxy}acetaldehyde (28 µL, 150 µmol) and shaken at 30 °C for 40 min. The reaction vial was cooled to 0 °C, sodium borohydride (17 mg, 450 µmol) was added, and the reaction was shaken at 30 °C for 100 min. The solvent was removed under reduced pressure, water (1 mL) was added and the mixture was extracted with ethyl acetate (3 x 1 mL). The combined organic layers were dried over sodium sulfate, filtered, concentrated under reduced pressure and purified via preparative thin layer chromatography.

Step 2: Synthesis of *N*-substituted 2-aminoethanol **C41.** A solution of the *N*-substituted 2-{[*tert*-butyl(dimethyl)silyl]oxy}ethanamine **C40** in methanol (500 µL) was treated with a solution of acetyl chloride (188 µL) in methanol (312 µL) at 30 °C for 16 h. The solvent was removed under reduced pressure.

Step 3: Synthesis of 2-substituted 7-(4-methyl-1*H*-imidazol-1-yl)-3,4-dihydro-2*H-*pyrido[1,2-*a*]pyrazine-1,6-dione **M1.** The *N*-substituted 2-aminoethanol **C41** was treated with 5-(4-methyl-1*H*-imidazol-1-yl)-6-oxo-1,6-dihydropyridine-2-carboxylic acid, hydrobromide salt **(P1)** (34.4 mg, 125 µmol), dichloromethane (2 mL), *N,N-*diisopropylethylamine (217 µL, 1.25 mmol) and *O*-(7-azabenzotriazol-1-yl)-*N,N,N',N'-*tetramethyluronium hexafluorophosphate (HATU, 97%, 122 mg, 320 µmol), then shaken at 30 °C for 16 h. The solvent was removed under reduced pressure and the residue was treated with saturated aqueous sodium bicarbonate solution (2 mL) and extracted with ethyl acetate (3 x 1 mL). The combined organic layers were dried over sodium sulfate, filtered, concentrated under reduced pressure and purified via preparative reversed-phase HPLC. Purifications were carried out using a Phenomenex Gemini C18 column (8-10 µm), with the non-aqueous mobile phase consisting of ammonium hydroxide in acetonitrile (pH 10) and employing an appropriate gradient.

### Method B

### Preparation of 2-(substituted benzyl)-7-(4-methyl-1H-imidazol-1-yl)-3.4-dihydro-2H-prido[1,2-a]pyrazine-1,6-dione (M2) via Suzuki coupling

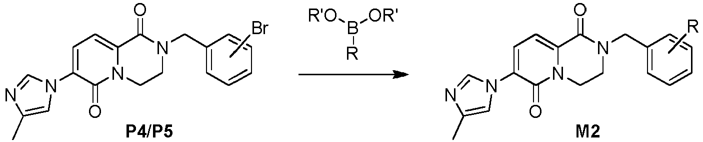

The appropriate boronic acid or ester (72 µmol) was weighed into a vial and treated with a solution of 2-(3-bromobenzyl)-7-(4-methyl-1*H*-imidazol-1-yl)-3,4-dihydro-2*H*-pyrido[1,2-*a*]pyrazine-1,6-dione **(P4)** or 2-(2-bromobenzyl)-7-(4-methyl-1*H*-imidazol-1-yl)-3,4-dihydro-2*H*-pyrido[1,2-*a*]pyrazine-1,6-dione **(P5)** (26.5 mg, 0.064 mmol) in 1,4-dioxane (750 µL). Next, a solution of cesium carbonate (43.2 mg, 0.12 mmol) in water (150 µL) was added and nitrogen was bubbled through the reaction. Dichloro[1,1'bis(di-*tert*-butylphosphino)]ferrocene palladium(II) (2 mg, 3 µmol) was added, nitrogen was bubbled through the reaction and the vial was capped and heated to 100 °C for 16 h. The reaction was filtered, the solvent removed under reduced pressure and the residue was purified by preparative reversed-phase HPLC. Purifications were carried out using an appropriate gradient on either a DIKMA Diamonsil(2) C18 column (5 µm) or a Boston Symmetrix C18 ODS-H column (5 µm), with the aqueous and the acetonitrile mobile phases each containing 0.225% formic acid.

### Method C

### Preparation of 2-substituted 7-(4-methyl-1H-imidazol-1-yl)-3,4-dihydro-2H-pyrido[1,2-a]pyrazine-1,6-dione (M1) via initial amine alkylation

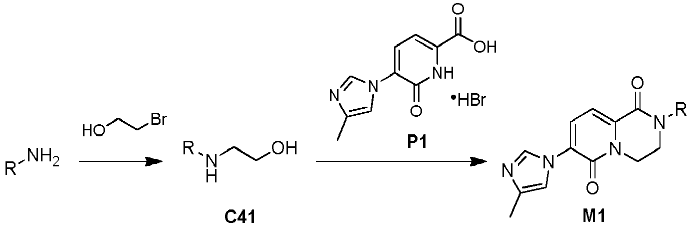

Step 1: Synthesis of *N*-substituted 2-aminoethanol **C41.** A solution of the primary amine (100 µmol) in *N,N*-dimethylformamide (1 mL) was treated with *N,N-*diisopropylethylamine (35 µL, 200 µmol) and 2-bromoethanol (10 µL, 180 µmol) and shaken at 50 °C for 48 h. The solvent was removed under reduced pressure and the title compound was used directly in the next step.

Step 2: Synthesis of 2-substituted 7-(4-methyl-1*H*-imidazol-1-yl)-3,4-dihydro-2*H-*pyrido[1,2-*a*]pyrazine-1,6-dione **M1.** The *N*-substituted 2-aminoethanol **C41** was treated with 5-(4-methyl-1*H*-imidazol-1-yl)-6-oxo-1,6-dihydropyridine-2-carboxylic acid, hydrobromide salt **(P1)** (27.5 mg, 100 µmol), dichloromethane (2 mL), *N,N-*diisopropylethylamine (174 µL, 1.00 mmol) and *O*-(7-azabenzotriazol-1-yl)-*N,N,N',N'-*tetramethyluronium hexafluorophosphate (HATU, 97%, 97.3 mg, 256 µmol), then shaken at 30 °C for 16 h. The solvent was removed under reduced pressure and the residue was diluted with saturated aqueous sodium bicarbonate solution (2 mL) and extracted with ethyl acetate (3 x 1 mL). The combined organic layers were dried over sodium sulfate, filtered, concentrated under reduced pressure and purified via preparative reversed-phase HPLC, as described for Method A.

### Method D

### Preparation of 7-(4-methyl-1H-imidazol-1-yl)-2-[(1S)-1-(6-substituted-pyridin-2-yl)ethyl]-3,4-dihydro-2H-pyrido[1,2-a]pyrazine-1,6-diones (M3) via Suzuki coupling

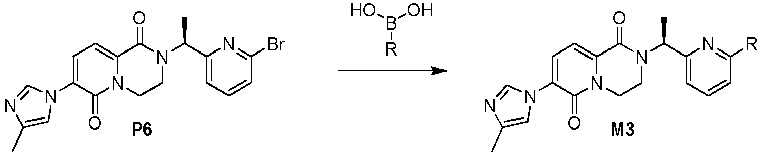

To 8 mL vials charged with a 1.0 M solution of the appropriate boronic acid (0.30 mL, 300 µmol) in 1,4-dioxane was added a 0.25 M solution of 2-[(1*S*)-1-(6-bromopyridin-2-yl)ethyl]-7-(4-methyl-1*H*-imidazol-1-yl)-3,4-dihydro-2*H*-pyrido[1,2-*a*]pyrazine-1,6-dione **(P6)** (0.30 mL, 75 µmol) in 1,4-dioxane. Next, a solution of potassium phosphate (0.30 mL, 150 µmol) was added, followed by Pd-118 [1,1'-bis(di-*tert*-butylphosphino)ferrocene palladium dichloride, 3.0 mg, 4.5 µmol] under nitrogen atmosphere. The vials were capped and shaken at 120 °C for 2 h, whereupon the solvent was removed under reduced pressure. The residue was purified by preparative HPLC using an appropriate gradient on either a Phenomenex Synergi C18 column, 150 x 30 mm, 4 µm, with the aqueous and the acetonitrile mobile phases each containing 0.1% TFA; or on a Phenomenex Gemini C18 column, 250 x 21.2 mm, 8 µm, eluting with aqueous ammonium hydroxide (pH = 10) and acetonitrile.

The compounds exemplified in Table 1 can be synthesized using the methods illustrated above, either alone or in combination with techniques generally known in the art.

**Table 1**

| | | | | |
|---|---|---|---|---|
| 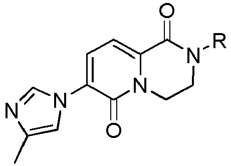 | | | | |

| Ex # | Structure | Method of Preparation; Non-commercial Starting Materials | IUPAC Name | ¹H NMR (400 MHz, CDCl₃), δ (ppm); Mass spectrum, observed ion *m*/*z* (M+1) or HPLC retention time (min); Mass spectrum *m*/*z* (M+1) (unless otherwise indicated) |
|---|---|---|---|---|
| | 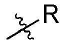 | | | |
| **8 (Ref.)** | 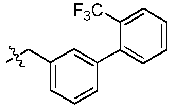 | Ex 1¹ | 7-(4-methyl-1*H*-imidazol-1-yl)-2-{[2'-(trifluoromethyl)biphenyl-3-yl]methyl}-3,4-dihydro-2H-pyrido[1,2-a]pyrazine-1,6-dione | ¹H NMR (400 MHz, CD₃OD) δ 2.23 (d, *J*=1.0 Hz, 3H), 3.67-3.72 (m, 2H), 4.27-4.31 (m, 2H), 4.83 (s, 2H), 7.25-7.30 (m, 2H), 7.28 (d, *J*=7.8 Hz, 1H), 7.31-7.33 (br m, 1H), 7.36 (br d, *J*=7.6 Hz, 1H), 7.41-7.44 (m, 2H), 7.53 (br dd, *J*=7.8, 7.5 Hz, 1H), 7.60-7.65 (m 1H), 7.74 (d, *J*=7.8 Hz, 1H), 7.76 (br d, *J*=8 Hz, 1H), 8.29 (d, *J*=1.3 Hz, 1H); 479.2 |
| **9 (Ref.)** | 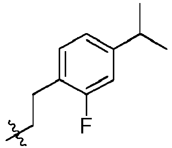 | Method A² | 2-[2-(2-fluoro-4-isopropylphenyl)ethyl]-7-(4-methyl-1*H*-imidazol-1-yl)-3,4-dihydro-2*H*-pyrido[1,2-*a*]pyrazine-1,6-dione | 2.84 min¹²; 409 |
| **10 (Ref.)** | 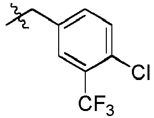 | Method A | 2-[4-chloro-3-(trifluoromethyl)benzyl]-7-(4-methyl-1*H*-imidazol-1-yl)-3,4-dihydro-2*H*-pyrido[1,2-*a*]pyrazine-1,6-dione | 2.74 min¹²; 437 |
| 11 **(Ref.)** | 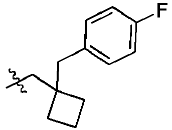 | Method A³ | 2-{[1-(4-fluorobenzyl)cyclobutyl]methyl}-7-(4-methyl-1*H*-imidazol-1-yl)-3,4-dihydro-2*H*-pyrido[1,2-*a*]pyrazine-1,6-dione | 2.82 min¹²; 421 |
| 12 **(Ref.)** | 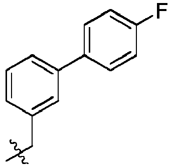 | Method B; **P4** | 2-[(4'-fluorobiphenyl-3-yl)methyl]-7-(4-methyl-1 *H*-imidazol-1-yl)-3,4-dihydro-2*H*-pyrido[1,2-a]pyrazine-1,6-dione, formate salt | 2.82 min¹²; 429 |
| 13 **(Ref.)** | 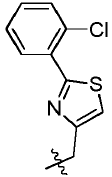 | Method C⁴ | 2-{[2-(2-chlorophenyl)-1,3-thiazol-4-yl]methyl}-7-(4-methyl-1*H-*imidazol-1-yl)-3,4-dihydro-2H-pyrido[1,2-*a*]pyrazine-1,6-dione | 2.69 min¹²; 452 |
| **14 (Ref.)** | 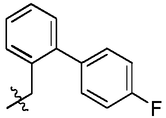 | Method B; **P5** | 2-[(4'-fluorobiphenyl-2-yl)methyl]-7-(4-methyl-1*H*-imidazol-1-yl)-3,4-dihydro-2*H*-pyrido[1,2-a]pyrazine-1,6-dione, formate salt | 2.71 min¹²; 429 |
| **15 (Ref.)** | 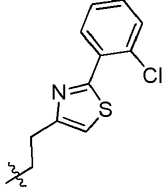 | Method C⁵ | 2-{2-[2-(2-chlorophenyl)-1,3-thiazol-4-yl]ethyl}-7-(4-methyl-1 *H-*imidazol-1-yl)-3,4-dihydro-2*H-*pyrido[1,2-*a*]pyrazine-1,6-dione | 2.63 min¹²; 466 |
| **16 (Ref.)** | 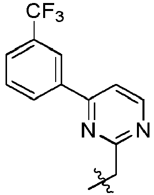 | Method C⁶ | 7-(4-methyl-1*H*-imidazol-1-yl)-2-({4-[3-(trifluoromethyl)phenyl]pyrimidin-2-yl}methyl)-3,4-dihydro-2H-pyrido[1,2-*a*]pyrazine-1,6-dione | 2.60 min¹²; 481 |
| **17 (Ref.)** | 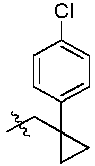 | Ex 1⁷ | 2-{[1-(4-chlorophenyl)cyclopropyl]methyl}-7-(4-methyl-1*H*-imidazol-1-yl)-3,4-dihydro-2*H*-pyrido[1,2-a]pyrazine-1,6-dione, trifluoroacetate salt | 2.40 min¹¹; 409.2, 411.2 |
| **18 (Ref.)** | 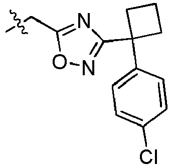 | Ex 1⁸ | 2-({3-[1-(4-chlorophenyl)cyclobutyl]-1,2,4-oxadiazol-5-yl}methyl)-7-(4-methyl-1*H*-imidazol-1-yl)-3,4-dihydro-2*H*-pyrido[1,2-*a*]pyrazine-1,6-dione, trifluoroacetate salt | 2.67 min¹¹; 491.2, 493.2 |
| **19 (Ref.)** | 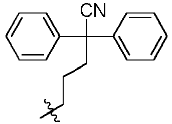 | Ex 1⁹ | 5-[7-(4-methyl-1*H*-imidazol-1-yl)-1,6-dioxo-1,3,4,6-tetrahydro-2*H-*pyrido[1,2-a]pyrazin-2-yl]-2,2-diphenylpentanenitrile | 2.59 min¹¹; 478.4 |
| **20 (Ref.)** | 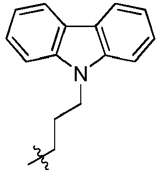 | Ex 1¹⁰ | 2-[3-(9*H*-carbazol-9-yl)propyl]-7-(4-methyl-1*H*-imidazol-1-yl)-3,4-dihydro-2*H*-pyrido[1,2-*a*]pyrazine-1,6-dione | 2.46 min¹¹; 452.1 |
| **21 (Ref.)** | 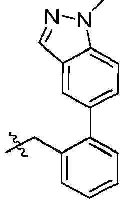 | Method B; **P5** | 7-(4-methyl-1*H*-imidazol-1-yl)-2-[2-(1-methyl-1*H*-indazol-5-yl)benzyl]-3,4-dihydro-2H-pyrido[1,2-*a*]pyrazine-1,6-dione, formate salt | 2.58 min¹²; 465 |
| **22 (Ref.)** | 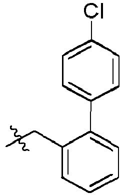 | Method B; **P5** | 2-[(4'-chlorobiphenyl-2-yl)methyl]-7-(4-methyl-1 *H*-imidazol-1-yl)-3,4-dihydro-2*H*-pyrido[1,2-a]pyrazine-1,6-dione, formate salt | 2.97 min¹²; 445 |
| **23 (Ref.)** | 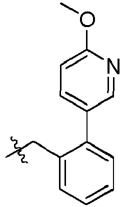 | Method B; **P5** | 2-[2-(6-methoxypyridin-3-yl)benzyl]-7-(4-methyl-1*H-*imidazol-1-yl)-3,4-dihydro-2*H-*pyrido[1,2-*a*]pyrazine-1,6-dione, formate salt | 2.48 min¹²; 442 |
| **24 (Ref.)** | 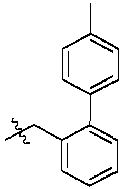 | Method B; **P5** | 2-[(4'-methyl biphenyl-2-yl)methyl]-7-(4-methyl-1*H-*imidazol-1-yl)-3,4-dihydro-2*H-*pyrido[1,2-*a*]pyrazine-1,6-dione, formate salt | 2.96 min¹²; 425 |
| **25 (Ref.)** | 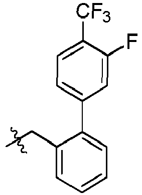 | Method B; **P5** | 2-{[3'-fluoro-4'-(trifluoromethyl)biphenyl-2-yl]methyl}-7-(4-methyl-1 *H-*imidazol-1-yl)-3,4-dihydro-2*H-*pyrido[1,2-*a*]pyrazine-1,6-dione, formate salt | 2.87 min¹³; 497 |
| **26 (Ref.)** | 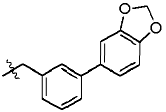 | Method B; **P4** | 2-[3-(1,3-benzodioxol-5-yl)benzyl]-7-(4-methyl-1*H-*imidazol-1-yl)-3,4-dihydro-2*H-*pyrido[1,2-*a*]pyrazine-1,6-dione, formate salt | 2.73 min¹²; 455 |
| **27 (Ref.)** | 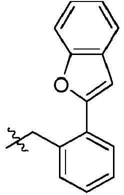 | Method B; **P5** | 2-[2-(1-benzofuran-2-yl)benzyl]-7-(4-methyl-1 *H*-imidazol-1-yl)-3,4-dihydro-2*H*-pyrido[1,2-a]pyrazine-1,6-dione, formate salt | 2.77 min¹³; 451 |
| **28 (Ref.)** | 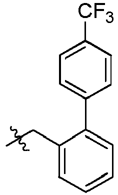 | Method B; **P5** | 7-(4-methyl-1*H*-imidazol-1-yl)-2-{[4'-(trifluoromethyl)biphenyl-2-yl]methyl}-3,4-dihydro-2*H-*pyrido[1,2-*a*]pyrazine-1,6-dione, formate salt | 2.84 min¹³; 479 |
| **29 (Ref.)** | 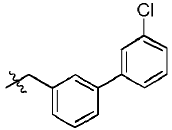 | Method B; **P4** | 2-[(3'-chlorobiphenyl-3-yl)methyl]-7-(4-methyl-1 *H*-imidazol-1-yl)-3,4-dihydro-2*H*-pyrido[1,2-a]pyrazine-1,6-dione, formate salt | 2.74 min¹³; 445 |
| **30 (Ref.)** | 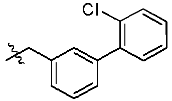 | Method B; **P4** | 2-[(2'-chlorobiphenyl-3-yl)methyl]-7-(4-methyl-1*H*-imidazol-1-yl)-3,4-dihydro-2*H*-pyrido[1,2-a]pyrazine-1,6-dione, formate salt | 2.65 min¹³; 445 |
| **31 (Ref.)** | 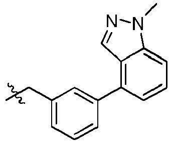 | Method B; **P4** | 7-(4-methyl-1*H*-imidazol-1-yl)-2-[3-(1-methyl-1*H*-indazol-4-yl)benzyl]-3,4-dihydro-2*H-*pyrido[1,2-*a*]pyrazine-1,6-dione, formate salt | 2.62 min¹²; 465 |
| **32 (Ref.)** | 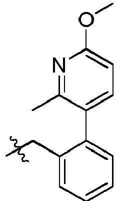 | Method B; **P5** | 2-[2-(6-methoxy-2-methylpyridin-3-yl)benzyl]-7-(4-methyl-1*H-*imidazol-1-yl)-3,4-dihydro-2*H-*pyrido[1,2-*a*]pyrazine-1,6-dione, formate salt | 2.42 min¹²; 456 |
| **33 (Ref.)** | 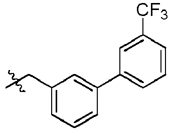 | Method B; **P4** | 7-(4-methyl-1*H*-imidazol-1-yl)-2-{[3'-(trifluoromethyl)biphenyl-3-yl]methyl}-3,4-dihydro-2*H-*pyrido[1,2-*a*]pyrazine-1,6-dione, formate salt | 2.82 min¹³; 479 |
| **34 (Ref.)** | 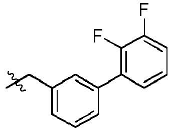 | Method B; **P4** | 2-[(2',3'-difluorobiphenyl-3-yl)methyl]-7-(4-methyl-1*H-*imidazol-1-yl)-3,4-dihydro-2*H-*pyrido[1,2-*a*]pyrazine-1,6-dione, formate salt | 2.82 min¹²; 447 |
| **35 (Ref.)** | 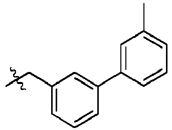 | Method B; **P4** | 2-[(3'-methyl biphenyl-3-yl)methyl]-7-(4-methyl-1*H-*imidazol-1-yl)-3,4-dihydro-2*H-*pyrido[1,2-*a*]pyrazine-1,6-dione, formate salt | 2.71 min¹³; 425 |
| **36 (Ref.)** | 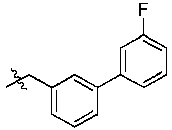 | Method B; **P4** | 2-[(3'-fluorobiphenyl-3-yl)methyl]-7-(4-methyl-1 *H*-imidazol-1-yl)-3,4-dihydro-2*H*-pyrido[1,2-*a*]pyrazine-1,6-dione, formate salt | 2.81 min¹²; 429 |
| **37 (Ref.)** | 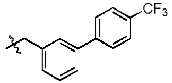 | Method B; **P4** | 7-(4-methyl-1*H*-imidazol-1-yl)-2-{[4'-(trifluoromethyl)biphenyl-3-yl]methyl}-3,4-dihydro-2*H-*pyrido[1,2-*a*]pyrazine-1,6-dione, formate salt | 2.84 min¹³; 479 |
| **38 (Ref.)** | 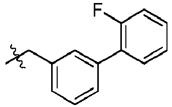 | Method B; **P4** | 2-[(2'-fluorobiphenyl-3-yl)methyl]-7-(4-methyl-1 *H*-imidazol-1-yl)-3,4-dihydro-2*H*-pyrido[1,2-a]pyrazine-1,6-dione, formate salt | 2.78 min¹²; 429 |
| **39 (Ref.)** | 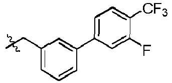 | Method B; **P4** | 2-{[3'-fluoro-4'-(trifluoromethyl)biphenyl-3-yl]methyl}-7-(4-methyl-1*H-*imidazol-1-yl)-3,4-dihydro-2*H-*pyrido[1,2-*a*]pyrazine-1,6-dione, formate salt | 2.85 min¹³; 497 |
| **40 (Ref.)** | 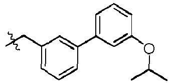 | Method B; **P4** | 7-(4-methyl-1*H*-imidazol-1-yl)-2-{[3'-(propan-2-yloxy)biphenyl-3-yl]methyl}-3,4-dihydro-2*H-*pyrido[1,2-*a*]pyrazine-1,6-dione, formate salt | 2.81 min¹³; 469 |
| **41 (Ref.)** | 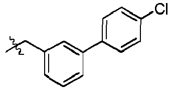 | Method B; **P4** | 2-[(4'-chlorobiphenyl-3-yl)methyl]-7-(4-methyl-1*H*-imidazol-1-yl)-3,4-dihydro-2*H*-pyrido[1,2-a]pyrazine-1,6-dione, formate salt | 2.75 min¹³; 445 |
| **42 (Ref.)** | 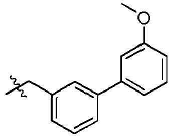 | Method B; **P4** | 2-[(3'-methoxybiphenyl-3-yl)methyl]-7-(4-methyl-1*H-*imidazol-1-yl)-3,4-dihydro-2*H-*pyrido[1,2-*a*]pyrazine-1,6-dione, formate salt | 2.77 min¹²; 441 |
| **43 (Ref.)** | 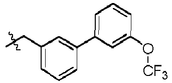 | Method B; **P4** | 7-(4-methyl-1*H*-imidazol-1-yl)-2-{[3'-(trifluoromethoxy)biphenyl-3-yl]methyl}-3,4-dihydro-2*H-*pyrido[1,2-*a*]pyrazine-1,6-dione, formate salt | 3.16 min¹⁴; 495 |
| **44 (Ref.)** | 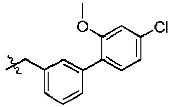 | Method B; **P4** | 2-[(4'-chloro-2'-methoxybiphenyl-3-yl)methyl]-7-(4-methyl-1*H-*imidazol-1-yl)-3,4-dihydro-2*H-*pyrido[1,2-*a*]pyrazine-1,6-dione, formate salt | 2.96 min¹²; 475 |
| **45 (Ref.)** | 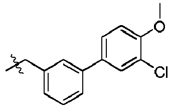 | Method B; **P4** | 2-[(3'-chloro-4'-methoxybiphenyl-3-yl)methyl]-7-(4-methyl-1*H-*imidazol-1-yl)-3,4-dihydro-2*H-*pyrido[1,2-*a*]pyrazine-1,6-dione, formate salt | 2.68 min¹³; 475 |
| **46 (Ref.)** | 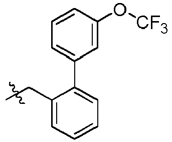 | Method B; **P5** | 7-(4-methyl-1*H*-imidazol-1-yl)-2-{[3'-(trifluoromethoxy)biphenyl-2-yl]methyl}-3,4-dihydro-2*H-*pyrido[1,2-*a*]pyrazine-1,6-dione, formate salt | 2.82 min¹³; 495 |
| **47 (Ref.)** | 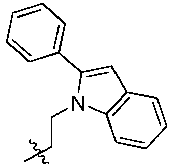 | Ex 1¹⁰ | 7-(4-methyl-1*H*-imidazol-1-yl)-2-[2-(2-phenyl-1*H*-indol-1-yl)ethyl]-3,4-dihydro-2*H*-pyrido[1,2-*a*]pyrazine-1,6-dione | 2.34 (d, *J*=0.9 Hz, 3H), 2.75-2.80 (m, 2H), 3.53-3.60 (m, 4H), 4.65-4.70 (m, 2H), 6.62 (d, *J*=0.7 Hz, 1H), 7.10 (d, *J*=7.7 Hz, 1H), 7.10-7.20 (m, 3H), 7.40-7.49 (m, 6H), 7.50 (d, *J*=7.7 Hz, 1H), 7.62-7.66 (m, 1H), 8.45 (d, *J*=1.1 Hz, 1H); 464.2 |
| **48 (Ref.)** | 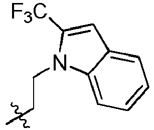 | Ex 1¹⁰ | 7-(4-methyl-1H-imidazol-1-yl)-2-{2-[2-(trifluoromethyl)-1*H*-indol-1-yl]ethyl}-3,4-dihydro-2*H-*pyrido[1,2-*a*]pyrazine-1,6-dione | 2.30 (d, *J*=1.0 Hz, 3H), 3.04-3.09 (m, 2H), 3.75-3.80 (m, 2H), 3.94 (dd, *J*=6.2, 6.0 Hz, 2H), 4.63 (dd, *J*=6.0, 6.0 Hz, 2H), 7.04 (br s, 1H), 7.12-7.15 (m, 1H), 7.19 (ddd, *J*=7.9, 7.1, 1.0 Hz, 1H), 7.26-7.32 (m, 1H), 7.30 (d, *J*=7.6 Hz, 1H), 7.46-7.50 (m, 1H), 7.47 (d, *J*=7.8 Hz, 1H), 7.69 (br d, *J*=8 Hz, 1H), 8.30 (d, *J*=1.0 Hz, 1H); 456.2 |
| **49 (Ref.)** | 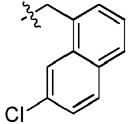 | Ex. 1¹⁵ | 2-[(7-chloronaphthalen-1-yl)methyl]-7-(4-methyl-1*H-*imidazol-1-yl)-3,4-dihydro-2*H-*pyrido[1,2-*a*]pyrazine-1,6-dione | 2.38 min²⁴; 418.9 |
| **50 (Ref.)** | 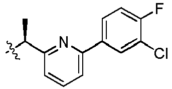 | Method D | 2-{(1*S*)-1-[6-(3-chloro-4-fluorophenyl)pyridin-2-yl]ethyl}-7-(4-methyl-1*H*-imidazol-1-yl)-3,4-dihydro-2*H*-pyrido[1,2-*a*]pyrazine-1,6-dione | 2.96 min²⁵; 478 |
| **51 (Ref.)** | 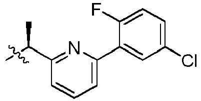 | Method D | 2-{(1S)-1-[6-(5-chloro-2-fluorophenyl)pyridin-2-yl]ethyl}-7-(4-methyl-1*H*-imidazol-1-yl)-3,4-dihydro-2*H*-pyrido[1,2-*a*]pyrazine-1,6-dione | 2.93 min²⁵; 478 |
| **52 (Ref.)** | 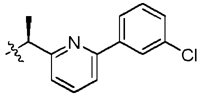 | Method D | 2-{(1*S*)-1-[6-(3-chlorophenyl)pyridin-2-yl]ethyl}-7-(4-methyl-1*H*-imidazol-1-yl)-3,4-dihydro-2*H*-pyrido[1,2-*a*]pyrazine-1,6-dione | 2.69 min²⁶; 460 |
| **53** | 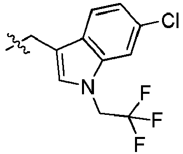 | Ex. 1¹⁶ | 2-{[6-chloro-1-(2,2,2-trifluoroethyl)-1*H*-indol-3-yl]methyl}-7-(4-methyl-1*H-*imidazol-1-yl)-3,4-dihydro-2*H-*pyrido[1,2-*a*]pyrazine-1,6-dione | 1.17 min²⁷; 490.1 |
| **54** | 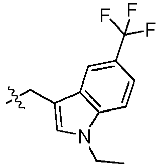 | Ex. 1¹⁷ | 2-{[1-ethyl-5-(trifluoromethyl)-1*H-*indol-3-yl]methyl}-7-(4-methyl-1*H*-imidazol-1-yl)-3,4-dihydro-2*H-*pyrido[1,2-*a*]pyrazine-1,6-dione | 1.15 min²⁸; 469.9 |
| **55** | 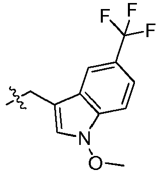 | Ex. 1¹⁸ | 2-{[1-methoxy-5-(trifluoromethyl)-1*H*-indol-3-yl]methyl}-7-(4-methyl-1*H*-imidazol-1-yl)-3,4-dihydro-2*H*-pyrido[1,2-*a*]pyrazine-1,6-dione | 2.52 min²⁹; 472.2 |
| **56** | 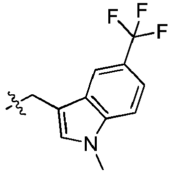 | Ex. 1¹⁷ | 7-(4-methyl-1*H*-imidazol-1-yl)-2-{[1-methyl-5-(trifluoromethyl)-1*H-*indol-3-yl]methyl}-3,4-dihydro-2*H-*pyrido[1,2-*a*]pyrazine-1,6-dione | 1.10 min²⁸; 455.9 |
| **57 (Ref.)** | 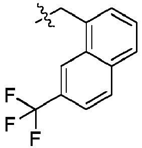 | Ex. 1²⁰ | 7-(4-methyl-1*H*-imidazol-1-yl)-2-{[7-(trifluoromethyl)naphthalen-1-yl]methyl}-3,4-dihydro-2*H-*pyrido[1,2-*a*]pyrazine-1,6-dione | 0.91 min³⁰; 453.1 |
| **58** | 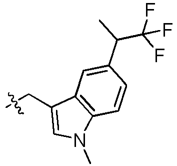 | Ex. 1²¹ | 7-(4-methyl-1*H*-imidazol-1-yl)-2-{[1-methyl-5-(1,1,1-trifluoropropan-2-yl)-1*H*-indol-3-yl]methyl}-3,4-dihydro-2*H-*pyrido[1,2-*a*]pyrazine-1,6-dione | 1.10 min²⁸; 484.0 |
| **59 (Ref.)** | 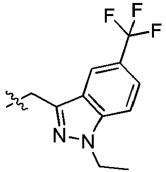 | Ex. 1¹⁷ | 2-{[1-ethyl-5-(trifluoromethyl)-1 *H-*indazol-3-yl]methyl}-7-(4-methyl-1 *H*-imidazol-1-yl)-3,4-dihydro-2*H-*pyrido[1,2-*a*]pyrazine-1,6-dione | 0.88 min³¹; 471.1 |
| **60** | 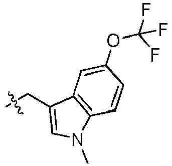 | Ex. 1²³ | 7-(4-methyl-1*H*-imidazol-1-yl)-2-{[1-methyl-5-(trifluoromethoxy)-1*H*-indol-3-yl]methyl}-3,4-dihydro-2*H*-pyrido[1,2-a]pyrazine-1,6-dione | 6.51 min³²; 472.0 |
| **61** | 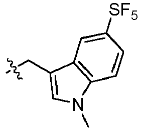 | Ex. 3³³ | 7-(4-methyl-1*H*-imidazol-1-yl)-2-{[1-methyl-5-(pentafluoro-λ⁶-sulfanyl)-1*H*-indol-3-yl]methyl}-3,4-dihydro-2*H*-pyrido[1,2-a]pyrazine-1,6-dione | ¹H NMR (400 MHz, CD₃OD) δ 2.29 (s, 3H), 3.59-3.73 (m, 2H), 3.87 (s, 3H), 4.18-4.31 (m, 2H), 4.97 (s, 2H), 7.36 (d, *J*=7.8 Hz, 1H), 7.42 (s, 1H), 7.46-7.57 (m, 2H), 7.63 (dd, *J*=9.4, 2.0 Hz, 1H), 7.85 (d, *J*=7.8 Hz, 1H), 8.24 (d, *J*=2.0 Hz, 1H), 8.60 (s, 1H); 514.2 |
| **62** | 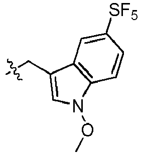 | Ex. 1³⁴ | 2-{[1-methoxy-5-(pentafluoro-λ⁶-sulfanyl)-1*H*-indol-3-yl]methyl}-7-(4-methyl-1*H*-imidazol-1-yl)-3,4-dihydro-2*H*-pyrido[1,2-*a*]pyrazine-1,6-dione | 2.26 min; 530.2³⁵ |
| **63** | 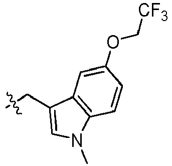 | Ex. 1³⁶ | 7-(4-methyl-1 *H*-imidazol-1-yl)-2-{[1-methyl-5-(2,2,2-trifluoroethoxy)-1 *H*-indol-3-yl]methyl}-3,4-dihydro-2*H-*pyrido[1,2-*a*]pyrazine-1,6-dione | 5.29 min; 485.8^{A5} |
| **64 (Ref.)** | 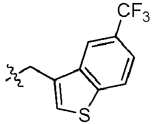 | Ex. 1³⁸ | 7-(4-methyl-1*H*-imidazol-1-yl)-2-{[5-(trifluoromethyl)-1-benzothiophen-3-yl]methyl}-3,4-dihydro-2*H*-pyrido[1,2-*a*]pyrazine-1,6-dione | 4.02 min; 459^{A7} |
| **65** | 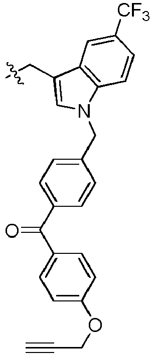 | Ex 3⁴⁰ | 7-(4-methyl-1*H*-imidazol-1-yl)-2-{[1-{4-[4-(prop-2-yn-1-yloxy)benzoyl]benzyl}-5-(trifluoromethyl)-1*H*-indol-3-yl]methyl}-3,4-dihydro-2*H-*pyrido[1,2-*a*]pyrazine-1,6-dione | 4.15 min; 690.4⁴¹ |
| **66 (Ref.)** | 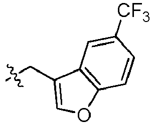 | Ex. 1⁴² | 7-(4-methyl-1 *H*-imidazol-1-yl)-2-{[5-(trifluoromethyl)-1-benzofuran-3-yl]methyl}-3,4-dihydro-2*H*-pyrido[1,2-*a*]pyrazine-1,6-dione | 5.52 min; 442.8^{A11} |
| **67** | 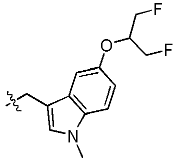 | Ex. 1⁴⁴ | 2-({5-[(1,3-difluoropropan-2-yl)oxy]-1-methyl-1 *H*-indol-3-yl}methyl)-7-(4-methyl-1 *H-*imidazol-1-yl)-3,4-dihydro-2*H-*pyrido[1,2-*a*]pyrazine-1,6-dione | 3.75 min; 481.8^{A7} |
| **68 (Ref.)** | 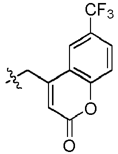 | Ex. 1⁴⁵ | 7-(4-methyl-1*H*-imidazol-1-yl)-2-{[2-oxo-6-(trifluoromethyl)-2*H-*chromen-4-yl]methyl}-3,4-dihydro-2*H*-pyrido[1,2-*a*]pyrazine-1,6-dione | 2.21 min; 471.1^{A11} |
| **69** | 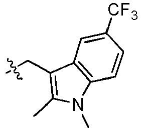 | Ex. 1⁴⁶ | 2-{[1,2-dimethyl-5-(trifluoromethyl)-1*H*-indol-3-yl]methyl}-7-(4-methyl-1*H-*imidazol-1-yl)-3,4-dihydro-2*H-*pyrido[1,2-*a*]pyrazine-1,6-dione | 6.17 min; 469.8^{A9} |

| | | | | |
|---|---|---|---|---|
| 1. 1-(3-Bromophenyl)methanamine was coupled to [2-(trifluoromethyl)phenyl]boronic acid via Suzuki reaction to give 1-[2'-(trifluoromethyl)biphenyl-3-yl]methanamine. This was converted to the requisite 2-aminoethanol using the general method described in Method A. 2. The requisite starting material can be accessed by palladium-mediated reaction of a 2-propyl zinc halide (see X. Luo et al., Org. Lett. 2007, 9, 4571-4574) with methyl (2-fluoro-4-iodophenyl)acetate (J. G. Varnes et al., Bioorg. Med. Chem. Lett. 2008, 18, 749-754). Conversion of the ester to a primary amine yields 2-[2-fluoro-4-(propan-2-yl)phenyl]ethanamine. 3. Cyclobutanecarbonitrile was alkylated with 1-(bromomethyl)-4-fluorobenzene, and the product was reduced with lithium aluminum hydride to generate 1-[1-(4-fluorobenzyl)cyclobutyl]methanamine. 4. 2-(3-Bromo-2-oxopropyl)-1*H*-isoindole-1,3(2*H*)-dione was reacted with 2-chlorobenzenecarbothioamide; the phthalimide protecting group of the product was removed using hydrazine to provide 1-[2-(2-chlorophenyl)-1,3-thiazol-4-yl]methanamine. 5. 2-[2-(2-Chlorophenyl)-1,3-thiazol-4-yl]ethanamine may be prepared by using 2-(4-bromo-3-oxobutyl)-1*H*-isoindole-1,3(2*H*)-dione as starting material for the chemistry in footnote 4. 6. Reaction of 1-[3-(trifluoromethyl)phenyl]ethanone with *N,N*-dimethylformamide dimethyl acetal provided 3-(dimethylamino)-1-[3-(trifluoromethyl)phenyl]prop-2-en-1-one. Treatment with *tert*-butyl (2-amino-2-iminoethyl)carbamate (prepared according to A. T. Wright and E. V. Anslyn, Org. Lett. 2004, 6, 1341-1344), followed by acidic deprotection, afforded 1-{4-[3-(trifluoromethyl)phenyl]pyrimidin-2-yl}methanamine. 7. The commercially available amine was converted to the requisite 2-aminoethanol using the general method described in Method A. 8. Reaction of 5-(chloromethyl)-3-[1-(4-chlorophenyl)cyclobutyl]-1,2,4-oxadiazole (see C. Cuiman et al., PCT Int. Appl. 2008**,** WO 2008117148 A1 20081002) with 2-aminoethanol provided the requisite 2-aminoethanol derivative. 9. 5-Bromo-2,2-diphenylpentanenitrile (which may be prepared according to J. W. Hulshof et al., J. Med. Chem. 2005, 48, 6461-6471), was reacted with 2-aminoethanol to afford 5-[(2-hydroxyethyl)amino]-2,2-diphenylpentanenitrile. 10. The appropriately substituted 9H-carbazole or indole was treated with 1,2-dibromoethane or 1,3-dibromopropane under basic conditions, and the resulting bromide was treated with 2-aminoethanol. 11. HPLC conditions. Column: Waters Atlantis dC18, 4.6 x 50 mm, 5 µm; Mobile phase A: 0.05% trifluoroacetic acid in water (v/v); Mobile phase B: 0.05% trifluoroacetic acid in acetonitrile (v/v); Gradient: 5% to 95% B over 4.0 min, linear; Flow rate: 2 mL/ min. 12. HPLC conditions. Column: Waters XBridge C18, 2.1 x 50 mm, 5 µm; Mobile phase A: 0.0375% trifluoroacetic acid in water (v/v); Mobile phase B: 0.01875% trifluoroacetic acid in acetonitrile (v/v); Gradient: 1% to 5% B over 0.6 min, then 5% to 100% B over 3.4 min; Flow rate: 0.8 mL/ min. 13. HPLC conditions. Column and mobile phases as in footnote 25. Gradient: 10% B for 0.5 min, then 10% to 100% B over 3.5 min; Flow rate: 0.8 mL/min. 14. HPLC conditions. Column: Waters XBridge C18, 2.1 x 50 mm, 5 µm; Mobile phase A: 0.05% ammonium hydroxide in water (v/v); Mobile phase B: acetonitrile; Gradient: 5% B for 0.5 min, then 5% to 100% B over 2.9 min, then 100% B; Flow rate: 0.8 mL/ min. 15. 7-Chloro-1-naphthaldehyde was subjected to reductive amination with ethanolamine to provide the requisite aminoalcohol coupling partner. 16. The requisite aminoalcohol coupling partner was prepared via alkylation of 6-chloro-1*H*-indole-3-carbaldehyde with 2,2,2-trifluoroethyl trifluoromethanesulfonate followed by reductive amination with ethanolamine. 17. Alkylation of 5-(trifluoromethyl)-1*H*-indole with ethyl iodide followed by formylation with phosphorous oxychloride and *N,N*-dimethylformamide gave 1-ethyl-5-(trifluoromethyl)-1*H*-indole-3-carbaldehyde. Reductive amination with ethanolamine gave the requisite aminoalcohol coupling partner. 18. 2-Methyl-1-nitro-4-(trifluoromethyl)benzene was heated with *N,N-*dimethylformamide dimethyl acetale and DBU to afford *N,N*-dimethyl-2-(2-nitro-5-(trifluoromethyl)phenyl)ethenamine, which in turn was converted to 5-(trifluoromethyl)-1*H*-indol-1-ol via partial reduction with zinc. Alkylation with methyl iodide followed by formylation using phosphorus oxychloride and *N,N*-dimethylformamide gave 1-methoxy-5-(trifluoromethyl)-1*H*-indole-3-carbaldehyde. Reductive amination with ethanolamine gave the requisite amino alcohol coupling partner. 19. Alkylation of 5-(trifluoromethyl)-1*H*-indole with methyl iodide followed by formylation with phosphorous oxychloride and *N,N*-dimethylformamide gave 1-methyl-5-(trifluoromethyl)-1*H*-indole-3-carbaldehyde. Reductive amination with ethanolamine gave the requisite aminoalcohol coupling partner. 20. 7-Bromo-1-naphthaldehyde was converted to 7-(trifluoromethyl)-1-naphthaldehyde using methyl 2,2-difluoro-2-(fluorosulfonyl)acetate and copper(l) bromide. Reductive amination with ethanolamine gave the requisite aminoalcohol coupling partner. 21. Suzuki coupling of 1-methyl-1*H*-indol-5-ylboronic acid with 2-bromo-3,3,3-trifluoroprop-1-ene followed by olefin reduction via hydrogenation gave 1-methyl-5-(1,1,1-trifluoropropan-2-yl)-1*H*-indole. Formylation with phosphorous oxychloride and *N,N*-dimethylformamide, followed by reductive amination with ethanolamine, gave the requisite aminoalcohol coupling partner. 22. 5-(Trifluoromethyl)-1*H*-indazole was iodinated with *N*-iodosuccinamide followed by *N*-alkylation with ethyl iodide to give 1-ethyl-3-iodo-5-(trifluoromethyl)-1*H-*indazole. Treatment with isopropylmagnesium bromide followed by *N,N-*dimethylformamide afforded 1-ethyl-5-(trifluoromethyl)-1*H*-indazole-3-carbaldehyde. Reductive amination with ethanolamine gave the requisite aminoalcohol coupling partner. 23. 4-(Trifluoromethoxy)aniline was converted to [4-(trifluoromethoxy)phenyl]hydrazine hydrochloride by reaction with NaNO₂ and SnCl₂. The resulting hydrazine was condensed with methyl pyruvate and cyclized to give ethyl 5-(trifluoromethoxy)-1*H*-indole-2-carboxylate. Hydrolysis of the ester followed by decarboxylation gave 5-(trifluoromethoxy)-1*H*-indole, which was alkylated with methyl iodide and treated with phosphorus oxychloride and *N,N*-dimethylformamide to afford 1-methyl-5-(trifluoromethoxy)-1*H*-indole-3-carbaldehyde. Reductive amination with ethanolamine delivered the requisite aminoalcohol coupling partner. 24. HPLC Conditions. Column: Waters Atlantis dC18, 4.6 x 50 mm, 5 µm; Mobile phase A: 0.05% TFA in water (v/v); Mobile phase B: 0.05% TFA in acetonitrile (v/v); Gradient: 5% B linear to 95% B over 4.0 min, HOLD at 95% B to 5.0 min. Flow rate: 1.5 mL/min. 25. HPLC Conditions. Column: XBridge C18, 2.1 x 50 mm, 5 µm; Mobile phase A: 0.0375% TFA in water (v/v); Mobile phase B: 0.01875% TFA in acetonitrile (v/v); Gradient: 1% B to 5% B over 0.6 min; then 5% B to 100 B% to 4.0 min. Flow rate: 0.8 mL/min. 26. HPLC Conditions. Column: XBridge C18, 2.1 x 50 mm, 5 µm; Mobile phase A: 0.05% NH₄OH in water (v/v); Mobile phase B: 100% acetonitrile (v/v); Gradient: 5% B until 0.5 min, 5% B to 100% B to 3.4 min, 100% B to 4.2 min. Flow rate: 0.8 mL/min. 27. HPLC conditions. Column: Xtimate C18, 2.1 x 30 mm, 3 µm; Mobile phase A: 0.0375% trifluoroacetic acid in water (v/v); Mobile phase B: 0.01875% trifluoroacetic acid in acetonitrile (v/v); Gradient: 0% to 60% B over 0.9 min, linear, and holding at 60% for 0.6 min. Flow rate: 1.2 mL/min. 28. HPLC conditions. Column: Ultimate XB C18, 2.1 x 30 mm, 3 µm; Mobile phase A: 0.0375% trifluoroacetic acid in water (v/v); Mobile phase B: 0.01875% trifluoroacetic acid in acetonitrile (v/v); Gradient: 0% to 60% B over 0.9 min, linear, and holding at 60% for 0.6 min; Flow rate: 1.2 mL/min. 29. HPLC Conditions. Column: Waters Atlantis dC18, 4.6x50 mm, 5 µm; Mobile phase A: 0.05% TFA in water (v/v); Mobile phase B: 0.05% TFA in acetonitrile (v/v); Gradient: 5% B linear to 95% B over 4.0 min, 95% B to 5.0 min. Flow rate: 2.0 mL/min. 30. HPLC conditions. Column: Xtimate C18, 2.1 x 30 mm, 3 µm; Mobile phase A: 0.0375% trifluoroacetic acid in water (v/v); Mobile phase B: 0.01875% trifluoroacetic acid in acetonitrile (v/v); Gradient: 10% to 80% B over 0.9 min, linear, and holding at 80% for 0.6 min; Flow rate: 1.2 mL/min. 31. HPLC conditions. Column: Xtimate C18, 2.1 x 30mm, 3 µm; Mobile phase A: 0.0375% trifluoroacetic acid in water (v/v); Mobile phase B: 0.01875% trifluoroacetic acid in acetonitrile (v/v); Gradient: 0% to 60% B over 0.9 min, linear, and holding at 60% for 0.6 min; Flow rate: 1.2 mL/ min. 32. HPLC conditions. Column: Zorbax SB C18, 50 x 4.6 mm, 1.8 µm; Mobile phase A: 0.05% trifluoroacetic acid in water (v/v); Mobile phase B: acetonitrile (v/v); Gradient: 2% to 98% B over 12.0 min, linear; Flow rate: 1 mL/min. 33. The requisite aminoalcohol coupling partner was prepared via alkylation of 5-(pentafluoro-λ⁶-sulfanyl)-1*H*-indole-3-carbaldehyde (Reference Example 3) with methyl iodide followed by reductive amination with ethanolamine. 34. 5-(Pentafluoro-λ⁶-sulfanyl)-1*H*-indole was reduced with sodium cyanoborohydride to provide 5-(pentafluoro-λ⁶-sulfanyl)-2,3-dihydro-1*H*-indole. Oxidation with sodium tungsten dihydrate and hydrogen peroxide followed by alkylation with methyl iodide afforded 1-methoxy-5-(pentafluoro-λ⁶-sulfanyl)-1*H*-indole. Formylation with phosphorus oxychloride and *N,N*-dimethylformamide gave 1-methoxy-5-(pentafluoro-λ⁶-sulfanyl)-1*H*-indole-3-carbaldehyde, which was subjected to reductive amination with ethanolamine to provide the requisite aminoalcohol coupling partner. 35. HPLC conditions. Column: Waters Atlantis dC18, 4.6 x 50 mm, 5 µm; Mobile phase A: 0.05% trifluoroacetic acid in water (v/v); Mobile phase B: 0.05% trifluoroacetic acid in acetonitrile (v/v); Gradient: 5% to 95% B over 4.0 min, linear, and holding at 95% B for 1 min; Flow rate: 2 mL/min. 36. Alkylation of 1-methyl-1*H*-indol-5-ol with 2,2,2-trifluoroethyl trifluoromethanesulfonate, followed by formylation with *N*-(chloromethylene)-*N-*methylmethanaminium chloride, gave 1-methyl-5-(2,2,2-trifluoroethoxy)-1*H*-indole-3-carbaldehyde. Reductive amination with ethanolamine gave the requisite aminoalcohol coupling partner. 37. HPLC conditions. Column: Gemini-NX C18, 100 x 4.6 mm, 5 µm; Mobile phase A: 10 mM ammonium acetate in water (v/v); Mobile phase B: acetonitrile; Gradient: 40% B for 1.0 min, then to 60% B over 5.0 min, then to 90% B over 4.0 min, and holding at this composition for 6.0 min; Flow rate: 1 mL/min. 38. The requisite aminoalcohol coupling partner was prepared via reductive amination of 5-(trifluoromethyl)-1-benzothiophene-3-carbaldehyde with ethanolamine. 39. HPLC conditions. Column: ZORBAX SB C18, 4.6 x 50 mm, 1.8 µm; Mobile phase A: 0.05% trifluoroacetic acid in water (v/v); Mobile phase B: acetonitrile; Gradient: 10% B for 0.5 min, then to 90% B over 4.0 min, and holding at this composition for 2.5 min; Flow rate: 1 mL/min. 40. The title compound was prepared via alkylation of 7-(4-methyl-1*H*-imidazol-1-yl)-2-{[5-(trifluoromethyl)-1*H*-indol-3-yl]methyl}-3,4-dihydro-2*H*-pyrido[1,2-*a*]pyrazine-1,6-dione (prepared using methods analogous to those described for the conversion of **C22** to **3** in Reference Example 3) with [4-(bromomethyl)phenyl][4-(prop-2-yn-1-yloxy)phenyl]methanone. 41. HPLC conditions. Column: ZORBAX SB C18, 4.6 x 50 mm, 1.8 µm; Mobile phase: A 0.05% trifluoroacetic acid in water (v/v); Mobile phase B: acetonitrile; Gradient: 10% B for 0.5 min, then to 90% B over 3.0 min, and holding at this composition for 6.5 min; Flow rate: 1 mL/min. 42. Alkylation of 2-iodo-4-(trifluoromethyl)phenol with allyl bromide gave 2-iodo-1-(prop-2-en-1-yloxy)-4-(trifluoromethyl)benzene, which was subjected to intramolecular Heck reaction to provide 3-methyl-5-(trifluoromethyl)-1-benzofuran. Bromination with *N-*bromosuccinimide in the presence of benzoyl peroxide gave 3-(bromomethyl)-5-(trifluoromethyl)-1-benzofuran, which was alkylated with ethanolamine to provide the requisite aminoalcohol coupling partner. 43. HPLC conditions. Column: Gemini-C18, 100 x 4.6 mm, 5 µm; Mobile phase A: 10 mM ammonium acetate in water (v/v); Mobile phase B: acetonitrile; Gradient: 20% B for 0.01 min, then to 30% B over 0.50 min, then to 70% B over 4.5 min, then to 90% B over 2 min, and holding at this composition for 5.5 min; Flow rate: 1 mL/min. 44. Mitsunobu reaction of 1-methyl-1*H*-indol-5-ol with 1,3-difluoropropan-2-ol gave 5-[(1,3-difluoropropan-2-yl)oxy]-1-methyl-1*H*-indole, which was formylated with N-(chloromethylene)-*N*-methylmethanaminium chloride to afford 5-[(1,3-difluoropropan-2-yl)oxy]-1-methyl-1*H*-indole-3-carbaldehyde. Reductive amination with ethanolamine gave the requisite aminoalcohol coupling partner. 45. 4-(Trifluoromethyl)phenol was alkylated with propargyl bromide to afford 1-(prop-2-yn-1-yloxy)-4-(trifluoromethyl)benzene. Deprotonation with n-butyllithium followed by alkylation with paraformaldehyde gave 4-[4-(trifluoromethyl)phenoxy]but-2-yn-1-ol, which was subjected to indium(III) iodide-mediated cyclization to afford [6-(trifluoromethyl)-2*H*-chromen-4-yl]methanol. Oxidation with pyridinium chlorochromate afforded 2-oxo-6-(trifluoromethyl)-2*H*-chromene-4-carbaldehyde, which underwent reductive amination with ethanolamine to provide the requisite aminoalcohol coupling partner. 46. Methyl 2-methyl-5-(trifluoromethyl)-1*H*-indole-3-carboxylate was prepared via a copper-catalyzed coupling of 2-bromo-4-(trifluoromethyl)aniline and methyl acetoacetate. *N*-Alkylation with methyl iodide followed by reduction with lithium aluminum hydride gave [1,2-dimethyl-5-(trifluoromethyl)-1*H*-indol-3-yl]methanol, which was oxidized to afford 1,2-dimethyl-5-(trifluoromethyl)-1*H*-indole-3-carbaldehyde. Reductive amination with ethanolamine provided the requisite aminoalcohol coupling partner. | | | | |

### Cell-based y-secretase assay with ELISA readout

The ability of compounds to modulate production of amyloid beta protein Aβ(1-42) was determined using human WT-APP overexpressing CHO cells. Cells were plated at 22,000 cells/100 µL well in 96 well tissue culture treated, clear plates (Falcon) in DMEM/F12 based medium and incubated for 24 h at 37 °C. Compounds for testing were diluted in 100% DMSO to achieve an eleven point, half log, dose response for IC₅₀ determinations. Compounds were added in fresh medium to achieve 1% final DMSO. Appropriate vehicle or inhibitor controls were added into control wells individually to obtain minimum or maximum inhibition values, respectively, for the assay signal window before the plates were incubated for -24 h at 37 °C. This procedure produces conditioned media in each well which is tested for Aβ(1-42) levels in the ELISA detection step described next. The remaining cell cultures in each well are also tested for cell toxicity as described below.

Coating of ELISA assay plates was initiated by addition of 50 µL/well of an in-house Aβ(1-42) specific antibody at (3 µg/mL) in 0.1 M NaHCO₃ (pH 9.0) into black 384-well Maxisorp® plates (Nunc) and incubated overnight at 4 °C. The capture antibody was then aspirated from the ELISA assay plates and plates were washed either 2 x 100 uL with a Matrical Squirt plate washer, or 3 x 90 uL with a Thermo Combi, using Wash Buffer (Dulbecco's PBS, 0.05% Tween 20). 90 µL/well of Blocking Buffer (Dulbecco's PBS, 1.0% BSA (Sigma A7030) was then added to plates. Ambient temperature incubation was allowed to proceed for a minimum of 2 h. Blocking buffer was then removed and 20 µL/well Assay Buffer (Dulbecco's PBS, 1.0% BSA (Sigma A7030), 0.05% Tween 20) was then added. At this point, 35 uL (40 uL prior to August, 2012) (in duplicate) of experimental conditioned media (described above) were transferred into wells of the blocked ELISA plates containing the capture antibody, followed by overnight incubation at 4 °C. Cell toxicity was also measured in the corresponding remaining cells after removal of the conditioned media for the Aβ(1-42) assay by a colorimetric cell proliferation assay (CellTiter 96® AQᵤₑₒᵤₛ One Solution Cell Proliferation Assay, Promega) according to the manufacturer's instructions.

After overnight incubation of the ELISA assay plates at 4 °C, unbound Aβ peptides were removed via either 2 x 100 uL washes with a Matrical Squirt plate washer, or 3 x 90 uL washes with a Thermo Combi, using Wash Buffer. Europium (Eu) labeled (custom labeled, PerkinElmer) Aβ(1-16) 6e10 Monoclonal Antibody (Covance #SIG-39320) was added, (50 µL/well Eu-6e10 @ 1:10,000, 20 uM EDTA) in Assay Buffer. Incubation at ambient temperature for a minimum of 2 h was followed by either 2 x 100 uL washes with a Matrical Squirt plate washer, or 3 x 90 uL washes with a Thermo Combi, using Wash Buffer, before 30 µL/well of Delfia Enhancement Solution (PerkinElmer) was added. Following 30 to 60 min ambient temperature incubation, the plates were read on an EnVision plate reader (PerkinElmer) using standard DELFIA TRF settings. Data analysis including inhibitory IC₅₀ determination was performed using nonlinear regression fit analysis (in-house software) and the appropriate plate mean values for the maximum and minimum inhibition controls.

Biological data for the compounds of Examples 2, 4, 5, 53-56, 58, 60-63, 65, 67 and 69 are found in Table 5 below:

**TABLE 5: Biological Data for Examples 2, 4, 5, 53-56, 58, 60-63, 65, 67 and 69**

| Example number | Aβ 42B IC₅₀ (nM) (Geometric Mean of 2-5 Determinations) |
|---|---|
| 2 | 8.8 |
| 4 | 2.5 |
| 5 | 51.8 |
| 53 | 71.5 |
| 54 | 17.3 |
| 55 | 21.5 |
| 56 | 21.7 |
| 58 | 45.9 |
| 60 | 13.6 |
| 61 | 5.6 |
| 62 | 9.5 |
| 63 | 20.5 |
| 65 | 32.1 |
| 67 | 136 |
| 69 | 7.5 |

## Claims

1. A compound that is selected from the group consisting of:
2-{[7-Fluoro-1-methyl-5-(trifluoromethyl)-1*H*-indol-3-yl]methyl}-7-(4-methyl-1*H-*imidazol-1-yl)-3,4-dihydro-1*H*-pyrido[1,2-*a*]pyrazine-1,6(2*H*)-dione;
2-{[1-Ethyl-5-(pentafluoro-λ⁶-sulfanyl)-1*H*-indol-3-yl]methyl}-7-(4-methyl-1*H-*imidazol-1-yl)-3,4-dihydro-2*H*-pyrido[1,2-*a*]pyrazine-1,6-dione;
2-{[1-Ethyl-5-(trifluoromethyl)-1*H*-pyrrolo[2,3-*b*]pyridin-3-yl]methyl}-7-(4-methyl-1*H-*imidazol-1-yl)-3,4-dihydro-2*H*-pyrido[1,2-*a*]pyrazine-1,6-dione;
2-{[6-chloro-1-(2,2,2-trifluoroethyl)-1*H*-indol-3-yl]methyl}-7-(4-methyl-1*H*-imidazol-1-yl)-3,4-dihydro-2*H*-pyrido[1,2-*a*]pyrazine-1,6-dione;
2-{[1-ethyl-5-(trifluoromethyl)-1*H*-indol-3-yl]methyl}-7-(4-methyl-1*H*-imidazol-1-yl)-3,4-dihydro-2*H*-pyrido[1,2-*a*]pyrazine-1,6-dione;
2-{[1-methoxy-5-(trifluoromethyl)-1*H*-indol-3-yl]methyl}-7-(4-methyl-1*H*-imidazol-1-yl)-3,4-dihydro-2*H*-pyrido[1,2-*a*]pyrazine-1,6-dione;
7-(4-methyl-1*H*-imidazol-1-yl)-2-{[1-methyl-5-(trifluoromethyl)-1*H*-indol-3-yl]methyl}-3,4-dihydro-2*H*-pyrido[1,2-*a*]pyrazine-1,6-dione;
7-(4-methyl-1*H*-imidazol-1-yl)-2-{[1-methyl-5-(1,1,1-trifluoropropan-2-yl)-1*H*-indol-3-yl]methyl}-3,4-dihydro-2*H*-pyrido[1,2-*a*]pyrazine-1,6-dione;
7-(4-methyl-1*H*-imidazol-1-yl)-2-{[1-methyl-5-(trifluoromethoxy)-1*H*-indol-3-yl]methyl}-3,4-dihydro-2*H*-pyrido[1,2-*a*]pyrazine-1,6-dione;
7-(4-methyl-1*H*-imidazol-1-yl)-2-{[1-methyl-5-(pentafluoro-λ⁶-sulfanyl)-1*H*-indol-3-yl]methyl}-3,4-dihydro-2*H*-pyrido[1,2-*a*]pyrazine-1,6-dione;
2-{[1-methoxy-5-(pentafluoro-λ⁶-sulfanyl)-1*H*-indol-3-yl]methyl}-7-(4-methyl-1*H-*imidazol-1 -yl)-3,4-dihydro-2*H*-pyrido[1,2-a]pyrazine-1,6-dione;
7-(4-methyl-1*H*-imidazol-1-yl)-2-{[1-methyl-5-(2,2,2-trifluoroethoxy)-1*H*-indol-3-yl]methyl}-3,4-dihydro-2*H*-pyrido[1,2-*a*]pyrazine-1,6-dione;
7-(4-methyl-1*H*-imidazol-1-yl)-2-{[1-{4-[4-(prop-2-yn-1-yloxy)benzoyl]benzyl}-5-(trifluoromethyl)-1*H*-indol-3-yl]methyl}-3,4-dihydro-2*H*-pyrido[1,2-*a*]pyrazine-1,6-dione;
2-({5-[(1,3-difluoropropan-2-yl)oxy]-1-methyl-1*H*-indol-3-yl}methyl)-7-(4-methyl-1*H-*imidazol-1-yl)-3,4-dihydro-2*H*-pyrido[1,2-a]pyrazine-1,6-dione; and
2-{[1,2-dimethyl-5-(trifluoromethyl)-1*H*-indol-3-yl]methyl}-7-(4-methyl-1*H*-imidazol-1-yl)-3,4-dihydro-2*H*-pyrido[1,2-*a*]pyrazine-1,6-dione;
or a pharmaceutically acceptable salt thereof.

2. The compound according to claim 1, or a pharmaceutically acceptable salt thereof, for use in the treatment of neurodegeneration and psychiatric disorders, including Alzheimer's disease or Niemann-Pick disease type C.

3. A pharmaceutical composition comprising a compound of claim 1, or a pharmaceutically acceptable salt thereof, and a pharmaceutically acceptable excipient.

## Patentansprüche

1. Verbindung, die ausgewählt ist aus der Gruppe, bestehend aus:
2-{[7-Fluor-1-methyl-5-(trifluormethyl)-1*H*-indol-3-yl]methyl}-7-(4-methyl-1*H*-imidazol-1-yl)-3,4-dihydro-1*H*-pyrido[1,2-*a*]pyrazin-1,6(2H)-dion;
2-{[1-Ethyl-5-(pentafluor-λ⁶-sulfanyl)-1*H*-indol-3-yl]methyl}-7-(4-methyl-1*H*-imidazol-1-yl)-3,4-dihydro-2H-pyrido[1,2-*a*]pyrazin-1,6-dion;
2-{-[1-Ethyl-5-(trifluormethyl)-1*H*-pyrrolo[2,3-*b*]pyridin-3 yl]methyl}-7-(4-methyl-1*H*-imidazol-1-yl)-3,4-dihydro-2H-pyrido[1,2-*a*]pyrazin-1,6-dion;
2-{[6-Chlor-1-(2,2,2-trifluorethyl)-1*H*-indol-3-yl]methyl}-7-(4-methyl-1*H*-imidazol-1-yl)-3,4-dihydro-2H-pyrido[1,2-*a*]pyrazin-1,6-dion;
2-{[1-Ethyl-5-(trifluormethyl)-1*H*-indol-3-yl]methyl}-7-(4-methyl-1*H*-imidazol-1-yl)-3,4-dihydro-2H-pyrido[1,2-*a*]pyrazin-1,6-dion;
2-{[1-Methoxy-5-(trifluormethyl)-1*H*-indol-3-yl]methyl}-7-(4-methyl-1*H*-imidazol-1-yl)-3,4-dihydro-2H-pyrido[1,2-*a*]pyrazin-1,6-dion;
7-(4-Methyl-1*H*-imidazol-1-y1)-2-{[1-methyl-5-(trifluormethyl)-1*H-*indol-3-yl]methyl}-3,4-dihydro-2H-pyrido[1,2-*a*]pyrazin-1,6-dion;
7-(4-Methyl-1H-imidazol-1-yl)-2-{[1-methyl-5-(1,1,1-trifluorpropan-2-yl)-1*H*-indol-3-yl]methyl}-3,4-dihydro-2H-pyrido[1,2-a]pyrazin-1,6-dion;
7-(4-Methyl-1*H*-imidazol-1-yl)-2-{[1-methyl-5-(trifluormethoxy)-1*H-*indol-3-yl]methyl}-3,4-dihydro-2H-pyrido[1,2-*a*]pyrazin-1,6-dion;
7-(4-Methyl-1*H*-imidazol-1-yl)-2-{[1-methyl-5-(pentafluor-λ⁶-sulfanyl)-1*H*-indol-3-yl]methyl}-3,4-dihydro-2H-pyrido[1,2-*a*]pyrazin-1,6-dion;
2-{[1-Methoxy-5-(pentafluor-λ⁶-sulfanyl)-1*H*-indol-3-yl]methyl}-7-(4-methyl-1*H*-imidazol-1-yl)-3,4-dihydro-2H-pyrido[1,2-*a*]pyrazin-1,6-dion;
7-(4-Methyl-1*H*-imidazol-1-yl)-2-{[1-methyl-5-(2,2,2-trifluorethoxy)-1*H*-indol-3-yl]methyl}-3,4-dihydro-2H-pyrido[1,2-*a*]pyrazin-1,6-dion;
7-(4-Methyl-1*H*-imidazol-1-yl)-2-{[1-{4-[4-(prop-2-yn-1-yloxy)benzoyl]benzyl}-5-(trifluormethyl)-1*H*-indol-3-yl]methyl}-3,4-dihydro-2H-pyrido[1,2-*a*]pyrazin-1,6-dion;
2-({5-[(1,3-Difluorpropan-2-yl)oxy]-1-methyl-1*H*-indol-3-yl}methyl)-7-(4-methyl-1*H*-imidazol-1-yl)-3,4-dihydro-2H-pyrido[1,2-*a*]pyrazin-1,6-dion und
2-{[1,2-Dimethyl-5-(trifluormethyl)-1*H*-indol-3-yl]methyl}-7-(4-methyl-1*H*-imidazol-1-yl)-3,4-dihydro-2H-pyrido[1,2-*a*]pyrazin-1,6-dion;
oder ein pharmazeutisch annehmbares Salz davon.

2. Verbindung gemäß Anspruch 1 oder ein pharmazeutisch annehmbares Salz davon zur Verwendung in der Behandlung von Neurodegeneration und psychiatrischen Störungen, einschließlich Alzheimerscher Krankheit oder Niemann-Pick-Krankheit, Typ C.

3. Pharmazeutische Zusammensetzung, umfassend eine Verbindung gemäß Anspruch 1 oder ein pharmazeutisch annehmbares Salz davon und ein pharmazeutisch annehmbares Exzipiens.

## Revendications

1. Composé qui est sélectionné dans le groupe consistant en :
la 2-{[7-fluoro-1-méthyl-5-(trifluorométhyl)-1*H*-indol-3-yl]méthyl}-7-(4-méthyl-1*H*-imidazol-1-yl)-3,4-dihydro-1*H-*pyrido[1,2-*a*]pyrazine-1,6(2H)-dione ;
la 2-{[1-éthyl-5-(pentafluoro-λ⁶-sulfanyl)-1*H*-indol-3-yl]méthyl}-7-(4-méthyl-1*H*-imidazol-1-yl)-3,4-dihydro-2*H-*pyrido[1,2-*a*]pyrazine-1,6-dione ;
la 2-{[1-éthyl-5-(trifluorométhyl)-1*H*-pyrrolo[2,3-*b*]pyridin-3-yl]méthyl}-7-(4-méthyl-1*H*-imidazol-1-yl)-3,4-dihydro-2*H*-pyrido[1,2-*a*]pyrazine-1,6-dione ;
la 2-{[6-chloro-1-(2,2,2-trifluoroéthyl)-1*H*-indol-3-yl]méthyl}-7-(4-méthyl-1*H*-imidazol-1-yl)-3,4-dihydro-2*H-*pyrido[1,2-*a*]pyrazine-1,6-dione ;
la 2-{[1-éthyl-5-(trifluorométhyl)-1*H*-indol-3-yl] méthyl}-7-(4-méthyl-1*H*-imidazol-1-yl)-3,4-dihydro-2*H-*pyrido[1,2-*a*]pyrazine-1,6-dione ;
la 2-{[1-méthoxy-5-(trifluorométhyl)-1*H*-indol-3-yl] méthyl}-7-(4-méthyl-1*H*-imidazol-1-yl)-3,4-dihydro-2*H-*pyrido[1,2-*a*]pyrazine-1,6-dione ;
la 7-(4-méthyl-1*H*-imidazol-1-yl)-2-{[1-méthyl-5-(trifluorométhyl)-1*H*-indol-3-yl]méthyl}-3,4-dihydro-2*H-*pyrido[1,2-*a*]pyrazine-1,6-dione ;
la 7-(4-méthyl-1*H*-imidazol-1-yl)-2-{[1-méthyl-5-(1,1,1-trifluoropropan-2-yl)-1*H*-indol-3-yl]méthyl}-3,4-dihydro-2*H-*pyrido[1,2-*a*]pyrazine-1,6-dione ;
la 7-(4-méthyl-1*H*-imidazol-1-yl)-2-{[1-méthyl-5-(trifluorométhoxy)-1*H*-indol-3-yl]méthyl}-3,4-dihydro-2*H-*pyrido[1,2-*a*]pyrazine-1,6-dione ;
la 7-(4-méthyl-1*H*-imidazol-1-yl)-2-{[1-méthyl-5-(pentafluoro-λ⁶-sulfanyl)-1*H*-indol-3-yl]méthyl}-3,4-dihydro-2*H*-pyrido[1,2-*a*]pyrazine-1,6-dione ;
la 2-{[1-méthoxy-5-(pentafluoro-λ⁶-sulfanyl)-1*H*-indol-3-yl]méthyl}-7-(4-méthyl-1*H*-imidazol-1-yl)-3,4-dihydro-2*H-*pyrido[1,2-*a*]pyrazine-1,6-dione ;
la 7-(4-méthyl-1*H*-imidazol-1-yl)-2-{[1-méthyl-5-(2,2,2-trifluoroéthoxy)-1*H*-indol-3-yl]méthyl}-3,4-dihydro-2*H-*pyrido[1,2-*a*]pyrazine-1,6-dione ;
la 7-(4-méthyl-1*H*-imidazol-1-yl)-2-{[1-[4-[4-(prop-2-yn-1-yloxy)benzoyl]benzyl}-5-(trifluorométhyl)-1*H*-indol-3-yl]méthyl}-3,4-dihydro-2*H*-pyrido[1,2-*a*]pyrazine-1,6-dione ;
la 2-({5-[(1,3-difluoropropan-2-yl)oxy]-1-méthyl-1*H-*indol-3-yl}méthyl)-7-(4-méthyl-1*H*-imidazol-1-yl)-3,4-dihydro-2*H*-pyrido[1,2-*a*]pyrazine-1,6-dione ; et
la 2-{[1,2-diméthyl-5-(trifluorométhyl)-1*H*-indol-3-yl]méthyl}-7-(4-méthyl-1*H*-imidazol-1-yl)-3,4-dihydro-2*H-*pyrido[1,2-*a*]pyrazine-1,6-dione ;
ou un sel pharmaceutiquement acceptable de celles-ci.

2. Composé selon la revendication 1, ou un sel pharmaceutiquement acceptable de celui-ci, pour une utilisation dans le traitement des troubles de neurodégénérescence et psychiatriques, incluant la maladie d'Alzheimer ou la maladie de Niemann-Pick type C.

3. Composition pharmaceutique comprenant un composé selon la revendication 1, ou un sel pharmaceutiquement acceptable de celui-ci, et un excipient pharmaceutiquement acceptable.
